# EUROPEAN PATENT APPLICATION

(11) **EP 2 123 762 A2**
(43) Date of publication of application: **25.11.2009**
(21) Application number: 09075123.1
(22) Date of filing: 18.03.2009
(51) Int. Cl.: C12N 15/82, C12N 15/85

(54) **Tandem repeat DNA constructs producing proteins that attack plant pathogenic viruses, fungi, and bacteria by disrupting transcription factors essential for replication thereof in plants**

(30) Priority: 19.03.2008 US 51096
(71) Applicant: Fernandez-Pol, Sebastian, Chesterfield, MO 63017-3446 (US); Fernandez-Pol, José A., Chesterfield, MO 63017-3446 (US)
(72) Inventor: Fernandez-Pol, Sebastian, Chesterfield, MO 63017-3446 (US); Fernandez-Pol, José A., Chesterfield, MO 63017-3446 (US)
(74) Representative: Gislon, Gabriele

(57) **Abstract**

Methods and compositions related to treating, controlling or inhibiting *Geminiviruses* by reducing or inhibiting growth of *Geminiviruses* (GV) employing a compound having the following structure: 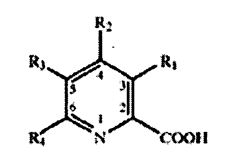 or a pharmaceutically acceptable salt thereof, wherein R₁ R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen. The present invention provides several classes of antiviral compounds which can be used to inactivate or disintegrate *geminiviruses*, such as the *African cassava mosaic geminiviruses* (ACMV) and *East Africa cassava mosaic geminiviruses* (EACMGV), by attacking the [ Zn ²⁺]-[CCHC] zinc finger domain or a variation thereof, such domain being present in the viral nucleocapsid or other viral ZFPs. The novel antiviral agents were designed to be used in plants infected by pathogenic viruses.

SEQUENCE LISTING

<110>  Fernandez-Pol, Jose A
       Fernandez-Pol, Sebastian

<120>  Tandem Repeat III

<130>  DN352

<140>  12/051,096
<141>  2008-03-19

<160>  22

<170>  PatentIn version 3.5

<210>  1
<211>  6
<212>  DNA
<213>  Artificial Sequence

<220>
<223>  Synthtetic


<220>
<221>  WRKY
<222>  (1)..(6)
<223>  WRKY promoter, functional in sythetic plant promoters

<400>  1
ttgacc                                                                 6


<210>  2
<211>  6
<212>  DNA
<213>  Petroselinum crispum


<220>
<221>  WRKY
<222>  (1)..(6)
<223>  W-box promoter functional in synthetic plant promoter, elicitor
       response element of Atcmpg1 gene

<400>  2
ttgact                                                                 6


<210>  3
<211>  7
<212>  DNA
<213>  Arabidopsis thaliana


<220>
<221>  AP2/ERF
<222>  (1)..(7)
<223>  Transgenic arabidopsis thaliana, synthetic plant promoter
       responsive to AP2/ERF, wound induced signal, functions in

signaling and transcriptional activation during plant pathogen
interactions

<400>  3
agccgcc                                                                 7


<210>  4
<211>  7
<212>  DNA
<213>  Petroselinum crispum


<220>
<221>  AP2/ERF
<222>  (1)..(7)
<223>  synthetic plant promoter responsive to AP2/ERF, pathogen and
       wound induced signaling, transcription responsive in plant
       pathogen interactions

<400>  4
agccacc                                                                 7


<210>  5
<211>  8
<212>  DNA
<213>  Arabidopsis thaliana


<220>
<221>  AP2/ERF
<222>  (1)..(8)
<223>  Transgenic arabidopsis thaliana, synthetic plant promoter
       responsive to AP2/ERF, wound healing promoter, transcription
       responsive in plant pathogen interaction

<400>  5
agaccacc                                                                8


<210>  6
<211>  5
<212>  DNA
<213>  Nicotiana tabacum


<220>
<221>  bZIP/NPR1
<222>  (1)..(5)
<223>  responder to transcription factor bZIP and to salicylic acid,
       bZIP/NPR1 promoter, promoter responds to oxidative stress

<400>  6
tgacg                                                                   5


<210>  7
<211>  12
<212>  DNA
<213>  French bean

<220>
<221>  Myb/bZIP/bHLH
<222>  (1)..(12)
<223>  unit consists of two elements, both required for function,
       synthetic promoter, G box and H box chalcone synthetic promoter,
       present in G box and H box of French bean

<400>  7
cctacccacg tg                                                          12


<210>  8
<211>  10
<212>  DNA
<213>  Nicotiana tabacum


<220>
<221>  PR-2d
<222>  (1)..(10)
<223>  transcription factors unknown, salicylic acid responsive
       promoter, pathogenic related promoter of 2d gene

<400>  8
ttcgacctcc                                                             10


<210>  9
<211>  6
<212>  DNA
<213>  Glycine max


<220>
<221>  GT1
<222>  (1)..(6)
<223>  responds to GT1 transcription factor, induced by pathogens, GT1
       box promoter

<400>  9
gaaaaa                                                                  6


<210>  10
<211>  12
<212>  DNA
<213>  Nicotiana tabacum


<220>
<221>  HRS203J
<222>  (1)..(12)
<223>  transgenic nicotiana tabacum, increase in hypersensitive
       response, promoter of tobacco gene HRS203J, pathogen responsive
       promoter

<400>  10
taaaattctt tg                                                          12

<210>  11
<211>  10
<212>  DNA
<213>  Arabidopsis thaliana

<220>
<221>  SR
<222>  (1)..(6)
<223>  Promoter binds to SR gene, calmodulin binding promoter, involved
       in plant defense mechanism, stress responsive proteins

<220>
<221>  misc_feature
<222>  (7)..(7)
<223>  n is an OR. This part of sequence represents G or T or C.

<220>
<221>  misc_feature
<222>  (9)..(9)
<223>  n is an OR. This part of sequence represents G or T or C.

<400>  11
vcgcggntnc                                                              10

<210>  12
<211>  14
<212>  DNA
<213>  Nicotiana tabacum

<220>
<221>  CEVI-1
<222>  (1)..(14)
<223>  promoter activated by DPB1 transcriptional factor which has
       phosphate activity, disease resistant promoter, involved in plant
       defense mechanism

<400>  12
taatatttgc cttt                                                         14

<210>  13
<211>  8
<212>  DNA
<213>  Nicotiana tabacum

<220>
<221>  CaMV
<222>  (1)..(8)
<223>  Synthetic plant promoter, W box promoter, disease resistant
       promoter, involved in plant defense, pathogen induced promoter

<400>  13
tatatagc                                                                 8

<210>  14

<211>  336
<212>  PRT
<213>  Homo Sapiens

<300>
<308>  Swiss protein bank/Q8TDX5
<309>  2004-01-10
<313>  (1)..(336)

<400>  14

Met Lys Ile Asp Ile His Ser His Ile Leu Pro Lys Glu Trp Pro Asp
1               5                   10                  15

Leu Lys Lys Arg Phe Gly Tyr Gly Gly Trp Val Gln Leu Gln His His
            20                  25                  30

Ser Lys Gly Glu Ala Lys Leu Leu Lys Asp Gly Lys Val Phe Arg Val
        35                  40                  45

Val Arg Glu Asn Cys Trp Asp Pro Pro Glu Val Arg Ile Arg Glu Met
    50                  55                  60

Asp Gln Lys Gly Val Ile Val Gln Ala Leu Ser Thr Val Pro Val Met
65                  70                  75                  80

Phe Ser Tyr Trp Ala Lys Pro Glu Asp Thr Leu Asn Leu Cys Gln Leu
                85                  90                  95

Leu Asn Asn Asp Leu Ala Ser Thr Val Val Ser Tyr Pro Arg Arg Phe
            100                 105                 110

Val Gly Leu Gly Thr Leu Pro Met Gln Ala Pro Glu Leu Ala Val Lys
        115                 120                 125

Glu Met Glu Arg Cys Val Lys Glu Leu Gly Phe Pro Gly Val Gln Ile
    130                 135                 140

Gly Thr His Val Asn Glu Trp Asp Leu Asn Ala Gln Glu Leu Phe Pro
145                 150                 155                 160

Val Tyr Ala Ala Ala Glu Arg Leu Lys Cys Ser Leu Phe Val His Pro
                165                 170                 175

Trp Asp Met Gln Met Asp Gly Arg Met Ala Lys Tyr Trp Leu Pro Trp
            180                 185                 190

Leu Val Gly Met Pro Ala Glu Thr Thr Ile Ala Ile Cys Ser Met Ile
        195                 200                 205

Met Gly Gly Val Phe Glu Lys Phe Pro Lys Leu Lys Val Cys Phe Ala
    210                 215                 220

His Gly Gly Ala Phe Pro Phe Thr Val Gly Arg Ile Ser His Gly Phe
225                 230                 235                 240

Ser Met Arg Pro Asp Leu Cys Ala Gln Asp Asn Pro Met Asn Pro Lys
                245                 250                 255

Lys Tyr Leu Gly Ser Phe Tyr Thr Asp Ala Leu Val His Asp Pro Leu
            260                 265                 270

Ser Leu Lys Leu Leu Thr Asp Val Ile Gly Lys Asp Lys Val Ile Leu
        275                 280                 285

Gly Thr Asp Tyr Pro Phe Pro Leu Gly Glu Leu Glu Pro Gly Lys Leu
    290                 295                 300

Ile Glu Ser Met Glu Glu Phe Asp Glu Glu Thr Lys Asn Lys Leu Lys
305                 310                 315                 320

Ala Gly Asn Ala Leu Ala Phe Leu Phe Leu Glu Arg Lys Gln Phe Glu
                325                 330                 335

<210>  15
<211>  74
<212>  PRT
<213>  Mungbean yellow mosaic, geminivirus, begomovirus, (MYMGV)

<220>
<221>  IR2a
<222>  (1)..(74)
<223>  In MYMGV it is located within position 242 to 466 in the complete
       sequence of MYMGV DNA A.

<300>
<308>  Gene Bank/991130 NP077085.1
<309>  1993-03-20
<313>  (1)..(74)

<400>  15

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser
    50                  55                  60

Leu Pro Arg Cys Arg Met Cys Gly Gly Gly
65                  70

<210>  16
<211>  63
<212>  PRT
<213>  Mungbean yellow mosaic, geminivirus, begomovirus, (MYMGV)

<220>
<221>  IR2b
<222>  (1)..(63)
<223>  In MYMGV it is located within positions 402 to 593 in the
       complete sequence of MYMGV DNA A.

<300>
<308>  Gene Bank/991131 NP 077086.1
<309>  1993-03-20
<313>  (1)..(63)

<400>  16

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                  40                  45

Thr His Val Glu Glu Thr Glu Ile Leu Ser Val Ile Pro Leu Ser
    50                  55                  60

<210>  17
<211>  244
<212>  PRT
<213>  Mungbean yellow mosaic, geminivirus, begomovirus, (MYMGV)

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein

<309>  1993-03-20
<313>  (1)..(244)

<400>  17

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn

<210>  18
<211>  45
<212>  PRT
<213>  Mungbean yellow mosaic, geminivirus, begomovirus, (MYMGV)

<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  18

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  19
<211>  83
<212>  PRT
<213>  Mungbean yellow mosaic, geminivirus, begomovirus, (MYMGV)

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  19

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  20
<211>  134
<212>  PRT
<213>  Mungbean yellow mosaic, geminivirus, begomovirus, (MYMGV)

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  20

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn

85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130

<210>  21
<211>  361
<212>  PRT
<213>  Mungbean yellow mosaic, geminivirus, begomovirus, (MYMGV)

<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  21

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  22
<211>  99
<212>  PRT
<213>  Mungbean yellow mosaic, geminivirus, begomovirus, (MYMGV)

<220>
<221>  AC4
<222>  (1)..(99)
<223>  In MYMGV it is located within positions 1620 to 2705 in the
       complete sequence of MYMGV DNA A

<300>
<308>  Gene Bank/991136 NP077091.1
<309>  1993-03-20
<313>  (1)..(99)

<400>  22

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr

SEQUENCE LISTING

<110>  Fernandez-Pol, Jose A
       Fernandez-Pol, Sebastian

<120>  Tandem Repeat III

<130>  DN352 based on DN299

<140>  12/051,096
<141>  2008-03-19

<160>  38

<170>  PatentIn version 3.5

<210>  1
<211>  6
<212>  DNA
<213>  Artificial Sequence

<220>
<223>  functional in synthetic plant promoters


<220>
<221>  promoter
<222>  (1)..(6)
<223>  WRKY promoter, functional in sythetic plant promoters

<400>  1
ttgacc                                                                 6


<210>  2
<211>  6
<212>  DNA
<213>  Petroselinum crispum


<220>
<221>  promoter
<222>  (1)..(6)
<223>  WRKY, W-box promoter functional in synthetic plant promoter,
       elicitor response element of Atcmpg1 gene

<400>  2
ttgact                                                                 6


<210>  3
<211>  7
<212>  DNA
<213>  Arabidopsis thaliana


<220>
<221>  promoter
<222>  (1)..(7)
<223>  AP2/ERF, Transgenic arabidopsis thaliana, synthetic plant
       promoter responsive to AP2/ERF, wound induced signal, functions
       in signaling and transcriptional activation during plant pathogen
       interactions

<400>  3
agccgcc                                                                7

<210>  4
<211>  7
<212>  DNA
<213>  Petroselinum crispum

<220>
<221>  promoter
<222>  (1)..(7)
<223>  AP2/ERF, synthetic plant promoter responsive to AP2/ERF, pathogen
       and wound induced signaling, transcription responsive in plant
       pathogen interactions

<400>  4
agccacc                                                                 7

<210>  5
<211>  8
<212>  DNA
<213>  Arabidopsis thaliana

<220>
<221>  promoter
<222>  (1)..(8)
<223>  Transgenic arabidopsis thaliana, synthetic plant promoter
       responsive to AP2/ERF, wound healing promoter, transcription
       responsive in plant pathogen interaction

<400>  5
agaccacc                                                                8

<210>  6
<211>  5
<212>  DNA
<213>  Nicotiana tabacum

<220>
<221>  promoter
<222>  (1)..(5)
<223>  bZIP/NPR1, responder to transcription factor bZIP and to
       salicylic acid, bZIP/NPR1 promoter, promoter responds to
       oxidative stress

<400>  6
tgacg                                                                   5

<210>  7
<211>  12
<212>  DNA
<213>  French bean

<220>
<221>  promoter
<222>  (1)..(12)
<223>  Myb/bZIP/bHLH, unit consists of two elements, both required for
       function, synthetic promoter, G box and H box chalcone synthetic
       promoter, present in G box and H box of French bean

<400>  7
cctacccacg tg                                                          12

<210>  8
<211>  10
<212>  DNA
<213>  Nicotiana tabacum


<220>
<221>  promoter
<222>  (1)..(10)
<223>  PR-2d, transcription factors unknown, salicylic acid responsive
       promoter, pathogenic related promoter of 2d gene

<400>  8
ttcgacctcc                                                              10


<210>  9
<211>  6
<212>  DNA
<213>  Glycine max


<220>
<221>  promoter
<222>  (1)..(6)
<223>  GT1, responds to GT1 transcription factor, induced by pathogens,
       GT1 box promoter

<400>  9
gaaaaa                                                                   6


<210>  10
<211>  12
<212>  DNA
<213>  Nicotiana tabacum


<220>
<221>  promoter
<222>  (1)..(12)
<223>  HRS203J, transgenic nicotiana tabacum, increase in hypersensitive
       response, promoter of tobacco gene HRS203J, pathogen responsive
       promoter

<400>  10
taaaattctt tg                                                           12


<210>  11
<211>  10
<212>  DNA
<213>  Arabidopsis thaliana


<220>
<221>  promoter
<222>  (1)..(6)
<223>  SR, Promoter binds to SR gene, calmodulin binding promoter,
       involved in plant defense mechanism, stress responsive proteins

<220>
<221>  misc_feature
<222>  (7)..(7)
<223>  n is an OR. This part of sequence represents G or T or C.

<220>
<221>  misc_feature

<222>  (9)..(9)
<223>  n is an OR. This part of sequence represents G or T or C.

<400>  11
vcgcggntnc                                                              10


<210>  12
<211>  14
<212>  DNA
<213>  Nicotiana tabacum


<220>
<221>  promoter
<222>  (1)..(14)
<223>  CEVI-1, promoter activated by DPB1 transcriptional factor which
       has phosphate activity, disease resistant promoter, involved in
       plant defense mechanism

<400>  12
taatatttgc cttt                                                         14


<210>  13
<211>  8
<212>  DNA
<213>  Nicotiana tabacum


<220>
<221>  promoter
<222>  (1)..(8)
<223>  CaMV, Synthetic plant promoter, W box promoter, disease resistant
       promoter, involved in plant defense, pathogen induced promoter

<400>  13
tatatagc                                                                 8


<210>  14
<211>  336
<212>  PRT
<213>  Homo Sapiens

<300>
<308>  Swiss protein bank/Q8TDX5
<309>  2004-01-10
<313>  (1)..(336)

<400>  14

Met Lys Ile Asp Ile His Ser His Ile Leu Pro Lys Glu Trp Pro Asp
1               5                   10                  15


Leu Lys Lys Arg Phe Gly Tyr Gly Gly Trp Val Gln Leu Gln His His
            20                  25                  30


Ser Lys Gly Glu Ala Lys Leu Leu Lys Asp Gly Lys Val Phe Arg Val
        35                  40                  45


Val Arg Glu Asn Cys Trp Asp Pro Pro Glu Val Arg Ile Arg Glu Met
    50                  55                  60

Asp Gln Lys Gly Val Ile Val Gln Ala Leu Ser Thr Val Pro Val Met
65                  70                  75                  80

Phe Ser Tyr Trp Ala Lys Pro Glu Asp Thr Leu Asn Leu Cys Gln Leu
                85                  90                  95

Leu Asn Asn Asp Leu Ala Ser Thr Val Val Ser Tyr Pro Arg Arg Phe
            100                 105                 110

Val Gly Leu Gly Thr Leu Pro Met Gln Ala Pro Glu Leu Ala Val Lys
        115                 120                 125

Glu Met Glu Arg Cys Val Lys Glu Leu Gly Phe Pro Gly Val Gln Ile
    130                 135                 140

Gly Thr His Val Asn Glu Trp Asp Leu Asn Ala Gln Glu Leu Phe Pro
145                 150                 155                 160

Val Tyr Ala Ala Ala Glu Arg Leu Lys Cys Ser Leu Phe Val His Pro
                165                 170                 175

Trp Asp Met Gln Met Asp Gly Arg Met Ala Lys Tyr Trp Leu Pro Trp
            180                 185                 190

Leu Val Gly Met Pro Ala Glu Thr Thr Ile Ala Ile Cys Ser Met Ile
        195                 200                 205

Met Gly Gly Val Phe Glu Lys Phe Pro Lys Leu Lys Val Cys Phe Ala
    210                 215                 220

His Gly Gly Ala Phe Pro Phe Thr Val Gly Arg Ile Ser His Gly Phe
225                 230                 235                 240

Ser Met Arg Pro Asp Leu Cys Ala Gln Asp Asn Pro Met Asn Pro Lys
                245                 250                 255

Lys Tyr Leu Gly Ser Phe Tyr Thr Asp Ala Leu Val His Asp Pro Leu
            260                 265                 270

Ser Leu Lys Leu Leu Thr Asp Val Ile Gly Lys Asp Lys Val Ile Leu
        275                 280                 285

Gly Thr Asp Tyr Pro Phe Pro Leu Gly Glu Leu Glu Pro Gly Lys Leu
    290                 295                 300

Ile Glu Ser Met Glu Glu Phe Asp Glu Glu Thr Lys Asn Lys Leu Lys
305                 310                 315                 320

Ala Gly Asn Ala Leu Ala Phe Leu Phe Leu Glu Arg Lys Gln Phe Glu
                325                 330                 335

<210>  15
<211>  74
<212>  PRT
<213>  Geminiviruses

<220>
<221>  IR2a
<222>  (1)..(74)
<223>  In MYMGV it is located within position 242 to 466 in the complete
       sequence of MYMGV DNA A.

<300>
<308>  Gene Bank/991130 NP077085.1
<309>  1993-03-20
<313>  (1)..(74)

<400>  15

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser
    50                  55                  60

Leu Pro Arg Cys Arg Met Cys Gly Gly Gly
65                  70

<210>  16
<211>  63
<212>  PRT
<213>  Geminiviruses

<220>
<221>  IR2b
<222>  (1)..(63)
<223>  In MYMGV it is located within positions 402 to 593 in the
       complete sequence of MYMGV DNA A.

<300>
<308>  Gene Bank/991131 NP 077086.1
<309>  1993-03-20
<313>  (1)..(63)

<400>  16

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                  40                  45

Thr His Val Glu Glu Thr Glu Ile Leu Ser Val Ile Pro Leu Ser
    50                  55                  60

<210>  17
<211>  244
<212>  PRT
<213>  Geminiviruses

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein
<309>  1993-03-20
<313>  (1)..(244)

<400>  17

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn

<210>  18
<211>  45
<212>  PRT
<213>  Geminiviruses

<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  18

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  19
<211>  83
<212>  PRT
<213>  Geminiviruses

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  19

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  20
<211>  134
<212>  PRT
<213>  Geminiviruses

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  20

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn
                85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130

<210>  21
<211>  361
<212>  PRT
<213>  Geminiviruses

<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  21

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  22
<211>  99
<212>  PRT
<213>  Geminiviruses

<220>
<221>  AC4
<222>  (1)..(99)
<223>  In MYMGV it is located within positions 1620 to 2705 in the
       complete sequence of MYMGV DNA A

<300>
<308>   Gene Bank/991136 NP077091.1
<309>   1993-03-20
<313>   (1)..(99)

<400>   22

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr


<210>   23
<211>   74
<212>   PRT
<213>   Mung bean yellow mosaic virus


<220>
<221>   IR2a
<222>   (1)..(74)
<223>   In MYMGV it is located within position 242 to 466 in the complete
        sequence of MYMGV DNA A.

<300>
<308>   Gene Bank/991130 NP077085.1
<309>   1993-03-20
<313>   (1)..(74)

<400>   23

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser

50                  55                  60

Leu Pro Arg Cys Arg Met Cys Gly Gly Gly
65                  70

<210>  24
<211>  63
<212>  PRT
<213>  Mung bean yellow mosaic virus

<220>
<221>  IR2b
<222>  (1)..(63)
<223>  In MYMGV it is located within positions 402 to 593 in the
       complete sequence of MYMGV DNA A.

<300>
<308>  Gene Bank/991131 NP 077086.1
<309>  1993-03-20
<313>  (1)..(63)

<400>  24

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                  40                  45

Thr His Val Glu Glu Thr Glu Ile Leu Ser Val Ile Pro Leu Ser
    50                  55                  60

<210>  25
<211>  244
<212>  PRT
<213>  Mung bean yellow mosaic virus

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein
<309>  1993-03-20
<313>  (1)..(244)

<400>  25

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn


<210>  26
<211>  45
<212>  PRT
<213>  Mung bean yellow mosaic virus


<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  26

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  27
<211>  83
<212>  PRT
<213>  Mung bean yellow mosaic virus

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  27

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  28
<211>  134
<212>  PRT
<213>  Mung bean yellow mosaic virus

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  28

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn
                85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130


<210>  29
<211>  361
<212>  PRT
<213>  Mung bean yellow mosaic virus


<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  29

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  30
<211>  99
<212>  PRT
<213>  Mung bean yellow mosaic virus

<220>
<221>  AC4
<222>  (1)..(99)
<223>  In MYMGV it is located within positions 1620 to 2705 in the
       complete sequence of MYMGV DNA A

<300>
<308>  Gene Bank/991136 NP077091.1
<309>  1993-03-20
<313>  (1)..(99)

<400>  30

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr

<210> 31
<211> 74
<212> PRT
<213> Begomovirus

<220>
<221> IR2a
<222> (1)..(74)
<223> In MYMGV it is located within position 242 to 466 in the complete
      sequence of MYMGV DNA A.

<300>
<308> Gene Bank/991130 NP077085.1
<309> 1993-03-20
<313> (1)..(74)

<400> 31

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser
    50                  55                  60

Leu Pro Arg Cys Arg Met Cys Gly Gly Gly
65                  70

<210> 32
<211> 63
<212> PRT
<213> Begomovirus

<220>
<221> IR2b
<222> (1)..(63)
<223> In MYMGV it is located within positions 402 to 593 in the
      complete sequence of MYMGV DNA A.

<300>
<308> Gene Bank/991131 NP 077086.1
<309> 1993-03-20
<313> (1)..(63)

<400> 32

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                  40                  45

Thr His Val Glu Glu Thr Glu Ile Leu Ser Val Ile Pro Leu Ser
    50                  55                  60

<210>  33
<211>  244
<212>  PRT
<213>  Begomovirus

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein
<309>  1993-03-20
<313>  (1)..(244)

<400>  33

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn

<210>  34
<211>  45
<212>  PRT
<213>  Begomovirus

<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  34

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  35
<211>  83
<212>  PRT
<213>  Begomovirus

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  35

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  36
<211>  134
<212>  PRT
<213>  Begomovirus

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  36

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn
                85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130

<210>  37
<211>  361
<212>  PRT
<213>  Begomovirus

<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  37

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  38
<211>  99
<212>  PRT
<213>  Begomovirus

<220>

<221>  AC4
<222>  (1)..(99)
<223>  In MYMGV it is located within positions 1620 to 2705 in the
       complete sequence of MYMGV DNA A

<300>
<308>  Gene Bank/991136 NP077091.1
<309>  1993-03-20
<313>  (1)..(99)

<400>  38

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr

SEQUENCE LISTING

<110>  Fernandez-Pol, Jose A
       Fernandez-Pol, Sebastian

<120>  Tandem Repeat III

<130>  DN352 based on DN299

<140>  12/051,096
<141>  2008-03-19

<160>  38

<170>  PatentIn version 3.5

<210>  1
<211>  6
<212>  DNA
<213>  Artificial Sequence

<220>
<223>  functional in synthetic plant promoters


<220>
<221>  promoter
<222>  (1)..(6)
<223>  WRKY promoter, functional in sythetic plant promoters

<400>  1
ttgacc                                                                 6


<210>  2
<211>  6
<212>  DNA
<213>  Petroselinum crispum


<220>
<221>  promoter
<222>  (1)..(6)
<223>  WRKY, W-box promoter functional in synthetic plant promoter,
       elicitor response element of Atcmpg1 gene

<400>  2
ttgact                                                                 6


<210>  3
<211>  7
<212>  DNA
<213>  Arabidopsis thaliana


<220>
<221>  promoter
<222>  (1)..(7)
<223>  AP2/ERF, Transgenic arabidopsis thaliana, synthetic plant
       promoter responsive to AP2/ERF, wound induced signal, functions
       in signaling and transcriptional activation during plant pathogen
       interactions

<400>  3
agccgcc                                                                7

<210>  4
<211>  7
<212>  DNA
<213>  Petroselinum crispum


<220>
<221>  promoter
<222>  (1)..(7)
<223>  AP2/ERF, synthetic plant promoter responsive to AP2/ERF, pathogen
       and wound induced signaling, transcription responsive in plant
       pathogen interactions

<400>  4
agccacc                                                                  7


<210>  5
<211>  8
<212>  DNA
<213>  Arabidopsis thaliana


<220>
<221>  promoter
<222>  (1)..(8)
<223>  Transgenic arabidopsis thaliana, synthetic plant promoter
       responsive to AP2/ERF, wound healing promoter, transcription
       responsive in plant pathogen interaction

<400>  5
agaccacc                                                                 8


<210>  6
<211>  5
<212>  DNA
<213>  Nicotiana tabacum


<220>
<221>  promoter
<222>  (1)..(5)
<223>  bZIP/NPR1, responder to transcription factor bZIP and to
       salicylic acid, bZIP/NPR1 promoter, promoter responds to
       oxidative stress

<400>  6
tgacg                                                                    5


<210>  7
<211>  12
<212>  DNA
<213>  French bean


<220>
<221>  promoter
<222>  (1)..(12)
<223>  Myb/bZIP/bHLH, unit consists of two elements, both required for
       function, synthetic promoter, G box and H box chalcone synthetic
       promoter, present in G box and H box of French bean

<400>  7
cctacccacg tg                                                           12

<210>  8
<211>  10
<212>  DNA
<213>  Nicotiana tabacum


<220>
<221>  promoter
<222>  (1)..(10)
<223>  PR-2d, transcription factors unknown, salicylic acid responsive
       promoter, pathogenic related promoter of 2d gene

<400>  8
ttcgacctcc                                                              10


<210>  9
<211>  6
<212>  DNA
<213>  Glycine max


<220>
<221>  promoter
<222>  (1)..(6)
<223>  GT1, responds to GT1 transcription factor, induced by pathogens,
       GT1 box promoter

<400>  9
gaaaaa                                                                   6


<210>  10
<211>  12
<212>  DNA
<213>  Nicotiana tabacum


<220>
<221>  promoter
<222>  (1)..(12)
<223>  HRS203J, transgenic nicotiana tabacum, increase in hypersensitive
       response, promoter of tobacco gene HRS203J, pathogen responsive
       promoter

<400>  10
taaaattctt tg                                                           12


<210>  11
<211>  10
<212>  DNA
<213>  Arabidopsis thaliana


<220>
<221>  promoter
<222>  (1)..(6)
<223>  SR, Promoter binds to SR gene, calmodulin binding promoter,
       involved in plant defense mechanism, stress responsive proteins

<220>
<221>  misc_feature
<222>  (7)..(7)
<223>  n is an OR. This part of sequence represents G or T or C.

<220>
<221>  misc_feature

<222>  (9)..(9)
<223>  n is an OR. This part of sequence represents G or T or C.

<400>  11
vcgcggntnc                                                              10


<210>  12
<211>  14
<212>  DNA
<213>  Nicotiana tabacum


<220>
<221>  promoter
<222>  (1)..(14)
<223>  CEVI-1, promoter activated by DPB1 transcriptional factor which
       has phosphate activity, disease resistant promoter, involved in
       plant defense mechanism

<400>  12
taatatttgc cttt                                                         14


<210>  13
<211>  8
<212>  DNA
<213>  Nicotiana tabacum


<220>
<221>  promoter
<222>  (1)..(8)
<223>  CaMV, Synthetic plant promoter, W box promoter, disease resistant
       promoter, involved in plant defense, pathogen induced promoter

<400>  13
tatatagc                                                                 8


<210>  14
<211>  336
<212>  PRT
<213>  Homo Sapiens

<300>
<308>  Swiss protein bank/Q8TDX5
<309>  2004-01-10
<313>  (1)..(336)

<400>  14

Met Lys Ile Asp Ile His Ser His Ile Leu Pro Lys Glu Trp Pro Asp
1               5                   10                  15


Leu Lys Lys Arg Phe Gly Tyr Gly Gly Trp Val Gln Leu Gln His His
            20                  25                  30


Ser Lys Gly Glu Ala Lys Leu Leu Lys Asp Gly Lys Val Phe Arg Val
        35                  40                  45


Val Arg Glu Asn Cys Trp Asp Pro Pro Glu Val Arg Ile Arg Glu Met
    50                  55                  60

Asp Gln Lys Gly Val Ile Val Gln Ala Leu Ser Thr Val Pro Val Met
65                  70                  75                  80

Phe Ser Tyr Trp Ala Lys Pro Glu Asp Thr Leu Asn Leu Cys Gln Leu
                85                  90                  95

Leu Asn Asn Asp Leu Ala Ser Thr Val Val Ser Tyr Pro Arg Arg Phe
            100                 105                 110

Val Gly Leu Gly Thr Leu Pro Met Gln Ala Pro Glu Leu Ala Val Lys
        115                 120                 125

Glu Met Glu Arg Cys Val Lys Glu Leu Gly Phe Pro Gly Val Gln Ile
    130                 135                 140

Gly Thr His Val Asn Glu Trp Asp Leu Asn Ala Gln Glu Leu Phe Pro
145                 150                 155                 160

Val Tyr Ala Ala Ala Glu Arg Leu Lys Cys Ser Leu Phe Val His Pro
                165                 170                 175

Trp Asp Met Gln Met Asp Gly Arg Met Ala Lys Tyr Trp Leu Pro Trp
            180                 185                 190

Leu Val Gly Met Pro Ala Glu Thr Thr Ile Ala Ile Cys Ser Met Ile
        195                 200                 205

Met Gly Gly Val Phe Glu Lys Phe Pro Lys Leu Lys Val Cys Phe Ala
    210                 215                 220

His Gly Gly Ala Phe Pro Phe Thr Val Gly Arg Ile Ser His Gly Phe
225                 230                 235                 240

Ser Met Arg Pro Asp Leu Cys Ala Gln Asp Asn Pro Met Asn Pro Lys
                245                 250                 255

Lys Tyr Leu Gly Ser Phe Tyr Thr Asp Ala Leu Val His Asp Pro Leu
            260                 265                 270

Ser Leu Lys Leu Leu Thr Asp Val Ile Gly Lys Asp Lys Val Ile Leu
        275                 280                 285

Gly Thr Asp Tyr Pro Phe Pro Leu Gly Glu Leu Glu Pro Gly Lys Leu
    290                 295                 300

Ile Glu Ser Met Glu Glu Phe Asp Glu Glu Thr Lys Asn Lys Leu Lys
305                 310                 315                 320

Ala Gly Asn Ala Leu Ala Phe Leu Phe Leu Glu Arg Lys Gln Phe Glu
                325                 330                 335

<210>  15
<211>  74
<212>  PRT
<213>  Geminiviruses

<220>
<221>  IR2a
<222>  (1)..(74)
<223>  In MYMGV it is located within position 242 to 466 in the complete
       sequence of MYMGV DNA A.

<300>
<308>  Gene Bank/991130 NP077085.1
<309>  1993-03-20
<313>  (1)..(74)

<400>  15

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser
    50                  55                  60

Leu Pro Arg Cys Arg Met Cys Gly Gly Gly
65                  70

<210>  16
<211>  63
<212>  PRT
<213>  Geminiviruses

<220>
<221>  IR2b
<222>  (1)..(63)
<223>  In MYMGV it is located within positions 402 to 593 in the
       complete sequence of MYMGV DNA A.

<300>
<308>  Gene Bank/991131 NP 077086.1
<309>  1993-03-20
<313>  (1)..(63)

<400>  16

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                  40                  45

Thr His Val Glu Glu Thr Glu Ile Leu Ser Val Ile Pro Leu Ser
    50                  55                  60

<210>  17
<211>  244
<212>  PRT
<213>  Geminiviruses

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein
<309>  1993-03-20
<313>  (1)..(244)

<400>  17

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn

<210>  18
<211>  45
<212>  PRT
<213>  Geminiviruses

<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  18

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  19
<211>  83
<212>  PRT
<213>  Geminiviruses

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  19

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  20
<211>  134
<212>  PRT
<213>  Geminiviruses

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  20

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn
                85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130

<210>  21
<211>  361
<212>  PRT
<213>  Geminiviruses

<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  21

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  22
<211>  99
<212>  PRT
<213>  Geminiviruses

<220>
<221>  AC4
<222>  (1)..(99)
<223>  In MYMGV it is located within positions 1620 to 2705 in the
       complete sequence of MYMGV DNA A

<300>
<308>  Gene Bank/991136 NP077091.1
<309>  1993-03-20
<313>  (1)..(99)

<400>  22

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr

<210>  23
<211>  74
<212>  PRT
<213>  Mung bean yellow mosaic virus

<220>
<221>  IR2a
<222>  (1)..(74)
<223>  In MYMGV it is located within position 242 to 466 in the complete
       sequence of MYMGV DNA A.

<300>
<308>  Gene Bank/991130 NP077085.1
<309>  1993-03-20
<313>  (1)..(74)

<400>  23

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser

50                  55                  60

Leu Pro Arg Cys Arg Met Cys Gly Gly Gly
65                  70

<210>  24
<211>  63
<212>  PRT
<213>  Mung bean yellow mosaic virus

<220>
<221>  IR2b
<222>  (1)..(63)
<223>  In MYMGV it is located within positions 402 to 593 in the
       complete sequence of MYMGV DNA A.

<300>
<308>  Gene Bank/991131 NP 077086.1
<309>  1993-03-20
<313>  (1)..(63)

<400>  24

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                  40                  45

Thr His Val Glu Glu Thr Glu Ile Leu Ser Val Ile Pro Leu Ser
    50                  55                  60

<210>  25
<211>  244
<212>  PRT
<213>  Mung bean yellow mosaic virus

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein
<309>  1993-03-20
<313>  (1)..(244)

<400>  25

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn


<210>  26
<211>  45
<212>  PRT
<213>  Mung bean yellow mosaic virus


<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  26

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  27
<211>  83
<212>  PRT
<213>  Mung bean yellow mosaic virus

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  27

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  28
<211>  134
<212>  PRT
<213>  Mung bean yellow mosaic virus

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  28

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn
                85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130

<210>  29
<211>  361
<212>  PRT
<213>  Mung bean yellow mosaic virus

<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  29

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  30
<211>  99
<212>  PRT
<213>  Mung bean yellow mosaic virus

<220>
<221>  AC4
<222>  (1)..(99)
<223>  In MYMGV it is located within positions 1620 to 2705 in the
       complete sequence of MYMGV DNA A

<300>
<308>  Gene Bank/991136 NP077091.1
<309>  1993-03-20
<313>  (1)..(99)

<400>  30

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr

<210> 31
<211> 74
<212> PRT
<213> Begomovirus

<220>
<221> IR2a
<222> (1)..(74)
<223> In MYMGV it is located within position 242 to 466 in the complete
      sequence of MYMGV DNA A.

<300>
<308> Gene Bank/991130 NP077085.1
<309> 1993-03-20
<313> (1)..(74)

<400> 31

Met Trp Asp Pro Leu Val Asn Asp Phe Pro Lys Ser Leu His Gly Phe
1               5                   10                  15

Arg Cys Met Leu Ala Ile Lys Tyr Leu Gln Tyr Ile Gln Ala Asn Tyr
            20                  25                  30

Pro Ser Asn Ser Leu Gly Tyr Val Tyr Leu Thr Glu Leu Ile Gln Val
        35                  40                  45

Leu Arg Ile Arg Lys His Ala Lys Ala Glu Leu Arg Ser Pro Pro Ser
    50                  55                  60

Leu Pro Arg Cys Arg Met Cys Gly Gly Gly
65                  70

<210> 32
<211> 63
<212> PRT
<213> Begomovirus

<220>
<221> IR2b
<222> (1)..(63)
<223> In MYMGV it is located within positions 402 to 593 in the
      complete sequence of MYMGV DNA A.

<300>
<308> Gene Bank/991131 NP 077086.1
<309> 1993-03-20
<313> (1)..(63)

<400> 32

Met Pro Lys Arg Asn Tyr Asp His Arg Leu Leu Tyr Pro Asp Val Glu
1               5                   10                  15

Cys Ala Glu Glu Ala Asp Leu Arg His Pro Ala Phe Leu Thr Cys His
            20                  25                  30

Cys Gly Lys Cys Pro Cys Gln Arg Glu Lys Glu Glu Val Asp Gln Pro
        35                      40                      45

Thr His Val Glu Glu Thr Glu Ile Leu Ser Val Ile Pro Leu Ser
    50                      55                      60

<210>  33
<211>  244
<212>  PRT
<213>  Begomovirus

<220>
<221>  AV1/CP
<222>  (1)..(244)
<223>  In MYMGV it is located within positions 442 to 1176 in complete
       sequence of MYMGV DNA A.

<300>
<308>  Gene Bank/991132 NP077087.1 coat protein
<309>  1993-03-20
<313>  (1)..(244)

<400>  33

Met Ser Asn Val Arg Arg Arg Leu Ile Phe Asp Thr Pro Leu Ser Leu
1               5                   10                  15

Pro Ala Thr Ala Gly Ser Val Pro Ala Ser Ala Lys Arg Arg Arg Trp
            20                  25                  30

Thr Asn Arg Pro Met Trp Arg Lys Pro Arg Tyr Tyr Arg Leu Tyr Arg
        35                  40                  45

Ser Pro Asp Val Pro Arg Gly Cys Glu Gly Pro Cys Lys Val Gln Ser
    50                  55                  60

Phe Glu Ala Lys His Asp Ile Ser His Leu Gly Lys Val Ile Cys Val
65                  70                  75                  80

Thr Asp Val Thr Arg Gly Asn Gly Ile Thr His Arg Val Gly Lys Arg
                85                  90                  95

Phe Cys Val Lys Ser Ile Trp Val Thr Gly Lys Ile Trp Met Asp Glu
            100                 105                 110

Asn Ile Lys Thr Lys Asn His Thr Asn Thr Val Met Phe Lys Leu Val
        115                 120                 125

Arg Asp Arg Arg Pro Phe Gly Thr Pro Gln Asp Phe Gly Gln Val Phe
    130                 135                 140

Asn Met Tyr Asp Asn Glu Pro Ser Thr Ala Thr Val Lys Asn Asp Leu
145                 150                 155                 160

Arg Asp Arg Tyr Gln Val Val Arg Lys Phe Gln Ala Thr Val Thr Gly
                165                 170                 175

Gly Gln Tyr Ala Ser Lys Glu Gln Ala Ile Val Ser Lys Phe Tyr Arg
            180                 185                 190

Val Asn Asn Tyr Val Val Tyr Asn His Gln Glu Ala Ala Lys Tyr Glu
        195                 200                 205

Asn His Thr Glu Asn Ala Leu Leu Leu Tyr Met Ala Cys Thr His Ala
    210                 215                 220

Ser Asn Pro Val Tyr Ala Thr Leu Lys Ile Arg Ile Tyr Phe Tyr Asp
225                 230                 235                 240

Ser Ile Ser Asn

<210>  34
<211>  45
<212>  PRT
<213>  Begomovirus

<220>
<221>  AL5
<222>  (1)..(45)
<223>  In MYMGV it is located within positions 570 to 707 in the
       complete sequence of MYMGV DNA A.

<300>
<308>  Gene Bank/991133 NP0770877.1
<309>  1993-03-20
<313>  (1)..(45)

<400>  34

Met Pro Leu Pro Arg Val Thr Ser Val Thr His Met Thr Leu Pro Arg
1               5                   10                  15

Cys Glu Ile Ser Cys Phe Ala Ser Lys Asp Trp Thr Leu Gln Gly Pro
            20                  25                  30

Ser Gln Pro Arg Gly Thr Ser Gly Glu Arg Tyr Asn Arg
        35                  40                  45

<210>  35
<211>  83
<212>  PRT
<213>  Begomovirus

<220>
<221>  AC5
<222>  (1)..(83)
<223>  In MYMGV it is located within positions 820 to 1071 in the
       complete sequence of MYMGV DNA A

<300>
<308>  Gene Bank/991134 NP077089.1
<309>  1993-03-20
<313>  (1)..(83)

<400>  35

Met Ile Phe Ile Leu Arg Cys Leu Leu Val Val Val Asn Asn Ile Ile
1               5                   10                  15

Val Asn Thr Ile Lys Phe Thr Asn Tyr Gly Leu Leu Leu Ala Arg Ile
            20                  25                  30

Leu Ala Thr Ser Asn Cys Gly Leu Lys Leu Thr His Asp Leu Ile Thr
        35                  40                  45

Ile Thr Lys Ile Val Leu His Ser Ser Ser Thr Gly Phe Ile Val Ile
    50                  55                  60

His Val Glu His Leu Thr Lys Val Leu Trp Arg Ala Lys Trp Thr Ser
65                  70                  75                  80

Val Thr His

<210>  36
<211>  134
<212>  PRT
<213>  Begomovirus

<220>
<221>  Ren/AC3
<222>  (1)..(134)
<223>  In MYMGV it is located within positions 1173 to 1577 in the
       complete sequence of MYMGV DNA A

<300>
<308>  Gene Bank/991135 NP077090.1
<309>  1993-03-20
<313>  (1)..(134)

<400>  36

Met Asp Phe Arg Thr Gly Asp Asn Ile Thr Ala Ala Gln Leu Arg Asn
1               5                   10                  15

Gly Val Phe Thr Trp Glu Val Arg Asn Pro Leu Ser Phe Lys Ile Met
            20                  25                  30

Gln His Arg Leu Ile Arg Pro Glu Ser Gln Met Tyr Val Thr Gln Ile
        35                  40                  45

Arg Ile Met Phe Asn His Gly Leu Lys Lys Ala Leu Leu Met His Lys
    50                  55                  60

Cys Phe Leu Asp Leu Lys Leu Tyr His Tyr Leu Thr Ala Thr Ser Gly
65                  70                  75                  80

Met Ile Leu Ser Ile Phe Ser Arg Gln Leu Phe Arg Tyr Leu Asn Asn
                85                  90                  95

Leu Gly Val Ile Ser Ile Gly Asn Val Leu Gln Gly Ala Ser His Ile
            100                 105                 110

Leu Tyr Glu Lys Leu His His Val Glu Asp Val Ser Phe Thr His Asn
        115                 120                 125

Val Gln Tyr Lys Ile Tyr
    130

<210>  37
<211>  361
<212>  PRT
<213>  Begomovirus

<220>
<221>  Rep
<222>  (1)..(361)
<223>  In MYMGV it is located within positions 1620 to 2705 in the
       complete sequence of MYMGV DNA A, potential NTP binding protein

<300>
<308>  Gene Bank/991136 NP 077091.1
<309>  1993-03-20
<313>  (1)..(361)

<400>  37

Met Pro Arg Leu Gly Arg Phe Ala Ile Asn Ala Lys Asn Tyr Phe Leu
1               5                   10                  15

Thr Tyr Pro Arg Cys Pro Leu Arg Lys Glu Asp Ala Leu Glu Glu Leu
            20                  25                  30

Leu Ala Leu Ser Thr Pro Val Asn Lys Lys Phe Ile Arg Val Cys Arg
        35                  40                  45

Glu Leu His Glu Asp Gly Glu Pro His Leu His Val Leu Leu Gln Phe
    50                  55                  60

Glu Gly Lys Phe Gln Thr Lys Asn Glu Arg Phe Phe Asp Leu Val Ser
65                  70                  75                  80

Ser Thr Arg Ser Ala His Tyr His Pro Asn Val Gln Ala Ala Lys Ser
                85                  90                  95

Ala Ser Asp Val Lys Ser Tyr Met Asp Lys Asp Gly Pro Val Val Asp
            100                 105                 110

His Gly Ser Phe Gln Val Asp Gly Arg Ser Ala Arg Gly Gly Lys Gln
        115                 120                 125

Ser Ala Asn Asp Ala Tyr Ala Glu Ala Leu Asn Ser Gly Ser Lys Leu
    130                 135                 140

Gln Ala Leu Asn Ile Leu Arg Glu Lys Ala Pro Lys Asp Tyr Ile Leu
145                 150                 155                 160

Gln Phe His Asn Leu Asn Cys Asn Leu Ser Arg Ile Phe Ser Asp Glu
                165                 170                 175

Val Pro Leu Tyr Val Ser Pro Tyr Ser Leu Ser Ala Phe Asp Lys Val
            180                 185                 190

Pro Ser Tyr Ile Ser Ser Trp Ala Ser Glu Asn Val Arg His Pro Ala
        195                 200                 205

Ala Pro Glu Arg Pro Ile Ser Ile Val Ile Glu Gly Asp Ser Arg Thr
    210                 215                 220

Gly Lys Thr Met Trp Ala Arg Ala Leu Gly Pro His Asn Tyr Leu Cys
225                 230                 235                 240

Gly His Leu Asp Leu Asn Ser Lys Ile Tyr Ser Asn Asp Ala Trp Tyr
                245                 250                 255

Asn Val Ile Asp Asp Val Asp Pro His Tyr Leu Lys His Phe Lys Glu
            260                 265                 270

Phe Met Gly Ala Gln Arg Asp Trp Gln Ser Asn Val Lys Tyr Gly Lys
        275                 280                 285

Pro Thr His Ile Lys Gly Gly Ile Pro Thr Ile Phe Leu Cys Asn Pro
    290                 295                 300

Gly Pro Lys Ser Ser Tyr Lys Glu Tyr Leu Asp Glu Ala Asp Asn Thr
305                 310                 315                 320

Ala Leu Lys Leu Trp Ala Ser Lys Asn Ala Glu Phe Tyr Thr Leu Lys
                325                 330                 335

Glu Pro Leu Phe Ser Ser Val Asp Gln Ser Ala Thr Gln Gly Cys Gln
            340                 345                 350

Glu Ala Ser Asn Ser Thr Leu Ser Asn
        355                 360

<210>  38
<211>  99
<212>  PRT
<213>  Begomovirus

<220>

<221>  AC4
<222>  (1)..(99)
<223>  In MYMGV it is located within positions 1620 to 2705 in the
       complete sequence of MYMGV DNA A

<300>
<308>  Gene Bank/991136 NP077091.1
<309>  1993-03-20
<313>  (1)..(99)

<400>  38

Met Lys Met Glu Asn Leu Ile Ser Met Phe Cys Phe Asn Ser Lys Gly
1               5                   10                  15

Ser Ser Lys Arg Arg Thr Lys Gly Ser Ser Thr Trp Phe Pro Gln Pro
            20                  25                  30

Asp Gln His Ile Thr Ile Arg Thr Phe Arg Gln Leu Lys Ala His Gln
        35                  40                  45

Met Leu Ser His Thr Trp Thr Lys Thr Glu Thr Ser Leu Thr Met Glu
    50                  55                  60

Val Ser Lys Ser Met Ala Asp Gln Leu Glu Glu Val Asn Ser Leu Pro
65                  70                  75                  80

Thr Thr Leu Met Pro Arg His Ser Thr Val Asp Pro Ser Tyr Arg Pro
                85                  90                  95

Ser Ile Tyr

## Description

### FIELD OF THE INVENTION

This invention relates to pharmaceutical agents for the treatment of viral diseases in plants and plant cells, and more specifically, to pharmaceutical agents containing picolinic acid, fusaric acid, and analogs and derivatives thereof. The instant invention also relates to the production of syngenic plants with increased resistance to viral infection by utilizing novel Tandem Repeated Sequence technology, denoted the **Cassette TRS Construct,** to increase production of Picolinic acid, Fusaric acid and derivatives thereof which attack and disrupt zinc finger proteins of pathogenic viruses. **The Cassette TRS Construct** technology can be used to treat a wide spectrum of plant and plant cell viral diseases mediated by zinc finger proteins or other metal-ion dependent proteins or enzymes.

It will be appreciated that various changes and modifications may be made in the methods described and illustrated in this invention without departing from the scope of the appended claims. For example, suitable preparations of other metal chelating compounds may be employed for the treatment of the viral diseases of plants using the chelating compounds added directly and externally (exogenously) to the plant, or endogenously produced by the TRS syngenic plants and plant cells. The preparations of this invention may be used alone or in combination with other preparations. Therefore, the foregoing specifications and accompanying drawings are intended to be illustrative only and should not be view in a limiting sense.

### BACKGROUND OF THE INVENTION

### ANTIVIRAL DRUGS

The majority of antiviral drugs used to control the spread of plant, animal, and human viruses are of limited use. The following factor is for the most part responsible for the failure of most of the antiviral drugs: The selection pressure of the drug generates viral mutants and the virus becomes resistant to the antiviral agents, resulting in new species of emerging viruses capable of leading to a new epidemic or pandemic. Similarly, antivirals used to control plant viruses results in resistance of the virus to the mutagenic antiviral drug. Furthermore, the antivirals used to experimentally control plant viruses are highly toxic and may damage the environment. The contamination of the plant with the antiviral mutagenic agent makes the plant inedible.

### DNA CONSTRUCTS AND METHODS TO ELIMINATE PATHOGENIC VIRUSES

A major goal is the production of crops with increased and durable resistance to a wide-spectrum of diseases. Experimental evidence exists that inducible promoters present in cassettes units which corresponds to a sequence able to bind to a specific viral transcription factor protein which is released when the pathogenic virus disassembles inside the infected cell.

Thus, viral promoters with specific sequences bind to specific pathogenic viral transcription factors necessary for viral replication. The promoters can be under combinatorial and permutation control, depending on the physical or chemical environment where they operate. It is highly likely that the promoters of the cassettes constructs of this invention will be most active in meristematic cells in all tissues, since these cells contain all the necessary factors and cofactors for cell duplication. Suitable promoters should switch an upstream gene on or off. Furthermore, a stringent requirement for the cassette construct is that both abiotic and biotic stress should be unable to activate the background expression of the transgene in the plant chromosome. The modular nature of the cassette constructs with both tandem repeat DNA promoters and genes will become apparent as the various examples are described.

In the case of plant resistance to pathogenic viruses, the combination of instructions in a string of DNA, with control elements connected in series and adapted to respond to a pathogenic viral transcription factor in a specific fashion, will result in the death of the pathogenic virus and the death of the infected cell.

The method herein described of operation of inducible resistant to the virus consist of activating in the cassette construct the viral promoter (s) responsive to the viral transcription factors in combination with the activation of a series of death genes in tandem repeat sequences.

Transgenic plants display, in certain instances, resistance, recovery (viral infected plants initially show systemic infection but newly develop leaves and roots become resistant to the invading virus. Susceptible phenotypes may succumb to systemic infection. In general, the resistant state is mediated at least in part at the cytoplasmic level by an activity that reduces the high steady state levels of RNA preventing the virus from replicating. It was shown that a cytoplasmic activity targets specific RNA sequences for inactivation. (Lindbo, J. A. et al., "Induction of a highly specific antiviral state in transgenic plants: Implications for regulation of gene expression and virus resistance", Plant Cell,, 5:1749-59 (1993). The low steady state RNA levels are due to post-transcriptional gene silencing.

The degradation mechanism is specific for the transcript that increases above the set point level for a given cell; and, if the transcripts that increase above the set point level, are a viral transgene, the virus resistance state is observed in the plant due to specific degradation of the virus RNAs targets.

There are numerous other naturally occurring and artificial mechanisms for degrading and disintegrating pathogenic viruses infecting plants, such as the preparation of specific cassettes that with certain viral resistant properties that will result in the protection of specific transgenic plant cells invaded by viruses either individually or systemically.

The present invention is directed to the production of pathogenic virus resistant plants by
transforming the plants with specific cassettes DNA constructs possessing the ability of destroying the invading virus and killing the cell (s) by the activation of associated death genes.

One way to defeat the survival strategy of viruses in plants is to use the **Cassette TRS Construct** technology of this invention to create plants and plant cells having a permanent resistance to viral infection. This resistance results from the introduction in the plant genome of a tandem repeated DNA sequence which encodes for an enzyme able to generate picolinic acid or derivatives thereof. The enzymatically produced picolinic acid, under the control of a strong viral promoter protein, can interact and disrupt essential ZFPs encoded by the invading virus, thus preventing the virus from replicating in the plant host cell. These TRS syngenic plants with increased virus resistance comprise the prevention of the expression of a plant protein such as the case of DnaJ-like proteins (a heat-shock ZFP) that interact with viral component that are rendered inactive. The **Cassette TRS Constructs** can be used to create TRS syngenic plants which can neutralized the powerful viral survival strategies, as clearly demonstrated in the Examples section of this invention.

The TRS syngenic plants in which the **cassette TRS construct** for resistance to viruses has been introduced are genetically stable and fertile. The TRS syngenic plants produce flowers and seeds that can be re-planted to produce new TRS syngenic plants resistant to viruses. Furthermore, the TRS syngenic plants of this invention are subjected to evolutive environmental pressures as if they were the wild-type counterpart. Since they contain no toxic exogenously or endogenously added agents, they are edible without further modifications.

The inventors believe TRS syngenic plants produced by the **cassette TRS construct** technology can be the solution to many of the plant viral infections which produce devastating famines in many parts of the planet. Many outstanding scientific institutions and large corporations are pursuing major genetic engineering projects that may benefit mankind once the technologies are established in the field, and proven to be safe and stable. The complexities of biological systems and the microenvironment demonstrate, however, that while the technology is sophisticated, solid and proven in the laboratory, it will not necessarily work in the fields. The failure to move from lab to field can have serious harmful consequences.

A recent example of catastrophic failure to control *Geminiviruses* by using unstable transgenic varieties of Cassava occurred in Africa in 2006. Pursuit of Disease-Resistant syngenic varieties of Cassava to help African farmers increase harvests and improve food security resulted in the experimentally unexpected demonstration that currently available transgenic plant technologies are unpredictable, most likely because the DNA constructs introduced at random in the plants are unstable and enable the invading viruses in the transgenic plants to recombine and generate a new type or species of virus.

A third way to defeat the survival strategy of viruses (resistance to drugs, instability of syngenics), is to use antiviral drugs that are non-toxic both to normal cells of the plant system being treated and to any life form in the environment in which the antiviral agent is released. This selective toxicity can be achieved by using pharmaceutical agents that are based or are derivatives of naturally occurring agents such as picolinic acid that attack specific elements of the virus intolerant to mutations such as ZFPs.

Lethal sequences for a large number of pathogenic viruses have been identified in two-third of all viruses, as the sequence of amino acids in a viral protein that create a motif denoted **Zinc Finger Protein (ZFP).** A third of viruses do not code for ZFPs; they, however, induce host cell ZFPs as their own transcription factors. ZFPs have been identified by the **US National Cancer Institute as targets for antiviral therapy** for these reasons. ZFPs sequences are highly conserved, and thus mutations in the zinc finger domain are lethal for the virus.

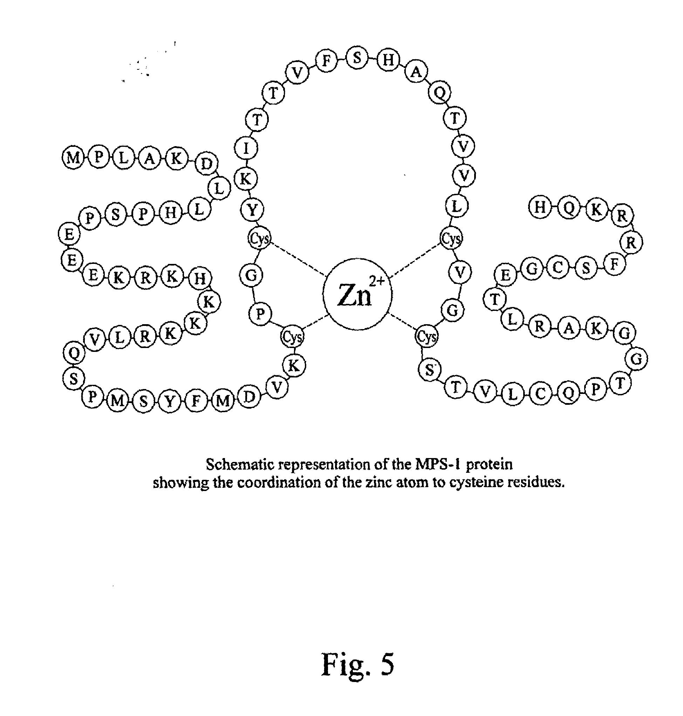

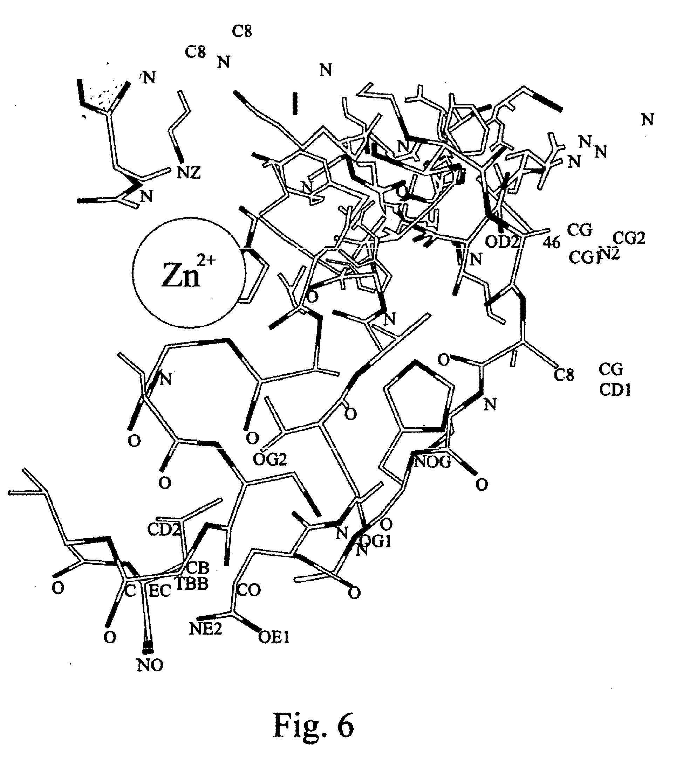

The biological significance and critical importance of ZFPs for viral survival is evident from an analysis of Gene Bank sequences. For example, the Gene Bank shows that the nuclecapsid (NC) proteins of Retroviridae contain sequences of 14 amino acids with 4 invariant residues, represented as: C_{ys}(X)₂Cys(X)₄His(X)₄Cys. This sequence chelates Zn ²⁺ through histidine imidazole and cysteine thiolates. The specificity of the binding of Zn ²⁺ to these chemical groups is shown by the fact that the Kd is less than 10⁻¹³ (Berg, JM, Science 232: 485 (1986). **(****FIG. 5** **and** **6****).** For example, these structures are denoted as CCCC (C4) ZFP and are very frequently observed in ribosomal ZFPs such as MPS-1/S27. In retroviruses, such as the AIDS viruses the zinc finger domain is of the type CCHC. Most viruses possess highly conserved viral zinc fingers. Examples of animal viruses possessing at least one C4 or CCHC zinc finger domain are shown in **Table 6 and 7. Table 8** shows examples of plants that can be infected with plant viruses containing ZFPs or use ZFPs from the host plant cells. Due to their highly conserved nature, the zinc finger domains perform essential functions in viral infectivity. Otherwise they would have never evolved as key factors in viral replication. In fact, it has been shown that mutations of the chelating residues (CCHC) in the zinc fingers produce a non-infectious virus. Viral ZFPs may have multiple functions. The HIV-1 nucleocapside protein (NCp7) has two zinc fingers of the CCHC type. It has been demonstrated that the two zinc finger domains are required for infectivity and also for packaging genomic RNA in the HIV-1.

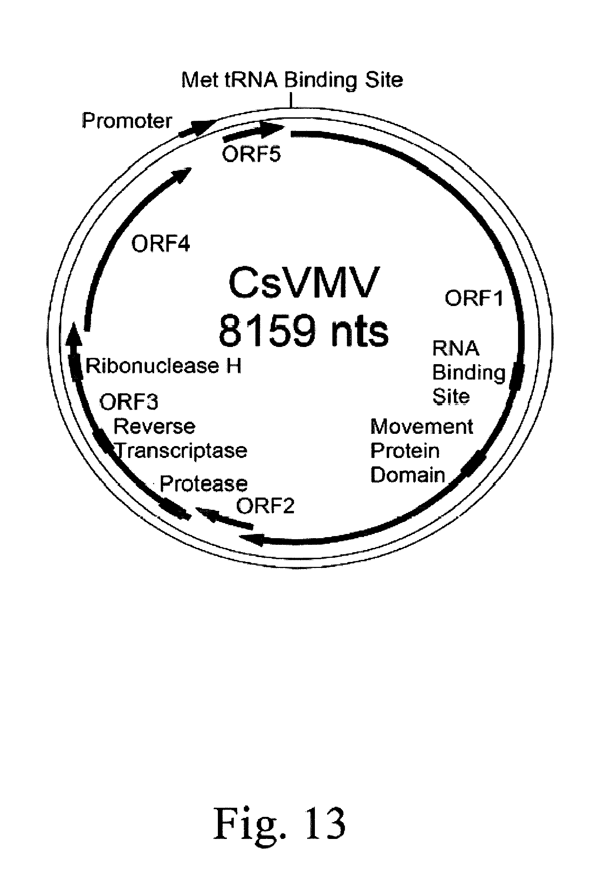

A gene bank DNA/protein sequence search of all known plant *Geminivirus* (complete genomic sequences of more than 60 are known from EMBO data base) and other plant viruses revealed that a number of plant viruses possess nucleoproteins with highly conserved zinc finger domains of several types such as C4, CCHC, CCHH or even more complex ZFP domains **(Table 1).** Examples of *Geminivirus* which possess zinc finger motifs in some of their proteins include, but are not limited to **(****FIG. 13****):** (1) *Tomato leaf curl* Bangalore [ToLCBV] virus [genus Begomovirus; family Geminiviridae]. The recombinant Coat Protein (CP) of TolCBV binds preferentially to ssDNA. A search for motifs responsible for ssDNA binding showed a conserved zinc finger motif in the CP (residues 65-85) of TolCBV. Site-directed mutagenesis of the conserved CCHH zinc finger motif [cysteines (C68, C72) and histidines (H81, H85)] conclusively showed that CCHH is responsible for binding of the CP protein to both Zn ²⁺ and ssDNA; (2) Recently, a zinc finger motif present in the C2 protein of TYLCV was shown to bind zinc and DNA and contribute to the function of C2 protein as a suppressor of posttranscriptional gene silencing; (3) A highly conserved zinc finger motif of the coat protein (CP) occurs only in the genus begomoviruses of the family Geminiviridae. This zinc finger motif present in the N-terminal region of begomoviral coat proteins is necessary for binding to ssDNA. It is thought that such interaction play an essential role in viral DNA encapsidation, DNA movement and DNA localization. (4) **Table III** shows the multiple alignments of representative begomovirus Coat Protein sequences in the region corresponding to the conserved zinc finger motif. Thus, the studies summarized here and other molecular studies of the zinc finger motif of the protein denoted CP of *Geminiviruses* (genus begomovirus) provide strong evidence that the zinc finger motif present in the N-terminal region of begomovirus coat proteins is a major target for antiviral therapy with novel anti-ZFP agents which should render the virus non-infectious. It has been discovered that mutations of the chelating residues in the zinc finger of CP yields a non-infectious *geminivirus,* possibly due to the fact that the CP of *geminivirus* is involved in viral ssDNA encapsidation, virus movement, and nuclear localization.

The genus *Begomovirus* of the family *Geminiviridae* are the most common viral disease affecting a myriad of plants. *Geminivirus* are the predominant viruses present in African Cassava mosaic virus (ACMV). Replication of the virus in the nucleus of plants under the control of the *Geminivirus* promoters destroys the transcriptional machinery of the plant nucleus and nucleolus, leading to Program Cell Death (PCD) and subsequent release of the virus into the circulatory system of the plant. The rapidly evolving role of *Geminiviruses,* as evidenced by synergism between ACMV and emerging double-recombinant infecting cassava in Cameroon and other parts of the world, reveals the urgency of the creation of novel methods and new antiviral agents to control a viral disease that may affect humanity in general. The unstable genetic materials utilized in attempts to control *Geminiviruses* and other plant diseases, intended to provide inhibition of growth or destruction of the virus have instead dramatically increased the growth conditions for numerous Begomoviruses to originate new recombinants of *geminiviruses,* leading to further spread of these dangerous viruses. Plant viruses data bases show that in certain regions the geminiviruses are evolving several orders of magnitude faster than before. These findings suggest the occurrence of synergistic interactions between viruses infecting both wild-type plants and unstable transgenic plants and the increase risk of widespread destruction of crops and propagation of Begomoviruses in agriculturally essential crops that feeds millions of people.

The purpose of this invention is the use of novel technologies to eliminate *Geminiviruses* by utilizing the intrinsic vulnerability of *Geminiviruses,* and other viruses that use ZFPs for survival. Finally, since the antiviral agents of this invention are non-toxic for plants, animals or human beings at the doses used in this invention, they are environmentally safe.

### PLANT VIRUSES AND ANTIVIRALS THAT TARGET ZINC FINGER PROTEINS

Previous research and inventions led the inventors to propose a theory of viral infection, prevention, and treatment applicable to both plant and animal cells. Essential viral and cellular Zinc Finger Proteins (ZFPs) and other metalloproteins can be targets for novel antiviral agents and for prevention and therapy of viral diseases. Furthermore, plants can be made resistant to pathogenic viruses by using the **Cassette TRS constructs** of this invention.

One of the purposes of this invention is to treat Cassava mosaic viral disease (and other viral diseases that affect most of the plants on this planet) with novel non-toxic antiviral agents. The work of the inventors in virology originates from work done by one of the inventors for more than 30 years in the area of animal cells transformed by viruses, and more recently in the area of plant cells exposed to physical and chemical agents with the purpose of defining the mechanisms of normal growth and transformed cell growth, in particular the integration of viruses in the genome of animal or plant cells.

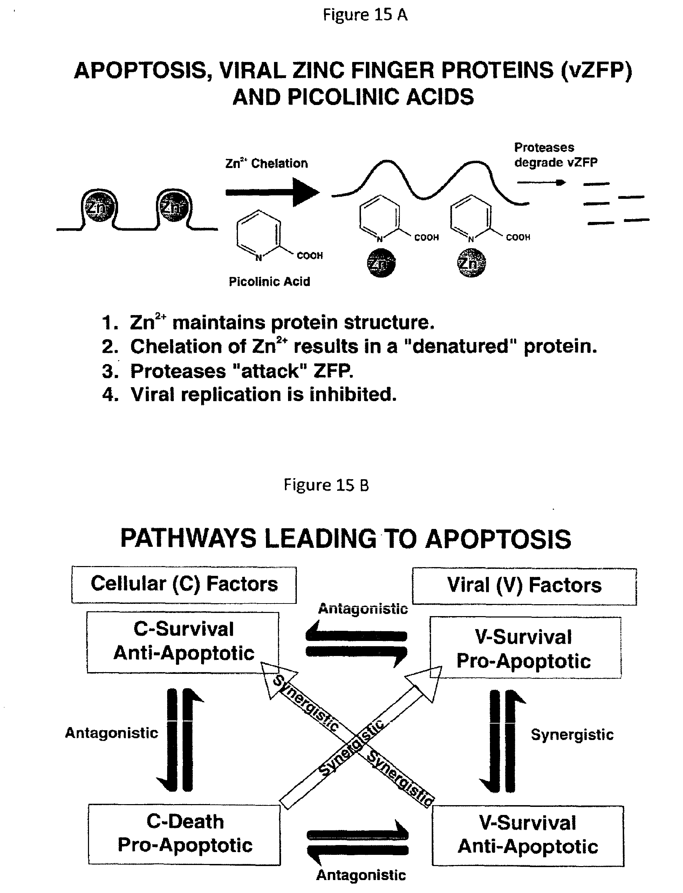

To clarify some aspects of the invention, a few general points are discussed as follows: (1) *Homeostasis between viruses and cells* **(****FIG. 15B****).** The field of plant viruses is ruled by the same physical, chemical - and up to certain extent - by the same biological principles of animal virus infection and dissemination of viruses in the infected organism; (2) *Proteomics.* Despite the fact that the information sequentially codified in the DNA or RNA of viruses sets the principles and characteristics of viral expression of proteins, the viral protein information carried in their amino acid sequences is the actual survival system of the virus. If a virus' combination of protein survival signals is deadly for the host, the virus will be hegemonic in its niche; lower predatory protein power might permit the virus to survive but not dominate. Thus, the currently denoted field of viral proteomics has the answers to the biggest questions in this field of virology; 3) **Zinc Finger Proteins (ZFP).** All animal and plant viruses posses DNA and RNA sequences that code for numerous Metalloproteins. From that group, a peculiar set of proteins denoted Zinc Finger Proteins (ZFP) play a key role in viral infection and viral replication; and 4) **Invariant Residues of ZFP are essential for the viral replication cycle and other critical viral functions.** In plant and animals ZFP have *invariant residues* that function to coordinate zinc binding to specific sequences. For example the following is a plant zinc finger protein of complex structure: X1-26-C27XXC28-X17-H46XXH47-X84. The Zinc Finger is formed by the binding of Zn2+ to the amino acids cysteine 27-28 and Histidines 46 and 47. The zinc finger provides to the mature protein a highly organized structure that facilitates nucleic acid binding *during almost every stage of the replication cycle of a virus.*

Animal and plant viruses typically divide within their host cells, when the host cells begin to duplicate. This shows that the viruses parasitically use the chemical components that are synthesized by the host cells in the **G1/S phases of the cell cycle**. Thus, despite the fact that they may invade all cells, **viruses duplicate in pre-mitotic or mitotically active cells.** In plants, the mitotically active cells in which most likely the viruses will divide and propagate are the cells denoted **meristematic cells.**

The antiviral compounds of this invention induce antiviral effects in virally infected cells that have started the program of cell division and viral division. In some infected cells in an early phase of viral infection, the virus is destroyed and the cell remains viable. If the number of viral particles in the cell is high, the picolinic acid and derivatives thereof attack not only the virus but also the host plant cells, zinc finger proteins. In this case, the end result is **Program Cell Death (PCD)** of host cells, and viral destruction of the intracellular virus by proteolytic enzymes. Thus, infected cells, may survive exposure to the antiviral agent, and survive virus-free, if treated sufficiently early.

In summary, the novel approach of the inventors of attacking conserved viral zinc finger proteins, highly expressed when duplicating in the cytoplasm or nucleus of the host plant cell, with specific agents that reach the viral replication site, can be used to eliminate *Geminivirus* and other plant viruses utilizing ZFPs for their replication strategy.

The present invention relates to the prevention and treatment of plant and plant cells which have been infected permanently by a virus resistant to elimination by currently utilized technologies, such as chemicals, genetic engineering methods, and transgenic methods in particular.

Numerous plant viruses infect agricultural plants essential to humanity's survival **(Tables 1, 8, and 9).** The viruses extensively damage crops each year. Safe and non-toxic antiviral agents for plant crops are currently unavailable. Thus, we continuously face the problem of wide-spread famine and the introduction of new mutagenic and carcinogenic agents into the environment. Moreover, while existing genetic engineering techniques promise the hope of controlling, eradicating, and terminating the infection of plants by pathogenic viruses in the near future, the promises of existing technology have not been fulfilled. In addition, there is the danger of further damage if something goes wrong with the genetic materials. It is highly likely that genetic materials produced under current technology and, used at present time, are unstable and may recombine with wild-type plant and viral genomes. As shown below in detail, *Geminiviruses* are particularly prone to mutate, recombine and integrate in the genomes of plants and plant cells, with the consequent emergence of new, more virulent pathogenic viral forms. Thus, currently different strategies are applied for the control of different pathogenic viruses. These strategies have been designed based on the type of replicating mechanism of the specific viral proteins and also the mode of infection by the virus. The approach embodied in this invention has the advantage of safety, specificity, broad (universal application), and effectiveness, and is non-toxic for animals, humans, and plants.

### Zinc finger ribosomal proteins: Metallopanstimulin (MPS-1) and extra-ribosomal functions of zinc finger ribosomal proteins related to viral infection.

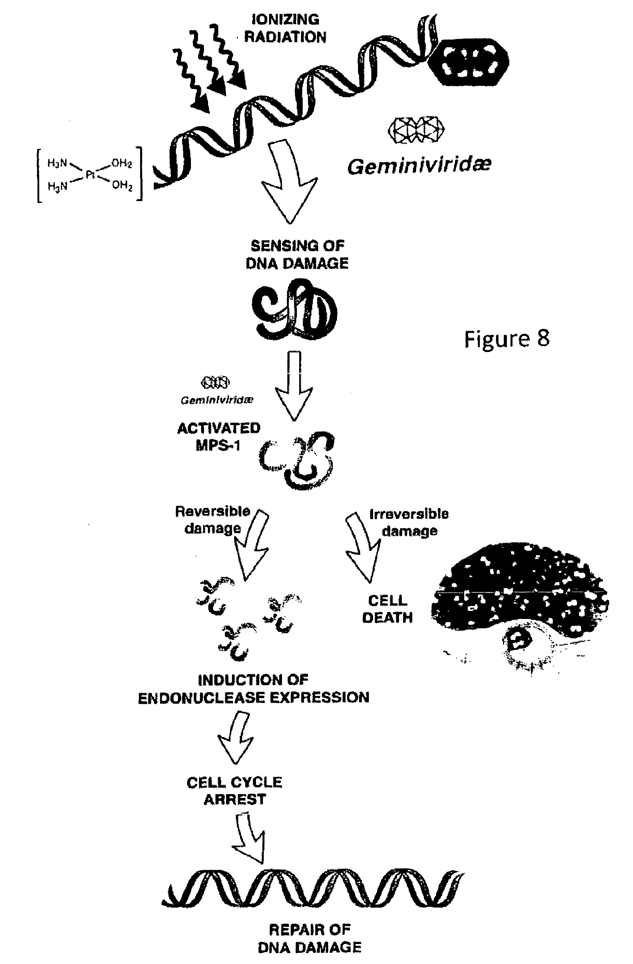

Fernandez-Pol *et al* have previously shown that metallopanstimulin/S27 (MPS-1) is a ubiquitous 9.4-kDa multifunctional ribosomal S27 protein, ZFP which is expressed at high levels in numerous virally infected cells and tissues. MPS-1 can be stimulated with specific growth factors and pathogenic viruses (Fernandez-Pol, 1992). MPS-1 is involved in normal cell growth and cancer cell growth of both animal and plant cells. The results of extensive experimentation by the inventors and other researchers determined that MPS-1 is involved in protein synthesis, repair of DNA, *elimination of mutated mRNA,* anti-apoptosis and rapid cell proliferation in both animal and plant cells. Thus, the information indicate that MPS-1 is a multifunctional zinc finger ribosomal protein involved, among other functions, in protection against viral infection and oncogenic processes *[elimination of mutated mRNA*] in both animal and plant cells (Fernandez-Pol, *et al;* Revenkova, *et al*) **(****FIGS. 8****,****9****,****10** **and** **11****).**

There are many reports indicating a connection between over expression of genes encoding some ribosomal proteins [proteins with or without zinc finger motifs] and viral transformation of cells of both animal and plant cells. Furthermore, a number of other ribosomal proteins have additional functions separated from both the ribosome and protein synthesis. Zinc finger motifs are characteristics of numerous ribosomal proteins, allowing them to tightly bind to nucleic acids [RNA; DNA] (Fernandez-Pol, *el al,* 1986, 2001, and 2004). These properties of ribosomal ZFP to interfere with both transcriptional and translational mechanisms during the process of viral infection, disassembly, assembly, and movement may be critical to the process of viral infection and propagation. Thus, in many instances it has been shown that extra ribosomal functions of numerous zinc finger ribosomal proteins are related to viral-induced oncogenesis. For example, the ribosomal protein S3a is identical to the product of the rat Fte-1 gene which encodes the viral-fos transformation oncogene (Fernandez-Pol, et al, 1994,2004).

In recapitulation, *overexpression* of certain ribosomal proteins is induced by viral genes which results in cellular transformation, mutations, PCD or apoptosis. Therefore, in conjunction with other cellular proteins such as DnaJs (heat shock proteins), ribosomal zinc finger proteins are key factors in the control of virus infection in both animal and plant cells. The agents of this invention which disrupt ribosomal ZFPs are useful in the control of plant and plant cells viral infection.

### Plant and plant cells with increased virus resistance and antiviral activity by increasing the production of Picolinic acid intracellularly which attack overproduction of both plant cell proteins and viral proteins under the control of strong viral promoters.

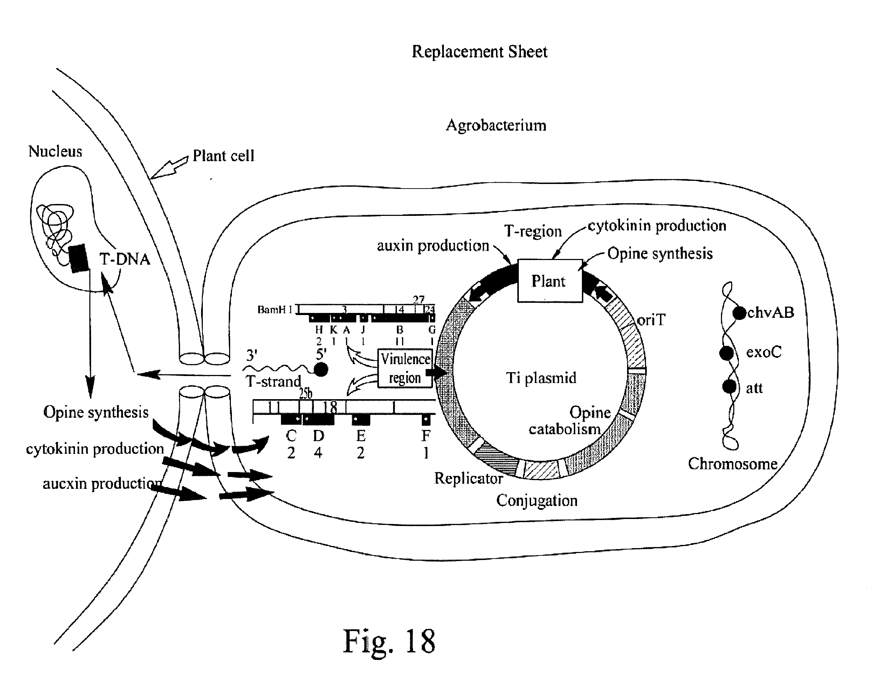

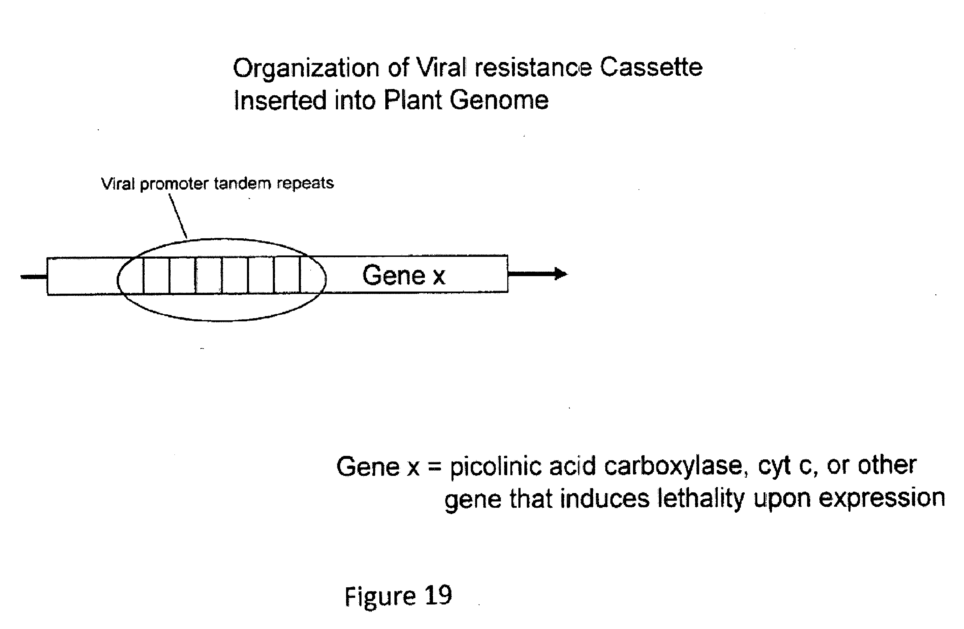

The invention also relates to plants and plant cells which can be designed to have either a transient or permanent virus resistance as a result of modulation of strong promoters of critical gene expression for the production of picolinic acid or derivatives thereof such as Picolinic Acid Carboxylase (PAC) in tandem repeated units. The enzymatic production of Picolinic acid or derivatives thereof can control the *overproduction* of plant ribosomal zinc finger proteins such as MPS-1/S27 or other ribosomal ZFP critical for the viral infection process. Furthermore, *overproduction* of heat shock DnaJ-like proteins which are ZFP, can be regulated and neutralized by the endogenous production of Picolinic acid by PAC. The syngenic plants and plant cells can be designed to respond to a strong viral promoter which is released by the virus during the infection and disassembly processes. This may be the promoter of choice, since PAC can be activated as soon as the virus releases the protein contents, effectively eliminating the virus and preventing any damage to adjacent plant cells. Other suitable strong promoters acting in the promoter DNA sequence that controls the enzyme that produces picolinic acid (PAC) may be equally useful. For example, a "constitutive plant promoter" (CPP) present in all tissues of the syngenic plant, are active under most environmental conditions, states of development or cell differentiation. The following are illustrative examples of promoters: mosaic virus from different origins; ribosomal 35S DNA sequence; 2' promoter from T-DNA of *Agrobacterium tumefaciens* (FIG. 18); Ubiquitin promoters; and the pEmu promoter. Plant tissue-specific promoters can be used to control enhanced expression of Picolinic Acid within a selected plant tissue, such as seeds, leaves, fruits, or roots. There are no restrictions in the combination of DNA sequences with different functions. Any combination of inducible, non-inducible, non-tissue specific, or organ specific can be use to control the expression of Picolinic acid and derivatives thereof by PAC.

It should be noted that pathogenic plant virus can activate PAC, by the practitioner applying this invention, which requires the design of the proper promoter for PAC. Strong promoters controlled in this fashion are those that respond rapidly to invasion by pathogenic viruses such as *Geminiviruses.* These viruses release viral ZFP that activate the PAC strong promoter, with the subsequent production of Picolinic acid or derivatives thereof in the presence of the appropriate enzyme substrate.

The invention also relates to methods for the production of TRS syngenic plants with increased *Geminivirus* resistance, wherein the expression of plant DnaJ-like proteins which interact with viral components for survival purposes, is inactivated by silencing the DnaJ-like proteins by Picolinic acid or derivatives thereof which will either eject the Zn2+ from the DnaJ or will form a ternary complex (PA-Zn-DnaJ) which will inactivate the DnaJ.

In summary, the invention relates to methods for the production of TRS syngenic plants and cells with increased virus resistance, as a result of the presence of a tandem repeated gene (PAC) that will produce PA intracellularly under the control of a viral protein promoter. As a result of the increased production of PA induced by the viral promoter (or other suitable promoter), the interaction of viral components with MPS-1/S27 and other ZFPs such as the heat shock DnaJ proteins can be prevented by the presence of picolinic acid and/or derivatives thereof. Furthermore, PA can inhibit or disintegrate ZFP from numerous viruses including *Geminivirus,* essentially eliminating the virus from the plant cell.

### TRS SYNGENIC PLANTS RESISTANT TO GEMINIVIRUSES PRODUCE INTRACELLULAR PICOLINIC ACID ONLY IN THE PRESENCE OF THE INVADING VIRUSES

The present invention also relates to plants and plant cells which have permanent *Geminivirus* resistance by the incorporation and modulation of intracellular production of Picolinic acid (PA) or derivatives thereof. The induction of increased production of PA [from uM to >3 mM] leads to several events, such as the inactivation of the ZFP of *Geminivirus;* neutralization of overproduction of plant DnaJ proteins induced and utilized by these viruses; and neutralization of overproduction of zinc finger ribosomal proteins required for viral replication. Methods for the production of such plants and plant cells will be illustrated in detail in Examples.

### DESCRIPTION OF RELATED ART RELEVANT TO THE INSTANT INVENTION.

This invention, produces TRS syngenic plants and cells with increased resistance and antiviral activity against *Geminiviruses,* using PAC to enzymatically increase intracellular production of picolinic acid or derivatives thereof from appropriate substrates.

This invention, vector(s) containing the nucleic acid sequences transferred to the plant cells are transcribed and translated, and producing an enzyme denoted Picolinic Acid Carboxylase (PAC). PAC interacts with a substrate (s) which is a precursor of the chelating agent picolinic acid or derivatives thereof. The PA then mediates both resistance and antiviral activity against *Geminiviruses* and other viruses depending upon ZFP for pathogenic effect.

With picolinic acid (PA) or fusaric acid (FU) being produced at high intracellular levels (PA = 1 uM to 3 mM; FU: 0.1 uM to 0.5 mM) viral activity decreases and the virus is disintegrated because PA or FU inhibit the following critical viral protein sequences: 1) viral capsid proteins (movement proteins) function to transport the virus inside the plant cells; 2) the systemic infection of the plant depends on viral capsid proteins; 3) during viral replication, the capsid proteins are unassembled, the zinc finger proteins of the virus are released, and PA or FU neutralizes the viral ZFP; 4) the viral capsid proteins are ZFP and thus are prevented from assembly into functional virus particles by the presence of PA, FU or derivatives thereof. Thus, the virus cannot propagate within the plant. It is not possible that a virus attacked in its critical **immutable** and therefore, *lethal zinc finger protein core designed elements* will be able to adapt to the lethal effects of picolinic acid or derivatives thereof.

One advantage of the TRS syngenic plants producing Picolinic acid or derivatives thereof, compared to transgenic plants, is that the TRS syngenic plants produces wide-**spectrum antiviral agents** able to destroy an invading virus, upon viral infection. Thus, Picolinic acid can attack any virus having a zinc finger protein in its structure or any virus which utilizes plant cell ZFP for specific viral functions (e.g. DnaJ, MPS-1/S27, etc). Viral (and cellular) ZFPs require zinc for specific functions such as replication, assembly and propagation in the plant vascular system. Picolinic acid effectively neutralizes all these functions, and renders the ZFPs inactive. The syngenic plant or plant cells endogenously produce the enzyme(PAC) which converts a precursor(s) of picolinic acid into the active **wide spectrum antiviral agent.**

Previously, plant resistance against viruses was induced by activating unspecific plant defense systems, such as in the case of the constitutive overexpression of salicylic acid (Verberne et al., 2000, Nat. Biotechn., 18: 779-783). De Fazio et al (Arch. of Virol. 63:3-4, 1980) have successfully used Virazole (Ribavirin) against tomato spotted wilt virus in tomato and tobacco plants. Ribavirin was most efficient in tomato plants. Furthermore, Sela (Naturwissenchaften, 70, July, 1983) have shown that Human Interferons can inhibit virus infection in plants.

There is an urgent need for the creation of novel methods for the production of syngenic plants with increased virus resistance. In particular, there is a need to produce syngenic plants resistant to a wide spectrum of virus groups. In the particular case of the instant invention, the targets are viral zinc finger proteins, and the antiviral agents are chelating agents of wide antiviral spectrum which attack zinc finger proteins. Thus, this invention addresses the problem of novel antiviral agents of wide spectrum with specific viral targets which are exogenously added to the plants or endogenously produced in the TRS syngenic plants with increased virus resistance.

There is evidence that Cassava plants are rapidly destroyed by evolving plant viruses in many areas of this planet. Thus, there is a need of controlling or eliminating both Cassava plant viruses and the newly emerging recombinant Geminiviruses infecting Cassava and other edible plants of commercial value.

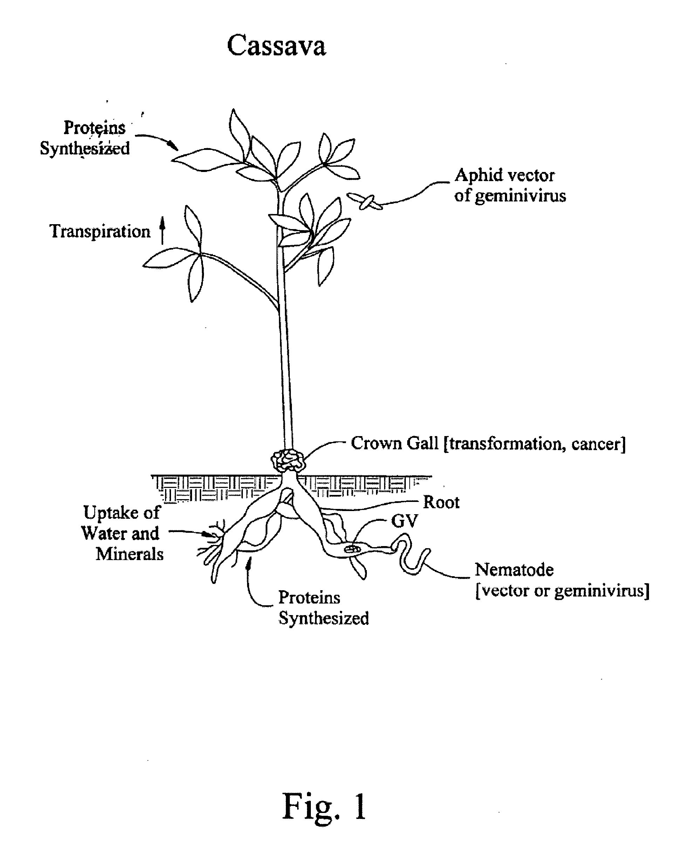

The Cassava *(Manihot esculenta)* is a woody shrub that is extensively cultivated as an annual crop in tropical and subtropical regions for its edible starchy tuberous root, which provides a major source of carbohydrate that can also be converted into alcohol **(****FIG. 1****). Cassava mosaic disease (CMD)** viral infection can be found in all areas including Africa, Brazil, India, and Sri Lanka where Cassava is commercially cultivated. Losses are estimated at one billion pounds sterling per year.

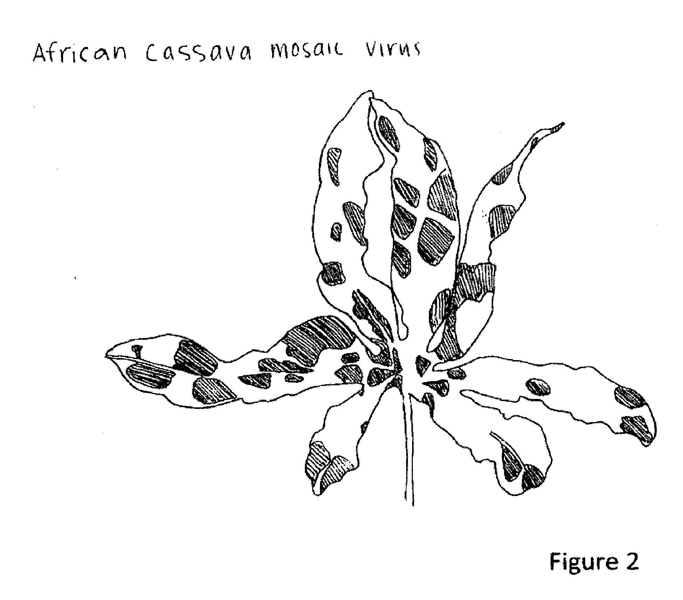

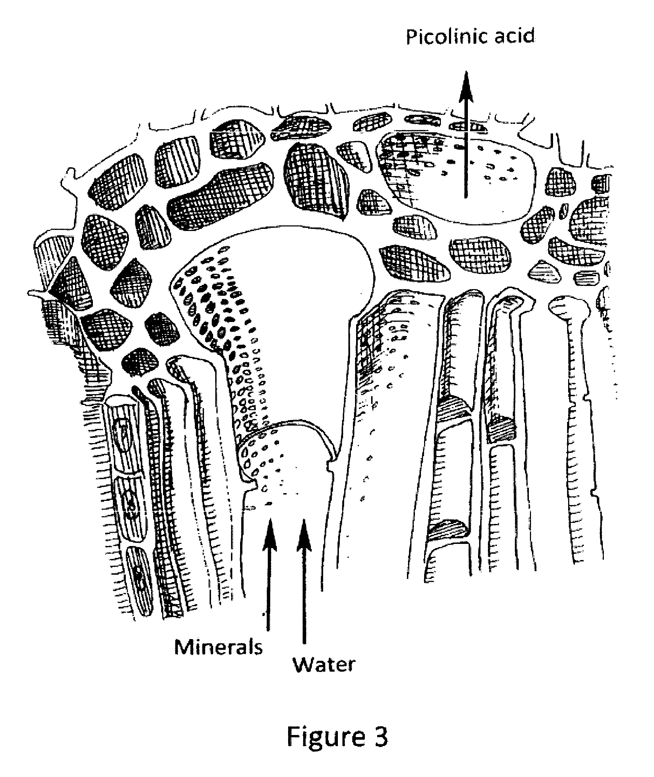

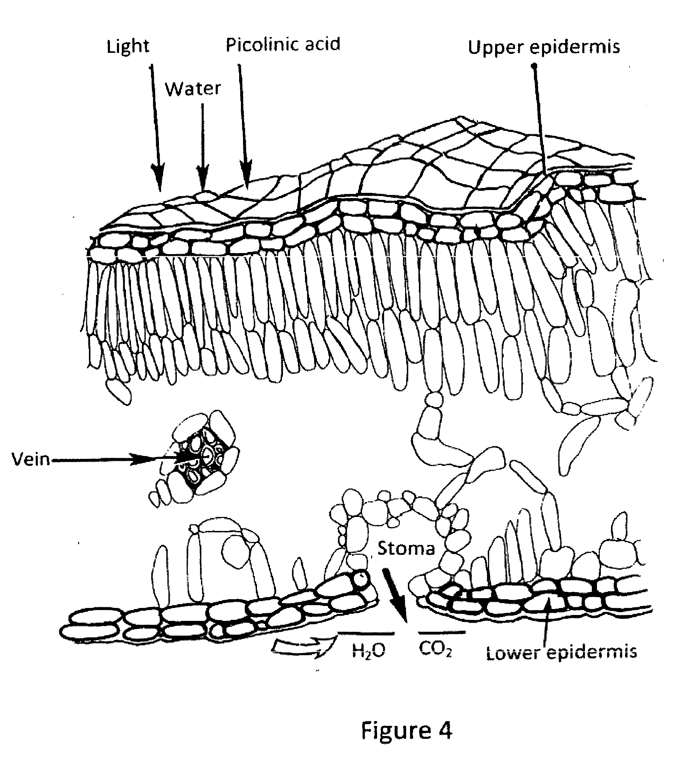

**FIG. 1** illustrates the infection of Cassava plants by *Geminivirus* vectors, an aphid and a nematode. **FIG. 2** is a drawing of the leaves of two different plant species infected by the African Cassava mosaic virus. **FIGS. 3** **and** **4** illustrate the transport and penetration of picolinic acid in the plants vascular system and leaves, respectively.

CMD is caused by viruses belonging to the genus ***Begomovirus*** of the family *Geminiviridae* **(Tables 2, 3 and 4).** The main characteristics of these viruses are: 1) small geminate particles; 2) particles contain circular single-stranded DNA molecules; 3) the viruses are transmitted by the whitefly *Bemicia Tabaci;* 4) the CDM is spread through the infected cuttings which are the usual mode of propagation. Swanson & Harrison (1994) identified three groups of Cassava mosaic viruses on the basis of their reaction to monoclonal antibodies.

In addition to the edible value of Cassava, the production of ethanol from this plant is of considerable importance for many environmental and medical reasons. For example, the eventual reduction in the consumption of petroleum will have many physical and chemical consequences, such as: 1) avoiding the excessive warming up of the planet ("green house effect") ; 2) eliminating the extensive and wide-spectrum pollution produced by petroleum and its by products, with grave consequences for the environment, and human beings (e.g. cancers, arrest of lung development in children, increased genetic mutations etc); and 3) destruction of the underground water supplies by detritus of petroleum covering the *aquifer.* Apart from food and pharmaceutical uses, ethanol from Cassava may be used as a biofuel. The Republic of Brazil, for example, depends entirely on alcohol to run its vehicles, although fermentation of sugar from Cassava is not the main source of this fuel. Cassava produces about 3000 to 4000 liters per day of alcohol at 96% tenor. The effluent of the processing of Cassava for production of alcohol can be disposed as animal feed.

Thus, in the absence of other alternatives, the inventors believe that the *Geminivirus* that attacks Cassava should be eliminated by using the less complex initial methods of this invention (exogenously added picolinic acid) and the safe and efficient Cassette TRS constructs designed by the inventors to increase the production of PA upon infection by a pathogenic plant virus. In conclusion, Cassava is a complex valuable plant that can be protected from damaging plant viruses by the methods of the instant invention.

### Geminate Structures of African Cassava Mosaic Virus

Like most animal DNA viruses, *geminiviruses* replicate in the nucleus. *Geminiviruses* require non-Structural Proteins (NSP) and Movement Proteins (MP) to move the viral genome across the nuclear envelope, the cytoplasm, and the plant cell wall. NSP is a nuclear transport protein that binds viral ssDNA and transports the ssDNA between the nucleus and proteins. The NSP nuclear shuttle protein also transports HIV RevT, adenovirus E1B-55 kd and E4-34 kd, and proteins with nuclear localization signals. The transport in plants by the NSP protein between the cytoplasm and the nucleus and vice-versa is analogous if not identical to the transport of proteins encoded by the RNA of human and animal viruses. The movement through the cell wall most likely is accomplished by channels (plasmodesmata) in the cell walls. Thus, the difference between plants and animal cells in transport systems between cells are more descriptive that real. The continuity of the cytoplasm of plant and animal cells allows distribution of the virus anywhere within and between cells. A pharmacological agent should by the same pathways also reach the same places anywhere within a cell.

The Begomovirus also contains packaging signals, short sequences or set of sequences that direct encapsidation of ssDNA. Simple viruses such as the Begomoviruses interact directly with capside proteins and the viral genome (ssDNA). They also contain some early proteins denoted zinc finger proteins.

Genomic packing of retroviruses has been extensively detailed by the inventors and others, and thus will not be explained in detail here. Suffice is to say that the HIV virus contains a nucleoprotein denoted Np7 which contains two zinc fingers. Nucleoprotein Np7 by means of the two zinc fingers impart additional order to the relatively long viral RNA, decreasing the entropy of this molecule to allow incorporation of the mRNA in a reduced space. (Fernandez-Pol, 1995). The inventors present in Examples the NMR spectroscopic studies related to this topic.

### A Novel Pararetrovirus: Cassava vein mosaic virus.

Recently a distinct plant *pararetrovirus* containing a Zinc Finger protein in its structure have been found to invade the vein system of Cassava with extensive pathologic effects. *Calvert et al* found that the Cassava vein mosaic virus (CVMV) was widespread throughout the north-eastern region of Brazil. One of the genomic proteins of 186 kDa has regions with zinc finger motifs. The Zinc Finger-like RNA-binding domains of 186 kDa protein are common elements in the *capsid proteins.* Furthermore, this ZFP have similarities to the *intercellular transport domain* of the plant pararetroviruses.

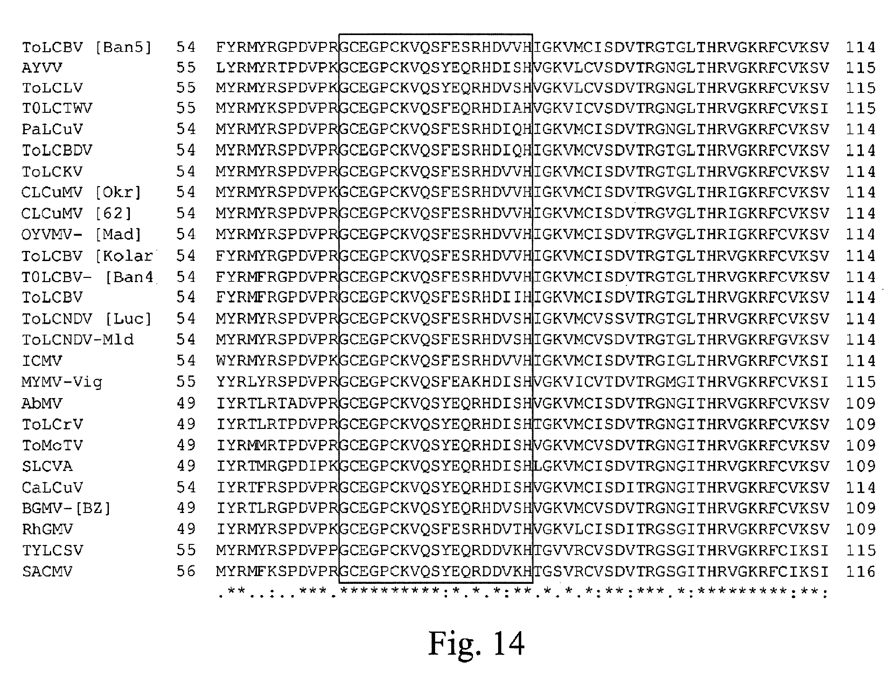

**Tables 2, 3 and 4 and** **FIGS 13** **and** **14****,** illustrate the genomic structure of the East African cassava mosaic Cameroon virus DNA A, East African cassava mosaic Zanzibar virus DNA B, and the complete genome of the Bhendi yellow mosaic virus, respectively. The genome of all these viruses is ssDNA. Tables 1, 2 and 3 are following.

### Conserved zinc finger motifs.

A conserved zinc finger (ZF) motif in the coat protein of the *Tomato leaf curl Bangalore* virus is responsible for binding to ssDNA **(****FIG. 14****).** The virus belongs to the genus Begomovirus of the family Geminiviridae. Kirthi *et al* determined that the Ban5 *Coat protein (CP)* synthesized in E. coli is characterized as the CP that binds to the ssDNA. A search for motifs responsible for ssDNA finding indicated a conserve putative ZF motif in the CPs (corresponding to residues 65-85 of Ban5 of Begomavirus **(****FIG.14****).** Site directed mutagenesis of the ZFPs cysteines and histidines conclusively indicated that the binding to zinc and DNA was due to such sequence.

As previously mentioned, Cassava mosaic disease (CMD) is caused by viruses belonging to the genus Begomovirus of the family *Geminiviridae,* which are characterized by small, geminate particles, containing circular, single-stranded DNA (ssDNA) molecules. The genomes of African Cassava mosaic virus (ACMV) and Indian Cassava mosaic virus (ICMV), are composed of two DNAs of similar size, denoted **DNA-A and DNA-B.** The complete nucleotide sequences of these virus species have been determined.

One way to defeat the survival strategy of plant *geminiviruses* which produce CMD is to develop antiviral agents that will focus on attacking specific protein sequences of the virus that are highly conserved and require zinc to maintain a proper functional configuration. In this invention, we have identified the ZFP motifs of *Geminiviruses* as the targets for the antiviral agents that can control CMD by inactivating, disintegrating and/or preventing further infections with the *Geminiviruses.*

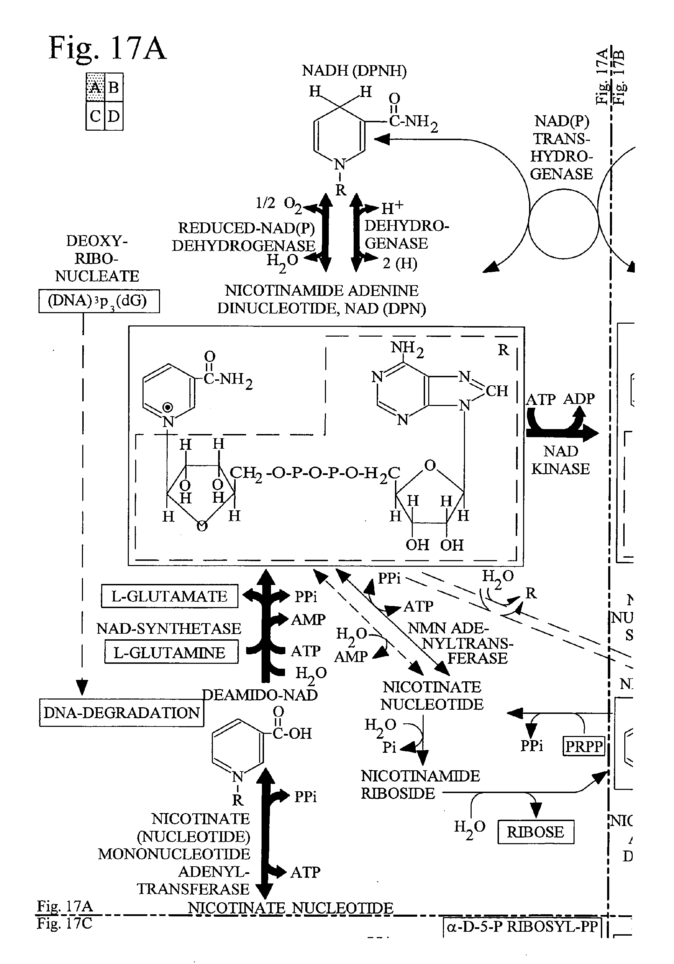

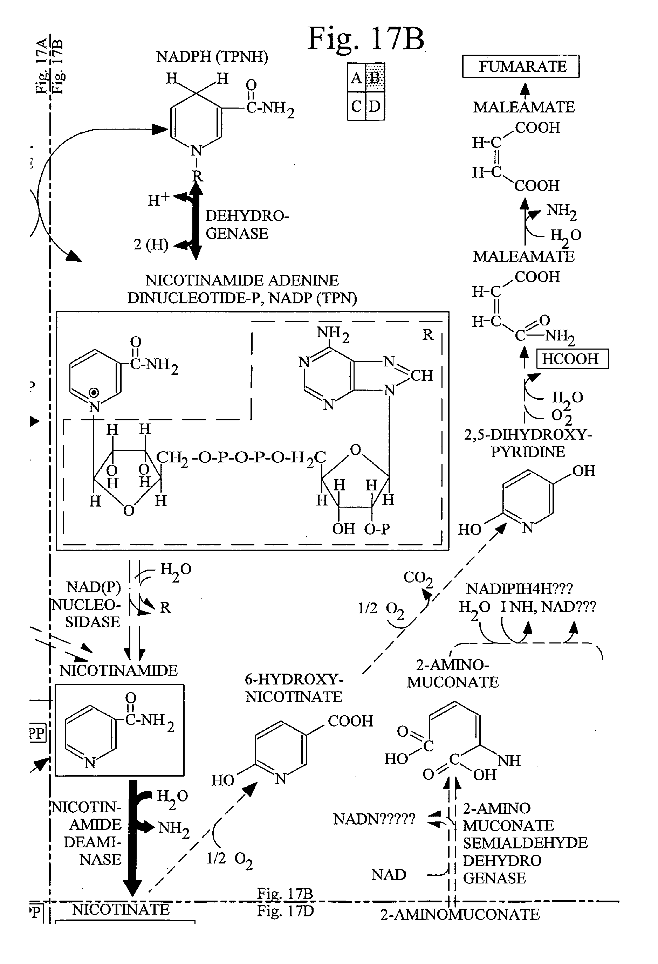

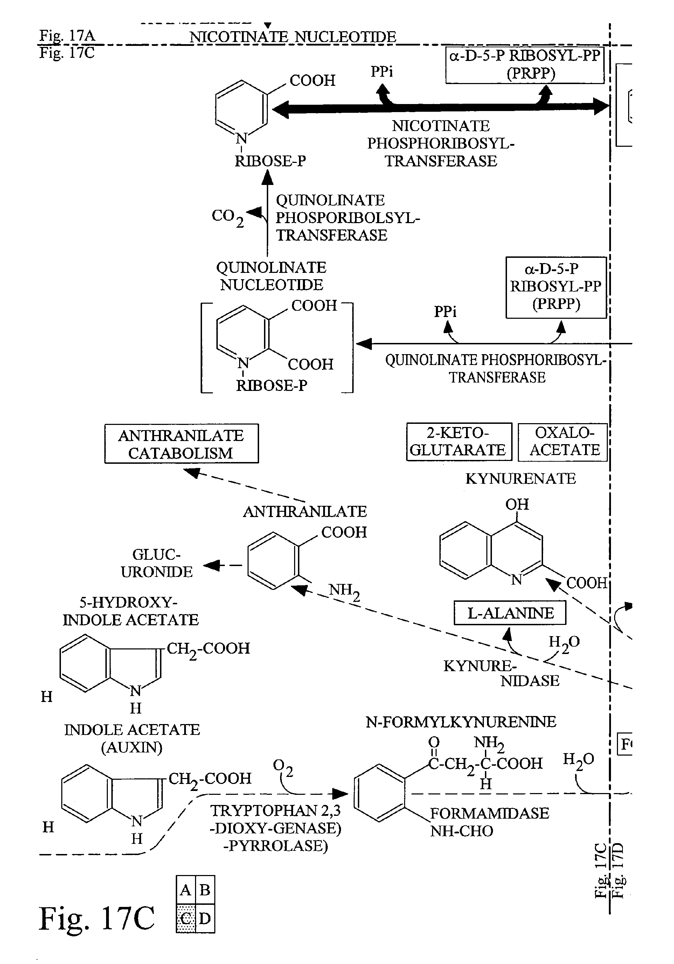

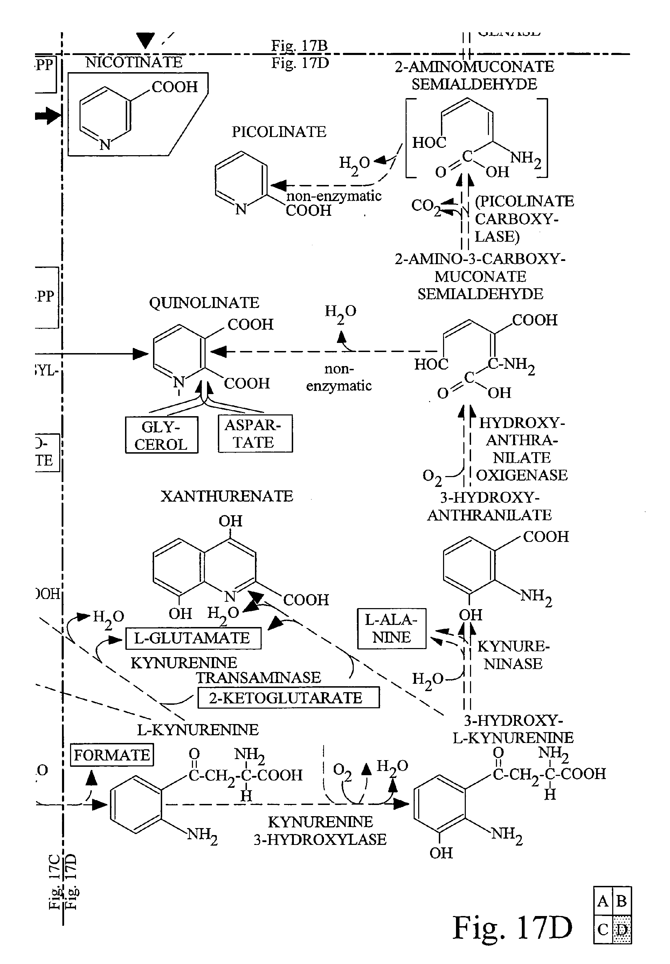

The antiviral agent, Picolinic acid (2-pyridine carboxylic acid) is a naturally occurring metal-chelating agent, and is also structurally related to nicotinic acid (3-pyridine carboxylic acid), a precursor in the biosynthesis of NAD⁺.**(****FIG. 17****).** Cells transformed (made cancerous) by different viruses that contain ZFP respond differently to picolinic acid. Flow Microfluorometric Analysis (FMF) of DNA demonstrated that the arrest induced by picolinic acid in Kirstern sarcoma virus transformed cells in the G₁ phase of the cell cycle. Normal rat kidney (NRK) cells were also arrested in G₁ but the points of arrest in the cycle were different. Numerous transformed cell lines (Fernandez-Pol, Proc. Natl. Acad. Sci. USA, 74:2889-2893, 1977) were exposed to PA and the points of arrest varied. Untransformed BALB 3T3, NRK and WI-348 were blocked in G₁. Two important facts emerged from studies with transformed cell lines. Cell transformed by different viruses responded differently to picolinic acid, but cell lines from different species transformed by the same virus were blocked by picolinic acid in similar manner. For details *see* Fernandez-Pol et al, Proc. Natl Acad. Sci. USA 774 (1977).

In addition to its effects on growth, picolinic acid has several biochemical actions related to the cell cycle: 1) PA can bind to and activate a transcriptionally active species of protein-metal ion complex in a manner similar to the binding of nicotinic acid to leghemoglobin (a plant protein); 2) PA csn alter the activity of poly (ADP-ribose) polymerase, the enzyme that catalyzes the polymerization of the ADP-ribose moiety of NAD+ - poly (ADP-ribose) levels, which are different in normal and cancer cells ; and 3) PA also alters the response to growth factors such as Epidermal Growth Factor (EGF), Transforming Growth Factors (TGFs), Nerve Growth Factor (NGF), Tumor necrosis factor (TNF), and numerous other critical growth factors; 4) In plant cells in culture media, picolinic acid interacts with plant cell hormones (denoted auxins) such as Zeatin, Kinetin, and Abscisic acid (Fernandez-Pol, Mol. Pathology, 1978), changing the plant cell metabolism, response to hormones and plant cell structure; and 5) in animal NRK cells treated with picolinic acid, prostaglandin E1 is two to five times more potent in elevating cyclic AMP, the mediator of the action to beta adrenergic agents (Fernandez-Pol, FEBS Letters, 1978).

In summary, the results of these extensive studies (PNAS, USA, 77; FEBS Letters, 1977) show that PA has effects on both the cell cycle and metabolism that are viral-transformation-dependent, may involved NAD+ metabolisms and most significant PA, may interact with the essential Zinc Finger Proteins in pathogenic viruses. The results of this study establish that PA has effects in cells that are transformation dependent. Furthermore, picolinic acid is a normal physiological substance that participates in regulatory physiologic mechanisms that are altered or damaged by viral transformation in plant and animal cells.

Based upon studies initiated by Dr. Fernandez-Pol in 1975, a novel concept developed about the control of cell growth and cell cycle by transition metal ions such as Zn, Fe and Cu, specific chelating agents and specific DNA/RNA binding proteins, which were later identified as metalloproteins and more specifically as zinc finger proteins.

The study of the control of growth in normal and virally transformed cells by utilizing a naturally occurring chelating agent, picolinic acid (2-pyridine carboxylic acid), led the inventors to the significant finding that the agent is an inhibitor of cell growth and reversibly arrests normal cells at a specific point in the cell cycle (G₁). In contrast, cells transformed by DNA or RNA viruses of different types showed growth arrest in different phases of the cell cycle (e.g.: S, G₂). The growth arrest was dependent upon the type of transforming virus. With further incubation in picolinic acid, the virally-transformed cells began to show signs of toxicity. Ultimately, cell death by apoptosis was observed in all transformed cells irrespective of the type of transforming virus. In contrast, normal cells showed no toxic effects from picolinic acid even with longer exposure to picolinic acid (one week) and the picolinic acid effects were reversibly. Most of the normal cells were arrested in the G₁ phase of the cell cycle.

Picolinic acid, fusaric acid, and derivatives thereof were shown to remove zinc from the eukaryotic CCCC zinc finger of Metallopanstimulin-1/S27 ribosomal protein. Further experiments demonstrated that picolinic acid and numerous derivatives thereof were able to selectively disrupt the binding of Zn to CCCC, CCHC, and numerous other viral zinc finger motifs. We have also shown the formation of ternary complexes among Zn²⁺, ZFP motifs, and picolinic acid (or other suitable analogue). The formation of the ternary complexes changed the configuration of the ZFPs which were subsequently degraded by proteolytic enzymes. NMR spectroscopy analysis of these experiments are presented in the instant invention in the section "Examples".

Support for the importance of this invention for the control of infections by plant viruses, viral and cellular ZFP, picolinic acids, virally transformed cells and plant viruses are reviewed in detail as follows:
Novel Metal-Chelating inhibitors of a ZFP of the HIV virus, denoted EP1 has a C2H2 type of ZFP domain which binds to DNA kB site present in the long terminal repeat of HIV provirus. Novel chelators comprising derivatives of both pyridine and aminoalkylthiol have significant inhibitory activity on the DNA of HIV-EP1. The inhibitory activity was increased 10-fold by the introduction of SH groups (Otsuka, M et al; ICR Annual Report, Vol. 3, 1996). Novel metal chelators can be designed to achieve the goal of silencing viral genes by inhibiting ZFP transcription factors.

Examples of specific effects of metal chelating agents, including picolinic acid, substitute picolinic acid derivatives and fusaric acid, as well as practical applications of these agents will now be described. Furthermore, to clarify the novel embodiments of this invention in the area of plant virology, details on Cassava infections, *Geminivirus,* plant responses to viruses, and treatment of *Geminivirus* diseases with the antiviral agents of this invention will now be described:

### PROTECTION OF PLANTS FROM PATHOGENIC VIRUSES BY PICOLINIC ACIDS

The cellular protective actions of the antiviral agents of this invention in plants such as *Arabidopsis* thaliana, and animal cell such as human *melanoma* after they were exposed to damaging agents, including chemical mutagens, UV radiation and pathogenic viruses were compared. Genotoxic responses to these damaging agents, in both plant and animal cells, involved complex repair processes. The antiviral agents were used to prevent cellular damage produced by the genotoxic agents. Specific descriptions of such experiments will be presented in Examples.

In the plant area, Revenkova *et al* (1996) studied the role of clones ofA. *thaliana,* which contained mutations in the ribosomal ZFP protein Metallopanstimulin-1/S27, which is involved in replication, protection against UV light, elimination of mutated mRNA, and destruction of viral mRNA (MPS-1 was discovered, cloned and isolated from human breast cancer cells by Fernandez-Pol, 1996). Mutations in MPS-1 resulted in apoptosis of *A. thaliana* plants, when exposed to genotoxic agents (Revenkova *et al;* 1996). Similarly, mutations in MPS-1 also resulted in apoptosis of human melanoma cells, when exposed to UV light. These results indicated, that one of the functions of MPS-1/S27 ribosomal ZFP is to eliminate mutated mRNA detrimental to both plant and animal cells. If not eliminated, such mutated mRNA can cause malignant transformation of the cells (Revenkova *et al* 1996; Fernandez-Pol *et al,* 1994).

Picolinic acid elicits hypersensitive-like response and enhances disease resistance in rice *(*Zhang et al; Cell Research (2004) 14, 27-33). Picolinic acid stimulates the protective response of rice plants to the deleterious effects of fungus, viruses and possibly bacteria. These results indicate that it may be feasible to protect any valuable crop for human consumption by using Picolinic Acid or derivatives thereof to elicit disease resistance.

### DISINTEGRATION OF VIRUSES BY DIFFERENT TYPES OF CHELATING AGENTS

**Previous art.** - It has been shown that non-specific chelating agents such as EGTA or EDTA can disintegrate Rotaviruses. In 1982, Wulderlich V. et al exposed *in vitro* various types of mammalian retroviruses to chelating agent such as EGTA or EDTA in millimolar concentrations. These physiological concentrations resulted in partial disintegration of viral membranes as measured by released of reverse transcriptase, an internal viral protein, without additional treatment required. Other important viruses that responded with disintegration to both chelators were mammalian hepatitis C viruses, primate D viruses and Bovine leukemia viruses. The response to the chelating agents was dose-dependent. The divalent cations appear to interact directly with the mammalian retrovirus structure. The viral disintegrating activity of EGTA or EDTA suggest that both Ca2+ and/or Mg2+ are integral constituent of viruses. These cations appear to be responsible for maintaining integrity of retroviral membranes which, after chelation of Ca2+ and/or Mg2+, are either disrupted or become permeable for the reverse transcriptase. (Archives of virology, 1982). Finally, chelating agents such as EDTA or EGTA, as well as chaotropic agents, dissociate the proteins and thus thermodynamically degrade their biological properties, inactivating the polymerase. As a consequence, the viral agents become non-infectious due to inactive polymerase.

Mammalian *rotaviruses* are also susceptible to the actions of chelating agents *in vitro.* When *rotaviruses* were exposed to EGTA or EDTA at millimolar concentrations, the rotaviruses showed partial disintegration of the viral membranes and released of Zinc Finger Proteins to the incubation media. It is noteworthy that EGTA or EDTA are preferentially chelators of Ca2+ and Mg2+, unlike Picolinic Acid and derivatives thereof which are chelators of transition metal ions (TMI) such as Zn²⁺, Fe²⁺, and Mn²⁺.

A novel cellular protein binding rotavirus NSP3 protein has been recently identified by Vitour et al (J Virol. 2004, 78:3851-62*).* This 110-kDa cellular protein, named RoXaN (rotavirus X protein associated with NSP3), contains a minimum of three regions predicted to be involved in protein-protein or protein-nucleic acid interactions. In the carboxyl terminus, *at least five zinc finger domains were observed.* RoXaN is a novel cellular protein containing zinc finger motifs detected in rotavirus infected cells. Thus, implicating RoXaN in translational regulation. These rotaviruses-induced cellular ZFPs, which are required for propagation of infection, may be useful targets to inhibit rotaviruses by disrupting zinc finger protein motifs.

### REGULATION OF TRANSGENE EXPRESSION IN PLANTS WITH POLYDACTYL ZINC FINGER TRANSCRIPTION FACTORS

Zinc finger transcription factors are among the most common transcription factors both in plants and animal cells. There are about 800 genes encoding such factors in the human genome. Fernandez-Pol *et al* (1993) experimentally utilized genes such as MPS-1/S27 zinc finger ribosomal protein to control the expression of the Metallopanstimulin MPS-1/S27 gene located in chromosome 1. In human breast cancer cells, certain fusion constructs of MPS-1 with an additional 17 amino acids to the 84 aa of MPS-1 (MPS-1/p17) arrested cell growth of breast cancer cells in tissue culture. The same MPS-1/p17 fusion protein prevented the stimulation with Epidermal Growth Factor (EGF) of melanoma cells in culture, in effect silencing the actions of the MPS-1/S27 ribosomal gene. MPS-1/p17 can be used to regulate transgene expression in animal cells (Fernandez-Pol, 1995).

Ordiz et al (2002) regulated transgene expression in plants with polydactyl zinc finger transcription factors. More recently, synthetic transcription factors based on the assembly of multiple zinc finger domains have been developed. Polydactyl zinc finger transcription factors can be used to regulate gene expression in plant cells. Transgene expression was regulated using a well characterized *polydactyl six-zinc finger protein* referred to as 2C7. The results suggest that the synthetic proteins may have applications in agricultural biotechnology. It is unclear, however, if they will be able to penetrate the plant cells or the nucleus in its present construction forms.

### SILENCING OF GENE FUNCTION OF ZINC FINGER PROTEINS: INHIBITION OF VIRUS ASSEMBLY

There are many ways to neutralize, disintegrate, eliminate or suppressed permanently a gene required by a plant pathogenic virus to complete its deleterious cycle for propagation inside the plant. One of the most interesting and effective is the method that follows.

Cellular and Virally Encoded Metalloproteins: Zinc containing Zinc Finger Proteins (ZFP) and ZFP in which the Zinc was replaced by iron: The "Iron Finger Proteins" Can Silence Specific DNA Response Elements (DNA-RE)

Ribosomes are essential in the process of viral protein synthesis. Some of the ribosomal proteins are ZFP with special functions. They are instrumental in the complex *hyper cycles* of protein synthesis induced by viral infection. Hyper cycles control the replication of viruses inside the host cells. A hyper cycle consists of interlocked feedback loops. The combined status of these cycles determines the proportions at which the viral components are generated, and thus the rate of viral replication. *Because errors accumulate during the hyper cycle, the virus has a tendency to mutate.* All viruses depend on their ability to infect cells and induce them to make more virus particles. If the virus is successful, the cell almost invariably dies in the process. This process is called "program cell death (PCD) or apoptosis". The DNA or RNA of viruses is always surrounded by a protein shell, or **capsid,** composed of nucleocapsid proteins. Some capsid proteins are metalloproteins or ZFP.

Conte et al (J. Biol. Chem; 271: 5125-5130, 1996) demonstrated the ability of cobalt and cadmium to structurally reconstitute the zinc finger motif of the ERDBD (Estrogen Receptor -DNA-Binding Domain). Conte et al established the ability of iron (Fe2+/Fe3+) to generate highly reactive free radicals and the increased requirement for iron by rapidly proliferating virally transformed and other malignant cells.

The inventors have found that in the ribosomal ZFP MPS-1, the Zn2+ can be replaced by Fe2+, generating an "iron finger protein". After dimerization of MPS-1, the iron in the "iron finger protein" MPS-1 protein can generate free radicals (hydroxyl). When the "iron finger protein" binds to the DNA-binding domain, the free radicals degrade the DNA genetic regulatory response elements.

Ribosomal ZFPs such as MPS-1/S27 can replace the zinc with iron and the resulting iron finger protein can generate free radicals (hydroxyl) which degrade the cells' DNA Genetic Regulatory Response Elements (GRRE) to which the new iron finger protein is specifically bound. Degradation of cell's DNA GRRE by iron finger proteins bound to DNA can be accomplished, by the presence of infecting viruses, by chemical agents such as Ascorbate + H₂O₂, by UV light, and by other means. In summary, the free radicals generated by the redox of Fe ²⁺/Fe³⁺ degrade DNA GRRE required for viral replication, and the invading virus-controlled, zinc finger DNA-binding protein becomes unable to function, stopping the virus from replication.

The following is an example of degradation of a specific DNA Response Element: 1) In a ZFP, such as MPS-1, the Zn2+ is replaced by Fe2+ (Fe2+/Fe3+= redox); 2) The "iron-finger protein" contacts with the specific DNA sequence recognized by MPS-1 in the plant cell DNA response element; 3) after contact of the complex ZFP-Fe2+-DNA specific sequence with added ascorbic acid, the deleterious free radical OH is generated by the redox of the Fe2+/Fe3+; 4) *The target DNA is specifically cut at the points of MPS-1 binding contacts in a **non-random** manner;* 5) the DNA is cut in ladders that correspond to the algorithms of the binding of MPS-1 to the DNA response element; thus, 6) the MPS-1 gene response element is silenced and becomes non-functional because it cannot neutralize the deleterious genotoxic effects of the free radicals OH- . Considering that animal and plant viruses utilize ribosomal and extra-ribosomal functions of zinc finger ribosomal proteins, it is highly likely that this process will cause plant viruses to be unable to assemble and thus degraded by proteases. This method was designed by the inventors to eliminate ZFP response elements that control genes coding for ZFPs involved in survival of pathogenic viruses, such as *Geminiviruses.*

In summary, numerous experiments showed that ZFP can strongly bind to DNA response elements (DNA-RE) specifically and either stimulate or inhibit cell growth. The second scenario is more attractive from the point of view of therapeutics. By exchanging Zn2+ by Fe2+ (Zn2+ cannot be oxidized) in a given ZFP and transformed it in an Fe2+ finger protein that could redox between Fe2+ and Fe3+, one can generate the most deleterious OH- (hydroxyl radical) and other deleterious oxidative agents. The Fe2+-ZFP binds specifically to the DNA-RE *in a non-random fashion.* The DNA-RE is permanently suppressed by oxidation of the DNA-RE by the OH-, rendering the DNA-RE site inactive. The posttranscriptional gene silencing blocks the proteins required for either animal or plant division, thus preventing activation by plant or animal pathogenic virus promoters carried by the viruses.

**Pharmacological method and agents for increasing resistance of plants to plant pathogenic viruses: Inhibition of virus-induced overproduction of plant cell DnaK (heat shock ZFP), ribosomal ZFPs, and viral ZFPs involved in the structure of the capside, viral replicating machinery, and transport of viral proteins carrying virulent ssDNA or ssRNA.**

This invention also relates to plants and plant cells which are invaded by pathogenic viruses which eventually destroy the plants. To prevent this deleterious effect, the infected plants are pretreated with one or more of the agents represented by picolinic acid, fusaric acid or derivatives thereof which may or may not have been yet synthesized as a new structure of matter. Picolinic acid (PA) and derivatives thereof can be used to specifically bind to the Zn 2+ in a viral ZFP domain to silence any viral ZFP product deleterious to the plant. An additional advantage these compounds is their negligible toxicity. PA and derivatives have also been shown to induce in infected plants protection not only against pathogenic viruses but also against opportunistic (or pathogenic) fungus that would otherwise eventually destroy the valuable plant *(*Zhang, et al; Cell Research (2004) 14:27-33). Protection against genotoxic agents that damage both DNA and RNA of plants can be also be accomplished by utilizing pyridine carboxylic acids (e.g., PA, Fusaric, etc). This resistance is induced as a result of modulation of gene expression of proteins that protect critical plant survival proteins necessary for the elimination of the virus. Examples of such proteins are heat shock (HSP) proteins such as **DnaJ** proteins, which are zinc finger protein involved in inflammation in animal cells and in plant viral assembly in the plant nucleus. *Dna J interacts with Geminivirus invasive proteins,* and thus, by neutralizing DnaJ with picolinic acid or derivatives thereof the viral cycle is interrupted and the virus cannot replicate with high fidelity and the DnaJ proteins are degraded by plant proteases, preventing viral replication. *Geminivirus* relies on its own viral ZFPs or if the vZFPs are not encoded in its genome, *Geminivirus* by its viral promoters induces cellular plant ZFPs to rapidly replicate and invade the entire plant as a virion and later as a full virus, which is eventually released as a "mosaic" dust structure, sometimes by the leaves.

The DnaJ proteins and other heat shock and ribosomal proteins such as MPS-1-S27 interact with the *Geminivirus* to enable replication. The compounds of this invention are highly effective to prevent such replication and also destroy overproduced plant proteins which normally (without treatment) would increase plant virus replication, propagation in the plant, and propagation by vectors. Thus, PA and its derivatives not only destroy the viral zinc finger proteins but also prevent the virus' use of and interaction with critical plant proteins stimulated to overproduction by the presence of the *Geminivirus.* Heat Shock proteins, ribosomal RNA proteins [e.g. ribosomal S27/Metallopanstimulin 1, 2 and 3-of *Arabidopsis thaliana],* and other metalloproteins involved in the pathogenic effects of the plant viruses can similarly be neutralized by PA and derivatives thereof.

Finally, since in normal uninfected cells PA and its derivatives do not induce any significant toxic effect, the deleterious propagation of the virus is arrested by picolinic acids. Since the *Geminiviruses* replicate *only in plant cells that have initiated cell division,* this results in destruction of virus-infected plant cells by the process of program cell death (PCD), which prevent transfer of any infectious virion to other cells because *Geminiviruses* are unassembled, degraded and precipitated by the process of PCD.

In essence, the inventors believe that if PA and its derivatives are timely and properly applied to the infected plants, the viral infection of plants can be arrested, controlled and prevented, and valuable crops such Cassava and other *Geminivirus* vulnerable plants can be protected and the virus eradicated from infected plants.

### BEGOMOVIRUSES AND GEMINIVIRUSES:

### Single-stranded ssDNA Plant Viruses

Viruses infect any kind of organism, including plants and plant cells.

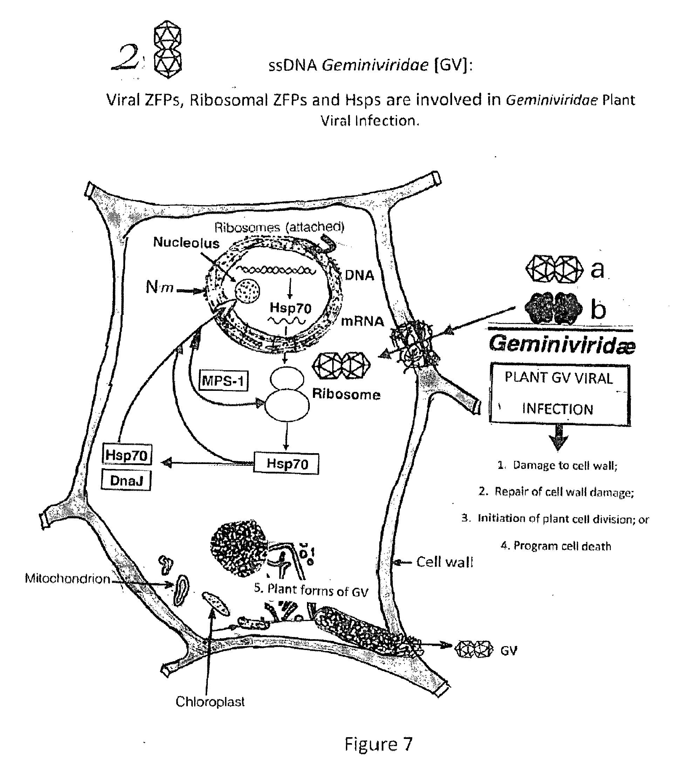

*Begomoviruses* that infect uncultivated Eudicots are one of four genera in the Family, *Geminiviradae.* This family is unique among plant viruses: They have small, circular, positive sense, single stranded DNA (ssDNA) genomes encapsidated in paired icosahedral structures. **(****FIGS. 7****,****8****,****12** **and** **13****).** Begomoviruses of the New World have two chromosomes (bipartite genome); Begomoviruses in the Eastern Hemisphere (Old World) can be either bipartite and monopartite (one chromosome) (Lazarowitz, 1982). Begomoviruses infect a wide range of dicotyledonous Eudicots in subtropical climates. Eudicot species are the most abundant plants on earth. Begomoviruses viruses have evolved mechanisms, including zinc finger proteins that facilitate plant-to-plant dispersal. More than 500 species of Bemisia tabaci (white fly) transmit these viruses from plant to plant.

Begomoviruses genomes contain DNA significant sequence homologies of prokaryotic ancestry. They have nevertheless captured and utilized genes that function in eukaryotic plants and arthropods such as Eudicots and whitefly vectors, respectively. Well known relatives of Begomoviruses are *bacterial phages.* Begomoviruses have a tendency to recombine in the presence of co-infecting counterparts. There is also evidence of genetic recombination with host plant genomes. For example, solanaceous species contain multiple begomoviral integrated repeated sequences into the genomes of tobacco and tomato.

The cross-kingdom versatility of Begomoviruses is manifested by the origin of replication (ori) of the ssDNA circle and the replication strategy, which are similar to those utilized by some ssDNA phages (Frischmuth *et al,* 1990). The ori contains the sequence TAATATT*AC, at which the circular genome is nicked (T*A). This structure initiates rolling circle replication. This sequence is conserved in all four genera of the Geminiviridae, and some ssDNA viruses of plants. In contrast, begomoviral protein coding regions and their respective *promoters have typical characteristic of eukaryotic promoters* (Bisaro, 1996). Introns are not present in begomoviruses transcripts. Thus, these viruses have the ability to control the plant cell cycle into the replication phase.

The main goal of this invention is to control Begomoviruses that produce diseases of agriculturally-important crops. Furthermore, the accumulating evidence for interspecies and intergenetic recombination in *Geminiviridae* provides mechanisms for the emergence of novel strains of viruses which may cross biological barriers, with consequences for world food supplies.

Although this invention is not restricted to the family of *Geminiviruses* ssDNA (ss=single-stranded), and can be used in essentially any other plant virus containing zinc finger proteins or viruses that hijack cellular zinc finger proteins for their replication strategy or utilize complete cellular ribosomes (e.g.: Arenavirus, agent of the lethal hemorrhagic fever of Junin, Argentina, have cellular ribosomes containing zinc finger proteins). Most of this invention refers in detail to the actions of PA and derivatives on *Geminivirus* ssDNA, viral ZFP, virus-induced plant ZFP, and other critical proteins for the successful transport, assembly and infectious characteristics of *Geminivirus.*

One of the most important reasons for the inventors focusing on *Geminiviruses* is that *Geminivirus* infect widely used plants of great edible value. *Geminivirus* is presently rapidly spreading in Africa, India, and Brazil. More effective methods of saving the crops, preventing the infection, and eradicating the virus could help prevent catastrophic famines.

The *Geminiviruses* have an unusual geminate morphology and a genome of two similar size DNAs **(DNA-A** and **DNA-B)** *single stranded (ss) DNA* **(Tables 1,2 and 3).** There are more than 50 recognized members and many other recombinant-geminivirus infecting Cassava. The capsids are composed of 110 polypeptide subunits. The twinned-isometric (geminate) morphology arises by fusion of two incomplete T=1 icosahedra. Each geminate particle *contains only one molecule of ssDNA.* The Open Reading Frames (ORF) can encode proteins of molecular Wt over 10kD. Transcription can occur leftward (L) or rightward (R) from large intergenic regions that separate the L and R ORFs **(****FIG. 13****).**

*Geminivirus* accumulate in the nucleus of infected cells, where they carry out viral DNA replication. Subgroups II and III viruses induce in the nucleus of the cells characteristic changes such as the formation of fibrillar rings composed of DNA-protein complexes. After replication of ssDNA to dsDNA and synthesis of ssDNA from dsDNA, encapsidation occurs. ssDNA synthesis can be coupled to encapsidation. The covalently closed circular supercoiled dsDNA is deposited inside the capsid.

One important characteristic of *Geminivirus* such as ACMV is that it appears to replicate only in dividing cells. This could reflect the requirements of *Geminiviruses* for cellular DNA polymerases that are synthesized during the *S phase of the cell cycle.* The known *geminiviruses* are able to infect monocots or dicots, but not both. This is due to the adaptation of the virus to particular replication characteristics of the host. **The coat protein (CP) determines the vector specificity.** The transmission, host range and geographic distribution of *geminiviruses* depend on the host range of the vector and the ability of the virus to replicate and establish a systemic infection in a given species of plant.

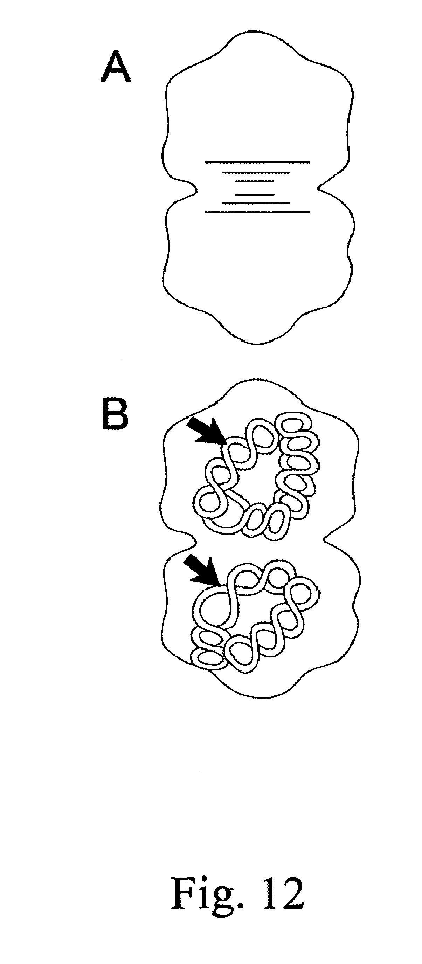

In order to clarified some of the more complex issues concerning antivirals that will be an integral part of this invention a brief description of the characteristics of *Geminivirus* relevant for the embodiments of this invention will be briefly described as follows:
• The *geminiviridae* are denoted for their unique capsular structure. Virions in this family have paired incomplete icosahedral (twenty faces) capsids (Gemini = twin moons). The size is small, 18 to 30 nm. **(****FIG. 12****)**
• The viral genomes are covalently closed circular ssDNA (about 2.5 to 3 kb), *with one single stranded ssDNA molecule encapsidated in each of the two adjacent capsules.* Thus the total number of ssDNA is two, with one circular ssDNA being slightly shorter than the other **(****FIG. 12** **and** **13****).**
• Geminiviridae have **three genera** which are based on several factors such as genome organization, vector specificity, and host range properties. The genera are named: Begomovirus, Curtovirus and Mastrevirus.
• *Geminivirus* express at least one protein with a conserved zinc finger (ZF) motif. For example, **the zinc finger coat protein (CP)** of the *Tomato leaf curl Bangalore* virus *is responsible for the binding to ssDNA. This virus belongs to the genus. Begomovirus of the family Geminiviridae.* Kirthi *et al* determined that the Ban5 Coat protein (CP) synthesized in E. coli is characterized as the CP that binds to the ssDNA. A Gene Bank search for motifs responsible for CP binding to ssDNA indicated a conserved putative ZF motif in the CPs corresponding to residues 65-85 of Ban5 . Site directed mutagenesis of the ZFP cysteines and histidines indicated conclusively that the binding to zinc and ssDNA was due to such ZFP sequence.

A second mechanical and evolutive reason for the inventors to solve this apparently complex problem is that the inventors has studied for more than 30 years, animal and some plant viruses and has arrived at the conclusion that many plant viruses are similar in their strategies, proteins, DNA and RNA replication. Most importantly, all of the plant and animal virus require ZFP for structural and functional purposes, indicating that although they have evolved along quite different paths, they were not able to replace the essential ZFP for vital functions such as structure (e.g., M1 protein of human influenza virus; icosahedral blocks of Geminiviridae associated with critical ZFPs); transcriptional factors; ZFP for links between ssDNA, capsid proteins and ZFP, etc). Finally, the ZFPs contain the metal binding zinc finger domains that are essential for survival of any living organism. Thus, any change to the highly conserved ZFP domain in a virus -whether by mutation, chemical silencing or destruction, or formation of an irreversible ternary complex, renders the virus neutral or kills the virus outright. To examine this important issue better, a brief description of the characteristics of *Geminivirus* will be described as follows. It is germane to note here that if the pathogenic virus is devoided of zinc finger proteins for replication, it utilizes the induced cellular (plant or animal) zinc finger proteins of the infected cells.

*Mastrevirus* (prototype virus, maize streak virus [MSV]) consist of a single molecule of ssDNA, are transmitted by leafhopper, and in general infect monocotyledonous plants. *Begomoviruses* (prototype virus, bean golden mosaic virus) are transmitted by white flies, infect dicotyledonous plants and with a few exceptions have two characteristic genomic components (A and B) that are required for infection. *Curtoviruses* (Prototype member, beet curly top virus), are transmitted by leafhoppers, have a single genomic molecule, and infect dicotyledonous plants.

In general, individual *Geminiviridae* have a narrow host range. Despite this selective disadvantage, in recent years they have emerged *as one of the most dangerous plant viruses worldwide.* These plant virus pathogens, among other advantages have the ability to recombine in mixed infections and thus give rise to new strains. (Fondong *et al:* Evidence of synergism between African cassava mosaic virus and a new double-*geminivirus* infecting cassava in Cameroon. J. of General Virology (2000),81,287-297)

The viral genes are transcribed bidirectionally from a region denoted intergenic region (IR). The encapsidated ssDNA is the virion-sense positive strand. **FIG. 13** shows the genome organization of different genera of the Geminiviridae family.

The Begomovirus encode proteins required for replication, transcriptional regulation, *zinc finger proteins,* encapsidation and movement proteins.

Plant viruses encode movement proteins that are critical for systemic infection of the host. Movement proteins are not necessary for viral disassembly, assembly, replication, and encapsidation. An example of *Geminivirus* protein that in indispensable for systemic infection is the *protein BL1. The BL1 movement protein* is associated with endoplasmic reticulum-generated microtubules in the developing phloem cells. Phloem cells conduct a variety of materials throughout the plant structures, mostly carbohydrates but also proteins. BL1, one of the two movement-encoded proteins by the *geminivirus,* bind to 40 nm microtubules that connect the walls of the procambium [meristematic or growing layer in the tip or root] cells seedlings. This study, done in the bipartite *geminivirus squash leaf curl virus* (Ward, B M et al, J. Virol. 1997; 71: 3726-3733). indicates that the virus established contact with the ER and used the ER cysternae as a way to travel from cell-to-cell. In animal cells, microtubules are also used to convey viruses from cell-to-cell. For example, retrograde transport of *Herpes Simplex* virus uses the polarity of afferent nerves to transport the virus to the paravertebral ganglionar cells (Topp KS, et al; J. Neurosc. (1994), 14: 318-325).

*African cassava virus* (ACMV) belongs to the genus *Begomovirus.* ACMV possess bipartite genomes, dicotyledonous hosts, and one whitefly species ***(Bemisia tabaci*),** as a vector. *Transmission by insects is dependent on the **Coat Protein (CP).*** Detailed structural data on the capsid morphology is available (Bottcher, B et al; J. Virol. 2004; 78:6758-6756)

Fondong *et al* (2000) found evidence of synergism between African cassava mosaic virus and a new double- recombinant *geminivirus* infecting cassava in Cameroon. The evidence of recombination was found in the AC2-AC3 region of the DNA-A component of ACMV-like virus. The DNA-B of EACMV-like viruses also contained evidence of recombination in the BC1 region. Both types of viruses retained gene arrangements typical of bipartite *begomovirus.* The two viruses interacted synergistically when plants were infected by them. (Fondong et al: J. of General Virology (2000), 81, 287-297).

### SOME GENERAL CHARACTERISTICS OF PLANT VIRUSES RELEVANT TO THE EMBODIMENTS OF THIS INVENTION.

As a result of the complex interaction of plant and animal systems, and the ecological features of higher and lower plant and animals, numerous complex mechanisms are used by plant and animal viruses to effectively infect members of the plant and animal kingdoms. Some of the general properties of pathogenic plant viruses will be briefly described to point out the significant consequences of the differences between plant and animal viruses. The similarity, however, in the utilization of chelating agents, ZFPs, and other metalloproteins in both plant and animal cells is critical. The purpose of this comparison in mechanism of viral replication is related to the fact that zinc finger proteins are targets for preventive and therapeutic agents in both plant and animal viruses. If properly used, the agents are *non-toxic* for normal cells and innocuous for the environment.

The different morphology, anatomy, and ecology of animals and higher plants, clearly appear to result in remarkable differences in the mechanisms employed by viruses to infect plant and animals corresponding to their two respective kingdoms. However, in numerous occasions the differences are more theoretical than factual. The relevant characteristics of animal cells and plant cells will be briefly described as follows.

The transmission of plant viruses utilizes a large number of both mechanistically simple mean and complex vectors and mechanisms. For example: the virus can be carried from plant to plant by aphids and also by soil-living organisms. Some viruses can be introduced in plants by nematodes feeding on root tissues. Virus infecting or contaminating spores, pollen or seeds can pass the virus to the progeny. The use of virus-infected-tubers and bulbs for propagation of plants is also an effective way of transmitting viruses

### PATHOGENESIS OF VIRAL INFECTION IN PLANTS: TRANSPORT OF VIRAL DNA TO THE NUCLEOUS, NUCLEOLUS AND TO OTHER CELL'S COMPARTMENT

The infection by *Geminiviruses* induces in Cassava plants the following signs: Striate mosaics and streaks, leaf curling, vein chlorosis, yellow and green leaf mosaics. The intensity and type of signs depends on the amount of virus replicating per cell and the number and type of cells infected. Specific deleterious interactions between viral proteins and cellular proteins induce direct and indirect effects on the cells resulting in damaging of cellular constituents or apoptosis. The primary target for *geminiviruses* is the nucleus as well as the nucleolus. *Geminiviruses* replicate and induce morphological changes in the cell nucleus and nucleolus. Interference with normal nuclear and in particular nucleolus functions (place of ribosomal assembly) is most likely the primary cause of the cytopathogenic effects of these viruses. The signs of viral infection described above can be explained by replication and propagation of the viruses in phloem cells with the consequent impairment of the plant's vascular system from properly functioning. From the vascular system of the plants the viruses invade other plant cells. *Geminivirus* mutation may either attenuate or increase the virulence of these viruses. *Mutation in zinc finger protein motifs of geminiviruses genes is lethal for the virus*

The movement protein (MP) and the coat protein (CP) of the *maize streak virus* (MSV) are both required for systemic infection. The MP diverts a CP-DNA complex from the nucleus (where viral DNA replication occurs) to the cell periphery. In cooperation with CP, the MP protein mediates cell-to-cell movement of viral DNA. Thus, in MSV, both MP and CP mediate cell to cell movement of ssDNA of *Maize streak* virus, indicating that these proteins have functional analogy with the BC1 and BV1 proteins, respectively, of the Begomavirus genus of *geminiviridae.*

Mutagenesis studies have shown that the MP gene encodes movement proteins (MP) which are required for cell-to-cell movement of virus. The coat protein encapsidation is required for the systemic movement and encapsidation of MSV ssDNA into virions and insect transmission.

The use of novel specific transition metal ion chelating compounds that inactivate plant viruses by attacking highly conserved viral ZFPs in the virally infected plant cells provides *a mechanism for new and safe antivirals* and a method which can be used to predict what antiviral compounds can effectively inactivate, disrupt, or form an inactivating ternary complex with the specific viral zinc finger domains of *geminivirus.*

This invention provides both a molecular mechanism for the action of novel and safe antivirals, and the antivirals themselves to enable one to inactivate, disrupt or prevent the propagation of *geminivirus.* With this system, in which picolinic acid (PA) is the lead compound, the only plant cells destroyed by PA are those containing *geminivirus* with active viral zinc finger proteins. At effective antiviral doses, the agents are non-toxic for the plants, for the users, for the consumers, and for the environment into which the antiviral spills.

### DNA CONSTRUCTS AND METHODS TO ELIMINATE PATHOGENIC VIRUSES

Transgenic plants display, in certain instances, resistance, recovery (viral infected plants initially show systemic infection but newly develop leaves and roots become resistant to the invading virus. Susceptible phenotypes may succumb to systemic infection. In general, the resistant state is mediated at least in part at the cytoplasmic level by an activity that reduces the high steady state levels of RNA preventing the virus from replicating. It was shown that a cytoplasmic activity targets specific RNA sequences for inactivation. (Lindbo, J. A. et al., "Induction of a highly specific antiviral state in transgenic plants: Implications for regulation of gene expression and virus resistance", Plant Cell,, 5:1749-59 (1993). The low steady state RNA levels are due to post-transcriptional gene silencing.

The degradation mechanism is specific for the transcript that increases above the set point level for a given cell; and, if the transcripts that increase above the set point level, are a viral transgene, the virus resistance state is observed in the plant due to specific degradation of the virus RNAs targets.

There are numerous other naturally occurring and artificial mechanisms for degrading and disintegrating pathogenic viruses infecting plants, such as the preparation of specific cassettes that with certain viral resistant properties that will result in the protection of specific transgenic plant cells invaded by viruses either individually or systemically.

The present invention is directed to the production of pathogenic virus resistant plants by transforming the plants with specific cassettes DNA constructs possessing the ability of destroying the invading virus and killing the cell (s) by the activation of associated death genes.

### SUMMARY OF THE INVENTION

The inventors have determined that topical administration of a composition comprising picolinic acid, or derivatives thereof, is effective in controlling or treating infections by common plant viruses. The present invention provides a pharmaceutical composition comprising compounds having the following structures: 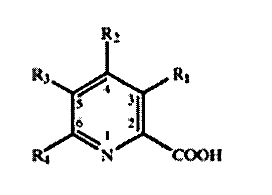 or a pharmaceutically acceptable salt thereof, wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen, and wherein the amount of the compound in the pharmaceutical composition is sufficient to reduce, destroy, or irreversibly precipitate (at low pH = 5.00) the virus intracellularly, and inhibit growth of *Geminiviruses* by several orders of magnitude.
The present invention further provides a method for treating, preventing or controlling *Geminiviruses* by administering to a plant or crop afflicted with *Begomoviruses* a therapeutically effective exogenous amount of a composition comprising a molecule (s) having the following structure: 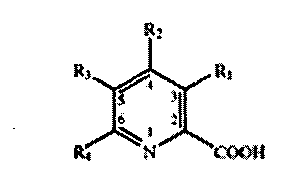 or a pharmaceutically acceptable salt thereof, wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen, and wherein the composition reduces or inhibits growth of *Geminiviruses.*

The mechanisms by which the zinc finger motifs of nucleocapsid proteins of *Geminiviruses* are disrupted and inactivated have now been discovered. Essentially these mechanisms can be summarized as follows: (1) The chelating antiviral agent can remove the Zn²⁺ from the zinc finger domains of proteins produced by the *Geminiviruses* (GV) rendering the protein inactive; (2) As a result of the binding of the chelating antiviral agent to the zinc in the zinc finger motif of the GV-ZFP, the GV-ZFP *changes configuration* and, in turn, causes a much greater susceptibility to proteolysis of GV-ZFP; (3) Certain intracellular conditions in the GV infected plant cell such as low pH = 5.00, presence of insoluble proteins, redox state, etc, may be more favorable to the formation of ternary complexes such as: Zn-Zn finger motif- Picolinic acid , which will result in inactivation of the GV-ZFP. Furthermore, it has been demonstrated that the GV infected cell, as a result of the chelating antiviral treatment, will enter into apoptosis which will result, in turn, in the elimination of the virus and the virally-damaged non-viable plant cell. This mechanism, denoted program cell death (PCD) or apoptosis, is very common in animal cells infected with viruses or virally infected cells treated with chelating agents.

### METHODS FOR IDENTIFYING AND USING COMPOUNDS THAT INACTIVATE GEMINIVIRUS AND OTHER BEGOMOVIRUSES BY ATTACKING HIGHLY CONSERVED ZINC FINGERS DOMAINS IN THE VIRAL NUCLEOCAPSID PROTEINS.

The chemical mechanisms delineated above provide the opportunity to predict which compounds disclosed in this invention or to be studied in the future can effectively interact with the zinc finger motif and subsequently inactivate the *Geminivirus* ZFPs of interest.

Fernandez-Pol *et al* (US Patent '04 1, Sep. 7, 1993) isolated, cloned and sequenced about 50 human proteins from human breast cancer. The results were published in Cancer, Genomic and Proteomics; 2: 1-24 (2005). Many of proteins, including MPS-1/S27 were ribosomal ZFP and ZFP transcription factors which are expressed in numerous cancer cells. The Fernandez-Pol US Patent '041 discloses methods that can be used to detect viral ZFP produced by expression vectors generated through recombinant DNA technology (US Patent '041; F-Pol; Can. Geno. Prot., 2004). These ZFP reconstituted with Zn2+ provide the source of the viral zinc finger nucleoproteins and capsid ZFPs that are the targets for attack by the compounds of this invention.

The inventors utilized several methods to determine the effectiveness of Picolinic acid, Fusaric acid and derivatives thereof, for disruption of specific viral ZFPs, as follows: 1) General methods: Several classes of compounds are provided in this invention which can be used to inactivate *Begomoviruses* containing CCHC, CCHH, CCCC (and permutations by mutations) zinc finger motifs. In the section Examples the inventors provides evidence that numerous types of compounds can be used to inactivate retroviruses such as HIV-1, by attacking the CCHC zinc fingers of the HIV-1 viral protein and ejecting the zinc at low pH; 2) Once the ZFP of the *Geminiviruses* is inactivated by removal of Zn2+, the viral ZFP can be used as a detector ZFP (after addition of Zn2+ and attaching the protein to the PVC plates) for detection of novel antiviral agents against *Geminiviruses* and other viruses containing ZFP; 3) ELISA assays utilizing *Geminiviruses* ZFP can be used for the early diagnosis of Cassava and other plants and plant cells infections (previous preparation of monoclonal antibodies); 4) Capillary Zone Electrophoresis (CZE): the presence or absence of Zn2+ in ZFP cause a change in the conformation, charge and mobility of the ZFP. When the ZFP is reacted with the test chelating agent compound, a change in the mobility occurs, which reveals if the interaction is suitable or not; 5) Release or formation of ternary complexes of Radioactive zinc-65 labeled ZFP from *Begomoviruses* ZFP. The release of Zn-65 or the stronger binding of the test compound to the Zn-65 bound to the viral ZFP can determine the degree of reactivity of the test compound (Fernandez-Pol, Cancer, Genomics and Proteomics, 2005). Addition of the DNA response element for the ZFP can further verify the results; 6) Similar experiments can be perform with fluorescent protein probes; 7) Detection of Gel-Mobility Shift Assays: Recombinant ZFP can be used to test novel compounds to determine if ternary complexes are formed with the test agent-Zn2+-viral protein; 8) High Pressure Liquid Chromatography (HPLC) can be used to determine the binding of Zn2+ to nucleoproteins and the disruption of the binding by the test agent.8) Nuclear Magnetic Resonance (NMR) detection of Zn2+, synthetic peptide, and test agent interactions. Numerous experiments on the utilization of this technique to study interaction between zinc finger proteins and test agents will be presented in the section of Examples. NMR is used to detect the loss of zinc from ZFP or the formation of strong ternary complexes with the agents of this invention having hydrophobic residues (Please see Examples).

In addition, the inventors have discovered that these compounds not only bind the Zn on the zinc finger domain but if they are synthesized with a lipidic (e.g. butyl) or hydrophobic amino acid extensions (16 to 21 amino acids) they can specifically interact with the amino acids surrounding the Zn atom in the zinc finger motif pocket. In this case, the inhibitory activity and inactivation of the ZFP is much greater and occurs at lower doses of the chelating antiviral agent. The result is the formation of a very strong ternary complex.

Based upon the foregoing, numerous classes of compounds based on picolinic acid, fusaric acid and derivatives thereof, have now been invented and tested which can be used to inactivate *Geminiviruses* by attacking the zinc finger domains of ZFP and also neutralizing the zinc atom. The same systems can be use to search and identify new classes of *anti-Geminivirus* drugs targeted against viral nucleoproteins or other essential GV viral ZF proteins such as the CP protein of GV. Not every compound showing reactivity with the ZFP will be able to reach and attack the ZFP in the GV. Thus, the discovery of the design characteristics that are necessary in a molecule of an antiviral agent to reach essentially any place in the plant and its cells where the GV may be located enables rational drug design aim at attacking the essential elements of a GV in any hiding place, including and particularly those with low pH. *Begomoviruses* have a propensity to proliferate in low pH cellular compartments.

### Test for zinc finger protein

An assay has been developed for the identification of small molecules which binds non-covalently to Zn²⁺ bound to a ZFP. The small molecule (e.g. pyridine carboxylic acids) distrupts indirectly the binding of the Zn²⁺ - ZFP to a specific DNA sequence. These small molecular weight agents act as nucleic acid antagonists in both animal and plant cells viruses by disrupting the binding of Zn²⁺- ZFP to DNA specific sequences. This in vitro test has been used for discovering novel antiviral-anti-ZFP compounds and it is based on the following principles: the assay is a solid phase ZFP bound to plastic. The small molecule binds to the zinc in the complex Zn²⁺- ZFP - oligonucleotide, non-covalently, and reversible. If the small molecule is active, the agent disrupts the complex structure bound to the plastic wells. The complex structure consist of a synthetic peptide of 12-36 amino acids, containing a Zn²⁺ - ZFP binding sequence of a pathogenic plant or animal virus which strongly binds to Zn²⁺ - ZFP. The complex Zn²⁺ -synthetic peptide is also strongly bound to an oligonucleotide DNA specific sequence forming a ternary complex.

Details of the Method to Test for zinc finger protein Disruption: An in vitro test for Disruption of animal and plant cell Zinc Finger peptides containing at least one zinc finger motif bound specifically to Zn²⁺. Then, a DNA oligonucleotide with a specific sequence binds the Zn²⁺-peptide with high affinity and specificity (Kd = 10⁻¹¹). Our work using Magnetic Resonance (NMR) Spectroscopy was performed to characterize the high affinity binding of a peptide representing a portion of the viral nucleocapsid protein of HIV-1 denoted Np7 to determine if solid-phase assays in 96 well-plates performed with the HIV-1 specific peptide, and Zn²⁺ can be used to determine if the Np7 peptide could bind with high affinity (Kd's in the low nanomolar range) to short repeats of dTG's (These assay was based, with some modifications introduced by Fernandez-Pol, on the assay developed by Rice et al, "Inhibition of HIV-1 infectivity by zinc-ejecting aromatic C-nitroso compounds ", Nature, Vol. 361, 4 February 1993; pp 473-475). Experiments performed in our laboratory confirmed Rice et al reports and indicated to Fernandez-Pol that the method could be used with modifications in the instant invention to detect novel antiviral compounds. The basis of this method is that small molecules by binding Zn²⁺ which is bound to the nucleoprotein p7, prevent and disrupt the interaction of the nucleoprotein p7, with nucleic acid [short repeats of dTG's]. The small molecules that are able to disrupt zinc bound to the peptide which in turn is bound to DNA, might be of therapeutic interest for further development in pre-clinical trials. Thus, the main objective of the inventors is that this test of disruption of binding of Zn²⁺ to nucleoproteins and other viral zinc finger proteins by small molecules is to find novel compounds that inhibit the high-affinity nucleic acid binding activity of ZFPs of *Geminivirus* and other plant viruses using synthetic peptide plant virus ZFPs for the pathogenic activities of the full length protein in the intact virus. The novel compounds if positive will have to bind in the test in a reversible and non-covalent fashion using 96-well plate assays and subsequently NMR- spectroscopy assays.

Results with picolinic acid and fusaric acid, tested separately by NMR- spectroscopy assays, will be described in great detail in Examples to demonstrate the sensitivity, specificity and efficiency of this method to detect new analogs of picolinic acid and derivatives thereof. Furthermore, the Example will show the binding of Zn²⁺ to the peptide and the Zn²⁺-peptide to the oligonucleotide DNA sequence. The 96- well plate assay (Falcon electrically charged plastics) has been used to immobilize to the plate animal and plant zinc finger proteins of interest, then the non-specific binding sites were blocked with Bovine Serum Albumin (BSA) and washed automatically 8-times. The 96-well plates with the various ZFPs bound to the plastic and the non-specific sites neutralized by excess BSA were stored at -20C until used for testing the compounds of interest. The target oligonucleotide [biotinylated-short repeats of dTG's] was then added with or without 1 µM and 10 µM compound of potential therapeutic interest in the presence of radioactive Zn ²⁺- 65 and a reducing agent such as 10 mM Ascorbic acid to prevent oxidation of proteins, and incubated for 1 hour at room temperature (25°C). After washing the plate with PBS containing BSA, streptavidin HRP was added and bound oligonucleotide was detected by luminescence. We have selected for screening over 100 analogs of picolinic acid, fusaric acid and derivatives thereof. The compounds were either Natural Product Extracts (NPE) [which we continue to tested], from the NCI collection of open compounds for qualified researchers (e.g. extracts of tannins), or were purchased from different commercial sources such as Sigma-Aldrich, St. Louis, MO. These efforts resulted in the confirmation of the effects of picolinic acid, fusaric acid, and other compounds unrelated to picolinic acid and described in detail in an initial patent application [Fernandez-Pol et al US 2003/0225155 A1] (now a full issued US Pat.) which may complement the uses of picolinic acid and derivatives thereof in disrupting plant and plant cell pathogenic virus such as *Geminiviruses.* Furthermore, in the last eight, Dr. Fernandez-Pol tested Natural Product Extracts from plant extracts of the Patagonian lake region of Argentina. The inventors found novel and powerful analgesics-non-narcotics unrelated to known narcotics. *In similar plant extracts the inventors is studying wide-spectrum plant and possibly animal antiviral agents against pathogenic viruses that infect plant and plant cells.* The inventors suggest that some of the antiviral plant agents act by disrupting viral zinc-finger proteins. The identification of these acids, unrelated to picolinic acids and other plant cell compounds that disrupt ZFP in plant cells may result in the development of potent inhibitors of plant and animal cell pathogenic viruses derived from plant cells of peculiar plants that are resistant to most if not all the known viruses.

The most promising results for nucleoprotein p7: nucleic acid antagonists have come from a small library of drug-like compounds: Picolinic acid, Fusaric acid and derivatives thereof. Within the overall hit rate of -1%, there were over 80 compounds with a high degree of structural homology but with different side-chains, and all of them are 2-pyridine carboxylic acid. From the over 80 compounds, four (4) were examined in detail using the assay technology described above and they were found to have Kd's (dissociation constant Kd of about 10⁻¹² M (Compound binding was done at physiological pH of 7.0). These compounds and some structurally related compounds were tested in animal NRK (normal rat kidney cells) transformed by the following viruses: SV-40 (DNA virus), Kirsten sarcoma virus (RNA virus), and the HSV-1(Herpes labialis) and HSV-2 (Herpes genitalis), HIV-1, Hepatitis B virus (contains ZFPs), Hepatitis C virus (contains metalloproteins) and other viruses representing several common families of human and animal pathogenic viruses. Common ornamental plant cells infected by viruses were also tested with antiviral agents such as picolinic acid or fusaric acid, with positive results (elimination of the disease leaves without any progression of the viral disease), but the viral strains were not determined. Previous results of the inventors showed that there was a correlation among the three most potent binders of Zn ²⁺ bound to the 18 amino acid peptide sequence (which is bound to Zn ²⁺) VKCFNCGKEGHIARNCRA which in turn is bound to the d(ACGCC) packaging signal of HIV-1 virus. The Zn²⁺ disrupting agents were clearly active (their anti-HIV potencies (EC50) paralleled their Kd's for compound binding to NCp7). All these compounds were very potent and inhibited and disrupted the Zn²⁺ -Peptide- DNA sequence in the *in vitro* assay. These classes of chelating agents competitive Zn²⁺ inhibitors of binding to the 18 amino acid peptide of the test may represent important derivatives of picolinic acid and fusaric acid analogs that may be very useful to control plant viral infections. Further details are presented in Examples.

In addition to the NMR spectroscopy experiments described above, and presented in **Examples,** to further characterized the interactions among the molecules (small potentially therapeutic molecules; Zn ²⁺, and DNA oligonucleotide) the inventors used SPR Spectroscopy to verify and characterized further the high affinity binding of *Geminivirus* nucleocapsid (NC-CV) proteins, Coat Proteins (CP), and other ZFPs of great importance to understand the Cassava vein mosaic virus. Figure 13 shows the genomic organization of CsVMV according to the nucleotide sequence. The inventors expects that the following experiments will lead to the discovery that the NC-CV protein of *Geminivirus* could bind with high affinity (Kd's in the low nanomolar range) to short repeats of DNA nucleotides of CsVMV (Cassava ssDNA circle). This will be used to demonstrate further that small molecules that prevent the interaction of Cassava NC-CV with nucleic acid of either A or B ssDNA strands could be of therapeutic use. Thus, one of the objectives of this investigation is to find novel small molecules the act in a reversible and non-covalent fashion using the 96-well plate assay, NMR-spectroscopy and SPR-spectroscopy determinations. Although the compounds presently available work with adequate properties such as efficacy, potency, specificity, dose-response relationship, safety and stability, the inventors believes that it might be possible to discover novel compounds that may be selective for each and every one of the ZFPs of any virus if properly designed, as has been previously shown with other compounds that disrupt zinc finger proteins (Fernandez-Pol, US Pat Dec 4, 2003).

In summary, the assay for the identification of pharmacological useful small molecules that disrupt Zn²⁺ binding to a synthetic sequence obtained from a plant cell protein region of about 13 to 30 amino acids (the sequence of the peptide represents a portion of a ZFP that binds to ssDNA with high affinity) consists of the following steps: 1) Criteria for ZFP and Small Molecule Screening: Novel, high affinity zinc finger dependent, nucleic acid binding activity to ZFP - Zn⁺²); 2) The synthetic plant peptide sequence provides an opportunity to detect a new class of small molecular weight plant viral inhibitors; 3) The aim of this work is to find novel and reversible chelating agent inhibitors of the complex ZFP - Zn⁺² -oligonucleotide that binds Zn²⁺, of high affinity of interaction between the *Geminivirus* ZFPs and nucleic acids (ssDNA; A and B); 4) The *Geminivirus* zinc finger protein is coated onto an electrostatically treated polystyrene microtiter plastic plate. The biotinylated DNA oligonucleotide corresponding to a high affinity binding site for the DNA denoted CsVMV of Cassava vein mosaic virus is added to the well prior solubilization in buffer at pH 7.0; After 1h the unbound oligonucleotide is washed out and what remains bound to the plastic well, named ligand oligonucleotide, is detected with Horseradish Peroxidase (HRP) covalently bound to Streptavidin. The HRP enzyme is detected by using a chemoluminescent substrate; washed with PBS and develop; 5) The wells are read in an automatic chemoluminesce reader; 6) 100% inhibition: is denoted: yes/no, determine the background for all measurements; 7) Use 10 positive and 10 negative compounds as controls; 9) Determine Kd of compounds; 10) Test the positive compounds *in vivo* in plant cells in tissue culture with and without *Geminivirus* infection; 10) Pharmacophore analysis to verify previous results: Analogs with many substitutions (R1-Rn) should be tested *in vivo* and the results should be consistent with the relative activity of the lead compound; Halogens may quench or stabilize the activity of the lead compound.

In summary, a series of compounds have been identified by their ability to inhibit the binding of NCp7 to nucleic acids by non-covalent mechanisms involving Zn ²⁺ chelated to picolinic acid or derivatives thereof; 2) the compounds were demonstrated to be non- toxic and posses anti-HIV-1 activity; 3) The compounds also inhibit proliferation of plant cells in tissue culture; 4) The compounds are active in an *in vitro* HIV-1 assembly assay; 5) The same compounds were active in disassembly of *Geminivirus,* as demonstrated by computer modeling; 6) A lead compound, picolinic acid, has been identify for modification to increase desirable properties.

More specifically, in one embodiment of the present invention, a method is provided for the dissociation of the zinc ion from the CCCC zinc finger of metallopanstimulin-1/S27, a multifunctional animal and plant ribosomal protein which is essential for the intracellular synthesis of most viral proteins, *including those of Geminiviruses,* the method comprising contacting the ribosomal protein with a compound selected from the group consisting of: 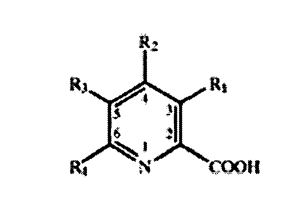 or a pharmaceutically acceptable salt thereof, wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen, and wherein the amount of the compound in the pharmaceutical composition is sufficient to reduce, destroy, or irreversibly precipitate the virus intracellularly, and inhibit growth of *Geminiviruses* by several orders of magnitude. Furthermore, a peptide (s) of 16 to 21 amino acids, preferably amphipatic (to penetrate the plant cells) or having a predominance of hydrophobic amino acids can be easily attached by those initiated in the art. These types of molecules will be necessary when high penetrability in lipophilic layers will be required for therapeutic activity.

The present invention further provides a method for treating, preventing or controlling *Geminiviruses* comprising administering to a plant or crop afflicted with *Begomoviruses* a therapeutically effective *exogenous* amount of a composition comprising a compound having the following structure: 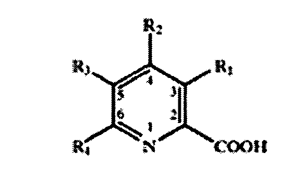 or a pharmaceutically acceptable salt thereof, wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen, and wherein the composition reduces, disintegrates or inhibits growth of *Geminiviruses.*

In addition, the present invention provides methods for detecting the dissociation or the formation of ternary complexes of a zinc ion with the synthetic CCHC-X14 zinc finger peptide of 18 amino acids corresponding to a region of the sequence of the retroviral nucleocapsid protein Np7. Similarly, ZFP of *Geminiviruses* can be used to detect the interaction of *synthetic zinc finger peptides* with plant antiviral agents.

It is among one of the objects of the present invention to provide a compound that can retard the growth, proliferation and movement of target plant viruses by blocking the activity of metal ion containing proteins in the viruses.

Another object of the present invention is to provide a compound which can retard the growth, proliferation and movement of target infective organisms including plant viruses such as *Geminiviridae,* by blocking the activity of metal ion containing viral or cellular proteins essential for viral replication such as *MPS-1*/*S27* ribosomal protein or heat-shock protein *DnaJ,* a zinc finger protein involved in viral infection.

Another object of the present invention is to provide a compound that can retard the growth, proliferation and movement of target viruses in virally infected plant cells by blocking the activity of transition metal ion-containing protein structures such as zinc finger, zinc ring proteins, or other metalloprotein associated with both cellular and viral replication.

It is another object of the present invention to provide a compound that can inhibit and prevent the growth, proliferation and movement of target viruses that infect plant cells, such as *Geminiviruses,* the compound being non-toxic to other plant cells, the environment, animals or humans.

It is another object of the present invention to provide a compound that can inhibit and prevent the growth, proliferation and movement of target viruses that infect plant cells, such as *Geminiviruses,* the compound having not only non-toxic effects but should be edible after treatment of the diseased plants such as cassava and tomato.

It is another object of the present invention to provide an agent that can halt the proliferation and transmission of the *Geminiviruses* that produce Cassava mosaic virus disease.

It is another object of the invention to provide a method of halting the function of zinc finger proteins, zinc ring proteins, and other proteins with zinc binding motifs heretofore unidentified by the administration to the plants or plant cells a zinc chelating agent, both topically or systemically to healthy plants to prevent viral infections or to diseased plants to eradicate the viral disease.

Another object of the invention is to provide a method of halting the function of metal containing protein structures containing metals other than zinc, metal containing ring proteins structures, and other proteins with metal binding motifs heretofore unidentified by the administration of a metal chelating agent, both topically and systemically.

Another object of the invention is to provide an antiviral compound that is effective in a broad range of pathologically reactive disorders of plants and plant cells *including diseases produced by either prokaryote or eukaryote infections and to chemical assault or radiation including but not limited to, ultraviolet, atomic or medical radiation.*

Yet another object of the invention is to provide such chelating agents in a relatively safe and nontoxic form such as picolinic acid or its derivatives for both topical and systemic use in plant and plant cells.

*One of the critical objectives of the present invention is to provide a novel method, to produce plants with increased virus resistance.* The method of the instant invention allows the production of plants which exhibit resistance to a broad spectrum of virus groups encoding zinc finger proteins (ZFP) or viruses inducing and utilizing plant cell ZFP. Another objective is to provide a general method for the production of plants with increased virus resistance which can be used in essentially any plant or plant cell.

The production of syngenic plants with increased virus resistance is provided in detail. The expression of plant DnaJ [a zinc finger protein] denoted also Heat Shock Proteins (Hsps) are induced by viral infection and interact with viral ZFPs of *Geminivirus* components are suppressed by the agents of the instant invention. This important objective is also achieved by a method for the production of syngenic plants whereby the interaction of viral components with plant DnaJ proteins, is substantially prevented by the expression of dominant negative mutants of DnaJ.

Accordingly, the invention relates to methods for the production of syngenic plants and plant cells with increased virus resistance through the interaction of essential viral ZFPs with Picolinic acid, produced by the syngenic plant containing the incorporated gene-enzyme designed to produced Picolinic acid in the presence of the invading virus. These essential viral ZFPs are disrupted and prevented from interacting with other viral and cellular proteins, and subsequently degraded by proteases (due to the change in configuration of viral ZFPs, the protein is susceptible to rapid degradation by proteases). By increasing the endogenous production of Picolinic acid, usually induced by the addition of a specific inducer of Picolinic acid such as a *Geminivirus* Promoter *[Geminiviruses* have the evolutive characteristic of having both prokaryotic and eukaryotic Promoters] or any other suitable enhancer of the promoter linked to the enzyme *picolinic acid carboxylase* (this enzyme converts the precursor of Picolinic acid (PA) into (PA).

### PATHOGENIC VIRUSES THAT ATTACK PLANT OR ANIMAL CELLS INDUCED OVEREXPRESSION OF CERTAIN CELLULAR PROTEINS REQUIRED FOR THEIR REPLICATION, MOVEMENT AND SURVIVAL.

Prokaryotes and eukaryotes express numerous proteins denoted heat shock proteins (Hsps) in response to stress, including heat shock, exposure to heavy metals, hormones and *viral infections* in both animal and plant cells. At present time the Hsps are denoted *Chaperone Machines* and involve numerous actions related to polypeptide folding and recognition of hydrophobic protein surfaces.

The DnaJ proteins (HSP40) are a heterogeneous group of multidomain proteins defined by a highly conserved domain of about **80 amino acids, the J domain,** often located near the NH2 terminus, which is essential for stimulation of the **Hsp70 ATPase activity.** DnaJ is an important protein of the *Chaperone Cycle of the DnaK system of animal and plant cells.*

Proteins participating in the Chaperone Cycle are involved in numerous protein processes such as ATP-dependent disaggregation and unfolding for degradation, conformational maturation of the Super-family of steroid-Thyroid Hormone Receptors and signal transduction by kinases; ATP-dependent stabilization of hydrophobic regions in extended polypeptide segments, ATP-dependent facilitation of folding to the native state, stabilization against aggregation during heat-shock and folding of glucosylated proteins in the Endoplasmic Reticulum (ER) in cooperation with glucosyltransferase. *Thus, they are involved in critical survival processes for both cells and viruses.* For example, virus assembly requires among other complex processes, the active folding of proteins, the prevention of protein aggregation, and the correct folding of nascent viral proteins.

DnaJ proteins have a zinc finger domain and thus they are targets for picolinic acid and derivatives thereof. They interact and stimulate *DnaKproteins (Hsps70), which possess an ATPase domain* which has a pocket that binds ATP and hydrolyses ATP. Thus, the mechanism by which the action of DnaJ proteins couples the regulated ATPase cycle of DnaK with protein substrate binding is the core of the functional cycle of DnaJ-mediated coupling of ATPase with substrate binding.

Therefore, due to the importance of the Chaperone System in both animal and plant cells one can be certain that plant viruses make use of the functions of the cellular chaperone system during viral infection and also use, amongst other things, DnaJ proteins. In this way, the DnaJ proteins provide a point of attack for antiviral strategies, particularly when DnaJ proteins are *over expressed* under the control of *Geminivirus* promoters. Because of their significance as chaperones for important cellular functions in the non-infected plant cells, the partial suppression of DnaJ proteins would lead to viable plants and plant cells with a phenotype which has not essentially been altered as long as the increased levels are reduced to normal or almost normal DnaJ levels after treatment with antiviral agents.

Viruses which attack plant or animal cells, also take advantage of the capability of the J domains to recruit other chaperones. Some viral proteins bind directly to Hsp70 proteins. Other viral proteins, such as the "large T antigen" are equipped with J domains. It is thought that these viral proteins use the cellular chaperone system either to disassemble or assemble viral protein complexes, or to break-up cellular complexes which prevent the spreading of viruses within the cell (Sullivan et al., 2001, Virology, 287: 1-8, Kelley, 1998, TIBS, 23: 222-227).

For plant *closteroviruses* it has been shown that they *contain their own Hsp70-like protein* which is essential for the assembly and spreading of the viruses (Alzhanova et al., 2001, EMBO J., 20: 6997-7007). The movement protein NSm, of the tomato spotted wilt virus (TSWV), interacts with DnaJ-like proteins from *Arabidopsis thaliana* and *Lycopersicon esculentum* (Von Bargen et al., 2001, Plant Physiol. Biochem., 39: 1083-1093 and Soellick et al., 2000, PNAS, 97: 2373-2378). The capsid protein CP, of the *potyviruses* potato virus Y (PVY) and tobacco etch virus (TEV), also interacts with DnaJ proteins. One can conclude that plant viruses utilize DnaJ proteins for their replication strategies. Thus, DnaJ zinc finger proteins provide a point of attack for antiviral strategies.

The inventors have previously shown in animal cells that Picolinic Acid inhibits human skin inflammation by suppression of the expression of DnaJ protein (s) by attacking the zinc finger motif (Fernandez-Pol, Pat. No. '393, Oct. 3, 2000). In the instant invention, suppression of the expression of DnaJ proteins by Picolinic acid, Fusaric acid and derivatives thereof leads to plants with increased virus resistance, but with a phenotype essentially unchanged from wild type plants. The present invention also enables the expression of dominant negative mutants of DnaJ proteins unable to interact with viral proteins or their cellular binding partners, thus enabling the production of plants which have increased virus resistance. The method according to the invention, by means of which plants with increased virus resistance are produced by changing the amount or binding characteristics of DnaJ proteins, not only offers a useful alternative to methods of the prior art, but it also offers considerable advantages to attack more than one target controlled by the pathogenic virus (Among other potential targets for the antivirals of this invention are Metallopanstimulin-1/S27, numerous ribosomal ZFP, and viral ZFP e.g. of *Geminiviruses).*

This invention includes a method for the production of plants and plant cells with increased virus resistance, characterized in that the interaction of Picolinic acid and derivatives thereof will also disrupt cellular plant components containing a zinc finger motif in their protein structures (e.g. DnaJ proteins). Thus, in addition to the interaction of picolinic acid with viral ZFP, picolinic acid can interact, neutralize and suppressed several cellular components with zinc finger motifs, which are specifically utilized and controlled by plant viruses for essential functions such as the modulation of heat-shock proteins (e.g.: DnaJ-like) or zinc finger ribosomal proteins such as MPS-1/S27.

The present invention also relates to a method for the production of syngenic plants and plant cells with increased virus resistance, characterized in that the interaction of viral components with viral ZFP protein is suppressed by eliminating the viral ZFP with Picolinic Acid or derivatives thereof which are produced by the syngenic plant in each cell transfected by DNA containing the appropriate sequences of Picolinic Acid Carboxylase (PAC). The transferring of the DNA vector containing PAC to plant cells can be transitory or alternatively the vector can be integrated into the plant genome. The inventors have determined that the integration of PAC into the plant genome of each cell susceptible to *Geminivirus* infection is the best mode of the invention to prevent infection by the viruses. Details of the procedure to accomplish this goal will be presented in the section Examples.

As a consequence of the production of Picolinic Acid by the syngenic plants and plant cells, the invention relates to the interaction of viral protein components with other zinc finger proteins produced by the plant and plant cells such as DnaJ proteins which are blocked by Picolinic Acid and such as MPS-1/S27 and other ribosomal proteins with zinc finger motifs, prevents the interaction among the viral ZFP with the ribosomal ZFPs which are necessary for viral replication.

In one embodiment, the invention comprise the creation of a DNA vector which contains the following sequence elements in 5'-3' orientation: a **strong promoter** being functional in plants and operatively linked to a sequence responsive for example to a *Geminivirus* ZFP (or other suitable viral protein stimulator) which is in a position suitable **to enhance the sequence encoding the *picolinic acid carboxylase enzyme*** *(PACE)* or functional fragments thereof; and **a termination signal;** followed by transferring the vector to plant cells and, a suitable signal sequence to be **integrated into the plant genome.** When the PACE is stimulated by the addition of *a pathogenic Geminivirus* to the plant or plant cells, the sequence responsive to the pathogenic virus protein bound to the strong promoter can enhanced the transcription of PACE, and the production of Picolinic acid or derivative thereof **in the presence of an adequate chemical precursor of PA** (PAC enzyme substrate) can increase in the steady state the PA concentration [in the intracellular plant space] at least 100- fold (from 0.00 mM to 3 mM) reaching levels of PA incompatible with the existence of *Geminivirus* ZFP and cellular ZFP induced by the virus. The presence of Picolinic acid disrupts both the cellular and viral ZFP and prevents a normal functional configuration of these proteins.

Briefly, the last steps in the enzymatic and non-enzymatic formation of Picolinate from Picolinate Carboxylase (PC) proceeds as follows: 1) 2-amino-carboxy-muconate semialdehyde is converted by PC [previous release of CO₂] in 2-aminomuconate semialdehyde; 2) the 2-aminomuconate semialdehyde in a spontaneous reconfiguration and cycling reaction loses H2O which result in the formation of Picolinate (2-pyridine carboxylic acid); 3) After this non-enzymatic step Picolinate is form, as a terminal useless metabolite, according to classical biochemistry of the decade of 1940; 4) In fact, we know now with certainty that picolinate has a myriad of functions and thus, among other important functions has a key regulatory role in the control of ZFP (Fernandez-Pol, JA, 1977-2006). **FIGURE 17** shows the formation of Picolinate (2-pyridine carboxylic acid), the equilibrium with Quinolinate and the formation in subsequent steps of nicotinate (3-pyridine carboxylic acid).

As it is well known in the art, to be functional, the DNA vector must contain certain necessary regulatory and functional sequences such as **strong promoters**; regulatory sequences (**enhancers**), replication signals, selection markers, vectors with signals for propagation in bacteria; **signals for replication in plant cells**; the vectors are generally plasmids or recombinant viruses. Typical vectors useful for expression of genes in higher plants are well known in the art and in widespread use, and include vectors derived from *tumor-inducing (Ti) plasmid of the Agrobacterium tumefaciens (Ti)* described by Rogers et al, Methods in Enzymology., 153:253-277 (1987). *These vectors are plant integrating vectors.* In other words: On transformation, the vectors integrate a portion of the vector DNA into the genome of the host plant. The strong promoters are constitutive promoters, such as the ubiquitin promoter, tissue-specific promoters, and virus-induced promoters. There are many means to transfer the vectors to the plants: transformation, transfection, injection, and electroporation, and lipophilic transfers.

The above methods can be tested and characterized to determine if the syngenic plants exhibit increased resistance to *Geminivirus, Nepoviruses, or other viruses utilizing ZFPs.* The syngenic plants can be monocotyledonous plants, such as Cassava, wheat, rye, or rice (Oryza), Sorghum, Zea (maize) and similar plants. The syngenic plants can also be dicotyledonous plants, such as **alfalfa, soy bean, tomato, sugar beet, potato**, as well as other plants and trees susceptible of introduction the *picolinic acid system* created by the inventors for the first time. The list of plants is only illustrative and not limiting. Numerous syngenic plants can be created with the Picolinic acid system presented in this invention. For example, ornamental plants can also be converted into syngenic plants. **Table 1 and 8** list a number of plants that are critical for the survival of millions of human beings who depend in these crops and that can be transformed in syngenic plants by the methods of this invention.

The invention can further and strongly enhanced the antiviral properties of Picolinic acid and derivatives by attacking zinc finger proteins overproduced by the plant and plant cells under the pathogenic control of various strong *Geminivirus* promoters which are necessary for virus survival, such as heat shock proteins (DnaJ, HSP70. etc), zinc finger ribosomal proteins with extra ribosomal functions such as MPS-1/S27, and numerous other ribosomal ZFP increased during the process of viral infection.

The inventors consider that picolinic acid and derivatives thereof have several pathogenic viral and plant targets which can be theoretically characterized as: 1) Main target: plant pathogenic virus ZFP such as *Geminiviruses;* 2) Secondary targets: *ZFP expressed by plant cells and plants that increase during viral infection under the control of the pathogenic plant virus.* The plant cells and plants producing ZFP interact with viral components which are necessary for viral survival. Thus, picolinic acid provides an additional advantage of disrupting zinc finger proteins required for plant virus proliferation, assembly and movement.

The present invention also provides another advantage of Picolinic Acid and derivatives thereof to increase the syngenic plant cells or plants with increased resistance to the pathogenic virus. In addition to the effects of Picolinic acid on ZFP of the plant viruses, it is well known that animal cells and plant cells express heat-shock ZFPs. In plant cells DnaJ proteins, which have a ZFP binding domain, interact with virus components. In animal cells, Picolinic acid inhibits the actions of animal DnaJ proteins (Fernandez-Pol, US Patent '393, Oct 3, 2000). Picolinic acid, being specific for interaction with zinc finger motifs of DnaJ proteins and other ZFPs which are expressed in the plant cells, can interact with ZFPs of the plant such as plant DnaJ proteins. *Thus, Picolinic acid has the ability* to *inhibit the important interaction between plant DnaJ proteins, which interact with pathogenic geminivirus components, and disrupts the subsequent interaction between DnaJ and viral proteins.* Thus, Picolinic acid and derivatives thereof can not only inhibit and disrupt the main target of Picolinic acid, the viral ZFPs of the plant viruses, but also secondary intracellular targets which are ZFPs produced by the plant and plant cells that are important for virus assembly, disassembly and movement by the vascular plant system.

The virus-resistant plant cells or plants enable to produce endogenous Picolinic acid are characterized by the following features of the **Cassette TRS construct**: **1**) they contain in the non-coding region of chromosomal DNA, the coding and/or regulatory sequences of the gene(s) for picolinic acid carboxylase (PAC) ; 2) this gene can be modulated to express the PAC enzyme which in the presence of the substrate (2-amino-3-carboxy-muconate semialdehyde) will produce picolinic acid (**FIG**. **17**) to disrupt endogenous viral ZFPs and plant cell ZFPs proteins that participate in viral replication. The plant ZFPs are not able to interact with viral components because of the presence of picolinic acid. Thus, picolinic acid disrupts the process of viral assembly by disrupting not only viral promoter ZFPs but also plant cell ZFPs which participate in the process of virus assembly, disassembly, and movement in the circulatory system of the plant carrying the ssDNA to other cells to perpetuate the viral infection. It will be appreciated that various changes and modifications may be made in the genetic engineering preparations and methods described and illustrated without departing from the scope of the appended claims. The preparations may be used to treat a ***wide spectrum*** of viral diseases mediated by ZFPs or other metal ion dependent proteins or enzymes. Therefore, the foregoing specifications and accompanying drawings are intended to be illustrative only and should not be viewed in a limiting sense.

These and other objects and characteristics of the invention will become more apparent when the following detailed description is combined with the drawings.

The present invention is directed to the production of plants which are resistant to multiple viruses. Most of the plants are susceptible to numerous viruses and other pathogenic agents such as fungus, bacteria and protozoaria. With advances in the identification of the sequences of specific transcription factors of pathogenic viruses and their respective promoters the inventors have designed cassettes constructs containing DNA sequences that can be incorporated into the plant genome to interrupt viral replication and to induced individual death of virally infected cells.

The present invention uses full genes or fragments of such genes to impart resistance to the plant against a plurality of viral pathogens. The cassettes are relatively easy and cost effective to produce. The cassettes include tandem repeated promoters for each of the upstream lethal genes which will be activated by the transcription factors of the pathogenic viruses. The promoter copies in tandem repeated sequences can range from 1 to 200 units separated by non-coding sequences. The cassettes contain promoters, several cell death genes. The cassettes can be incorporated in transformation systems to introduce the resistance cassette into the plant genome of interest. The reason for the essentially identically phenotype of the transgenic plant versus the wild type is due to the fact that the promoters are repressed in the transgenic plant and are only derepressed in the presence of an invading pathogenic virus releasing transcription protein factors which will derepressed and activate the upstream death genes. These and other characteristics of the invention will become apparent after the analysis of the transgenic plants.

This type of technology which provides one strategy for resistance to multiple viruses in transgenic plants possessing the integrated cassette units potentially will eliminate most if not all the pathogenic viruses that affect countless of agricultural edible crops.

For example, in the case of Geminivirus which infects and destroys Cassava plants, transgenic plants have been developed and found to be completely resistant under greenhouse and long term open field conditions. Since the Cassava strains from other parts of the world trigger the same resistance mechanism, no new or different Cassava plants will need to be developed with different transgenic plants viral protection systems.

Thus, the present invention provides resistance against a plurality of different viruses. However, the cassettes can be designed to impart resistance to one family of plants , in accordance with the versatility of this invention based on the tandem repeated promoter sequences and the associated tandem repeated death genes.

### BRIEF DESCRIPTION OF THE DRAWINGS

**FIG**. **1** is a schematic representation of the Cassava plant. The vectors that carry *geminivirus* and infect Cassava are also shown. The Cassava plant is made up of relatively few kinds of tissues. New cells are formed in meristematic tissue found at the tips of both the stem and the root systems. Meristematic cells have the capacity to continuously divide. *Geminiviruses* (GV) infect and replicate only in meristematic cells. Meristematic cells respond with cell division to viral infection, wounds and other types of injury to the plant. The Aphid vector of *Geminivirus* (GV) infects the leaves of the plant. The nematode vector of GV infects the apical portion of roots. Cassava synthesizes protein in both the leaves and the roots.
**FIG. 2****. African cassava mosaic virus. A. (top)** Symptoms in leaf of cassava *(Manihot esculenta)* naturally infected with the Kenya coast strain. **B. (bottom**) Symptoms in *Nicotiana benthamiana* systemically infected with the type strain.
**FIG. 3** illustrates that, in vascular plants, the tissue denoted xylem conducts water from the roots to all portions of the plant. The diagram of a photomicrograph shows the hollow dead cells that make up the xylem tissue. *Geminiviruses* are transported by both the xylem system and the Phloem tissues-sieve tubes which transport a variety of materials (not shown) throughout all portions of the Cassava plant.
**FIG. 4** illustrates the cells in plant leaves. Plant leaves use light energy to make carbohydrates. The Stoma is open to let gases in and out of the leaves. Picolinic acid as an aerosol can penetrate the stomata. Other picolinic acid derivatives soluble in the waxy coating of the leaves can penetrate the cuticle and reach the leave veins.
**FIG**. **5**. is a schematic representation of the MPS-1/S27 multifunctional ribosomal protein showing the coordination of the zinc atom to the sulfur atoms of cysteine residues.
**FIG. 6**. Molecular model of the region denoted zinc finger binding domain. This backbone model corresponds to the sequence of MPS-1. The atom represented by the dark circle is zinc which is coordinately bound to four sulphur atoms present in each of four cysteine residues. The cysteine residues interact non-covalently with the zinc atom.
**FIG. 7** illustrates the interactions between *Geminivirus* infection, ribosomal ZFP proteins, and heat shock proteins such as DnaJ which is a plant host protein involved in *Geminivirus* replication. (a) geometry of GV; (b) surface characteristics of GV. Cassava plant GV infection results in: 1. Damage to plant cell wall; 2. An active process of repair of the damage; 3. Initiation of meristematic tissue cell division; or 4. Program cell death.
**FIG. 8****.** Genotoxic stress in plants. As in the case of chemotherapeutic compounds (e.g. Platinum) and ionizing radiation, viruses such as *Geminiviridae* (GV) can produce genotoxic stress in plants. The GV are represented by geometric structures. MPS-1 is a ribosomal endonuclease enzyme which is expressed as a result of damage to DNA. MPS-1 is activated in response to signals generated by a variety of genotoxic stress agents, including GV plant cell infection. The intact MPS-1 pathway helps to maintain genomic integrity by eliminating defective mRNA molecules. This function of MPS-1prevents propagation of defective mRNA messages. If the damage to DNA induced by GV is reversible, the meristematic cell cycle arrests and the DNA damage are repaired. Irreversible damage to DNA induced by GV results in Program Cell Death and propagation of the GV to other cells.

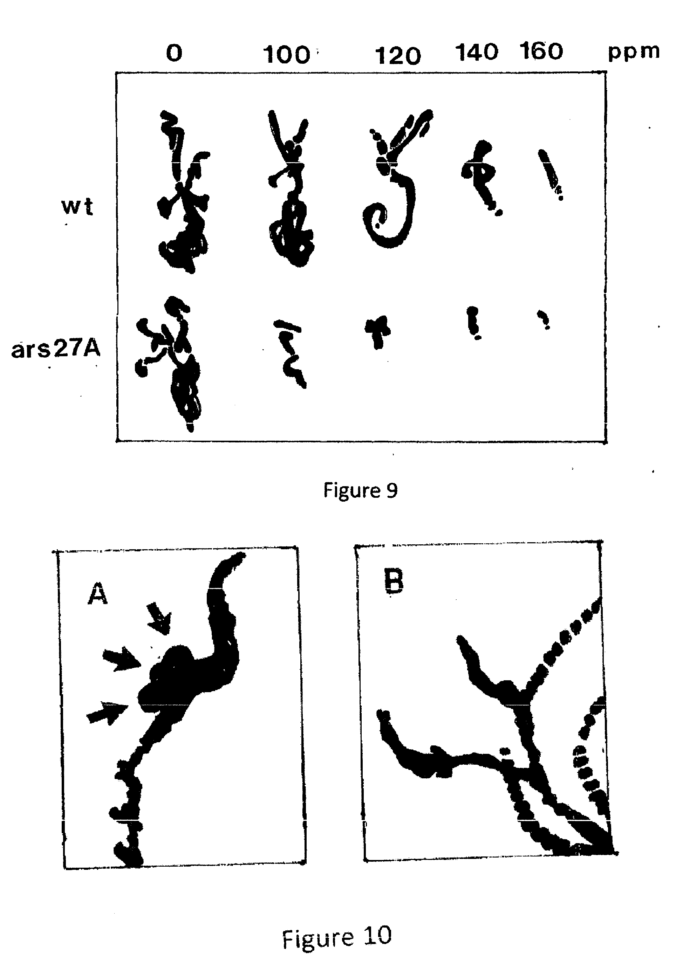

**FIG. 9** illustrates the genotoxic stress growth response to Methyl Methane Sulphonate (MMS) of both the wild-type and the ars27A mutant of MPS-1/S27 protein of the plant *Arabidopsis thaliana .* Seedlings were pre-germinated in the absence of MMS and transferred to liquid media containing MMS at the concentrations indicated in the figure. The photograph was taken after 3 weeks. This experiment shows that the mutant ars27A prevents both normal growth and size *of A. thaliana* ars27A as shown by the fact that growth and size of the plant were strongly inhibited by MMS in ars27A, at concentrations not affecting the wild type. Drawings are based on Revenkova et al, The EMBO J. Vol. 18:101-110, 1999.
**FIG. 10** illustrates that in plants, MPS-1/S27 ribosomal protein is essential for resistance to carcinogenic agents. This drawing shows the phenotype *of Arabidopsis thaliana* ars27A mutant in which MPS-1/S27 was deleted (**A**), and the phenotype of A. *thaliana* wild-type plant (**B**). Three-week-old seedling were grown for weeks two and three in the presence of Methyl Methane Sulphonate (MMS). (**A**) root of an ars27A seedling; (**B**) root of a wild-type seedling. Drawings are based on Revenkova et al, The EMBO J. Vol. 18:101-110, 1999. The mutation in ars27S is a deletion of the ribosomal protein MPS-1/S27. Thus, as shown in drawing A, the deletion of MPS-1/S27 in plants exposed to chemical carcinogens such as Methyl Methane Sulphonate (MMS) is accompanied by the formation of tumors instead of auxiliary roots (B).

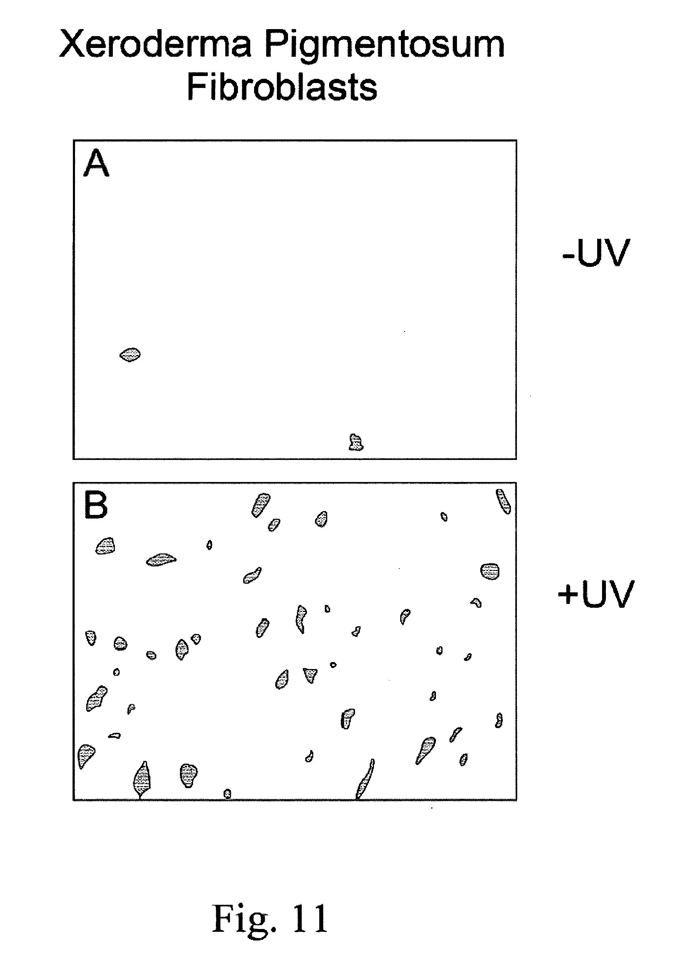

**FIG. 11** illustrates the effect of UVC radiation on the expression of MPS-1/S27 protein in Xeroderma pigmentosum (XP) cells. As is the case in plants such as in *Arabidopsis thaliana,* ribosomal protein MPS-1/S27 is essential for resistance to carcinogenic agents. This experiment was done with cells susceptible to genotoxic stress. Subconfluent cultures of XP-17BE cells were rinsed with Earl's balanced salt solution (EBSS), covered with EBSS, and immediately irradiated with UVC at room temperature. The cells were irradiated with 300 Joules/meter² with 254 nm UV bulbs (exposure time of approximately 8 sec). Control cells were identically treated but were protected with opaque covers during the irradiation process. Immediately after irradiation, the EBSS culture medium was removed and serum-free DME/F12+F medium was added. After 16 hours in a tissue culture incubator at 37°C, the cells were fixed and processed for fluorescence immunochemistry. Immunofluorescence staining was performed with affinity purified anti-peptide A rabbit antibodies at a 1:250 dilution. Detection of primary antibody was done with rhodamine-conjugated goat anti-rabbit IgG. (A) Control, non-radiated cells; B) irradiated cells. (Magnification 200X).
**FIG. 12** Illustrates external views of *Geminivirus* structures of the stained *African Cassava mosaic geminivirus* (ACMV). Particles were analyzed by electron microscopy and image reconstruction. (A) Surface representation of stained ACMV virus showing some of the capsomers; (B) diagram of the genomic organization of the DNA in upper and lower internal compartments of *Geminivirus* particles. Arrows indicate coiled DNA; the diagram is hypothetical. Both electron micrograph reconstructions (A, B) were adapted from: Virol. 2004 July; 78(13):6758-6765; 2004, AS for Microbiology. The ssDNA are hypothetical.
**FIG. 13** illustrates the genomic organization ofCsVMV according to the nucleotide sequence determined by *Kochko et al* (1998). The positions of the five open reading frames (*ORFs*) are indicated by arrows. Boxes represent conserved motifs of predicted proteins
**FIG. 14** : A conserved zinc finger motif is present in the **Coat Protein (CP)** *of Tomato leaf curl Bangalore virus.* This is responsible for binding to ssDNA. Computer search for DNA binding motifs in *begomoviruses* Coat Protein (CP). (Kirthi, N. and Savithri, H.S., Arch Virol (2003) 148:2369-2380). In Fig. 3 of the Kirthi et al paper, the authors performed multiple alignments of representative *begomoviruses* Coat Protein sequences in the region identified as containing a conserved zinc finger motif. The conserved zinc finger motifs are highlighted. The full names of the *begomoviruses* analyzed and their respective Coat Protein accession numbers in Gene Banks are listed as follows. '*' indicates identical residues, ':' indicates similar residues and '.' Indicates dissimilar residues. ToLCBV-[Ban 5] Tomato leaf curl Bangalore virus-[Ban 5] [AAK19178] AYVV Ageratum yellow vein virus [CAA52622] ToLCLV Tomato leaf curl Laos virus [AAF04152], tOlctwv Tomato curl Taiwan virus [AAB61142] PaLCuV Papaya leaf curl virus [CAA75884], ToLCBDV Tomato leaf Bangladesh virus [AAF04836], ToLCKV Tomato leaf curl Karnataka virus [AAB08929], CLCuMV_[Okr] Cotton leaf curl Multan virus-[okra] [CAA05469], CLCuMV-[62] Cotton leaf curl Multan virus-[62] [CAA0563], BYVMV-[Mad] Bhendi yellow vein mosaic virus-[Mad] [AAF63751], ToLCBV-[Kolar] Tomato leaf curl Bangalore virus-[Kolar] [AA126553], Tolcbv-[Ban 4] Tomato leaf curl Bangalore virus-[Ban 4] [AAD1286], Tolcbv-[Ban 1] Tomato leaf curl Bangalore virus-[Ban 1] [CAA88227], Tolcndv-[Luc] Tomato leaf curl New Delhi virus [Lucknow] [CAA7209], Tolcndv-Mld Tomato leaf curl New Delhi [mild][AA92817], ICMV Indian cassava mosaic virus [CAA80885], MYMV-Vig Mungbean yellow mosaic virus - Vigna [CAA10704],AbMV Abutilon mosaic virus [CAA34110], ToLCrV Tomato leaf crumple virus [AAD05245], ToMOTV Tomato mottle Taino virus [AAD09665], SLCV Squash leaf curl virus [AAC32411], CaLCuV Cabbage leaf curl virus [AAB17960], BGMV-[BZ] Bean golden mosaic virus [Brazil] [AAA46313], RhGMV Rhynchosia golden mosaic virus [AAF44668], TYLCSV Tomato yellow leaf curl Sardinia virus [CAA43467], SACM South African cassava mosaic virus [AAF34893].
**FIG. 15A and B**. All viruses depend on their ability to infect cells and induced them to make more virus particles. In virus-infected plant cells, the process denoted Program Cell Death (PCD) can be induced by destroying critical genes involved in viral cell growth. FIG. 15A, illustrates the action of picolinic acid on viral zinc finger proteins. The Zn²⁺ maintains the structure of a two zinc finger viral nucleoprotein. Chelation of Zn²⁺ by picolinic acid results in a denatured protein which is subsequently degraded by cellular proteases. **FIG. 15B****,** illustrates some of the pathways leading to apoptosis or program cell death induced by viruses in animal and plant cells.

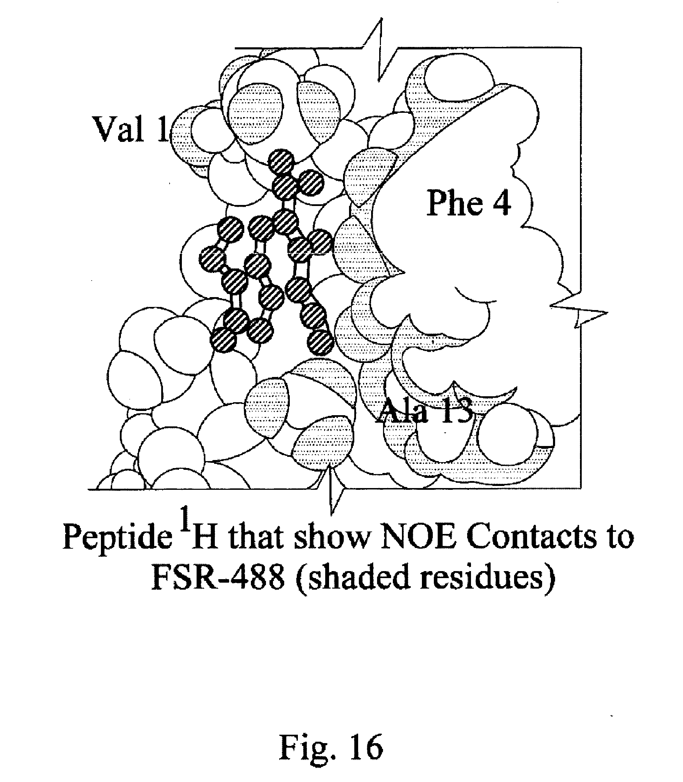

**FIG 16****.** illustrates the space-filling model obtained by NMR, which depicts the structure of the Zinc finger peptide bound to the nucleic acid binding domain. NMR of peptide ¹H shows NOE contacts with Fusaric acid (shaded residues). Fusaric acid experimental NMR solution was denoted FSR-488. FIG. 16.2, 16.3, and 16.4 illustrate various experimental results obtained by NMR of FSR-488 interacting with binding peptide and the nucleic acid binding domain.
**FIG. 17** illustrates the cell's biochemical pathway of production of Picolinate from tryptophan. The illustration shows the region J-1-2 of the Biochemical Pathways table, by Gerhard Michal, 1974 (Bushranger Mannheim GMBH- W.-Germany).
**FIG**. **18** illustrates the T-DNA transfer from *Agrobacterium* to plant cells. The chemical signaling events between *Agrobacterium* and the transformed plant cell consist of signals released from wounded plant cells undergoing the infection process. Opines are released from the wounded plant cells and activate receptors for opine functions, resulting in growth of the infecting bacteria. The Ver. gene region contributes with several products to the T-DNA transfer process. As shown in the scheme, the T-region of the Ti-plasmid codes for the synthesis of Phytohormones in the plant cells such as opines, auxins and cytokines. The T-DNA is shown in the plant cell incorporated into the plant DNA.
**FIG**. **19** illustrates the organization of viral resistance cassette, inserted into plant genome. The gene X can be picolinic acid carboxylase, cytochrome c, or other gene that induces lethality upon expression in the form of program cell death (PCD). The viral promoter tandem repeats gene has an alternation of transcription unit and nontranscribed spacer. For details see text.
**FIG. 20** illustrates the interaction between the viral resistant cassette (viral promoter tandem repeats in plant genome) with viral transcription factor which binds to viral promoter tandem repeats in the plant genome and transcribes viral genome or Gene X by binding to viral promoter sequences present in the cassette unit. Subsequently, Gene X is transcribed and translated and induces Programmed Cell Death of infected cells. The tandem repeats can be 50 to 100 or more. For details see text.

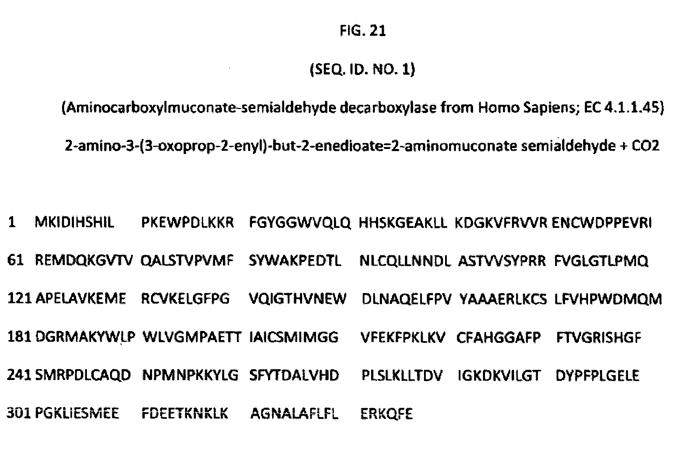

**FIG. 21** illustrates SEQ ID NO: 1, the polypeptide sequence of Picolinic Acid Carboxylase [PAC] (Aminocarboxylmuconate-semialdehyde decarboxylase from Homo Sapiens; EC 4.1.1.45. Accession code : Q8TDX5; Organism: Homo sapiens; number of amino acids 336; Molecular weight: 38035. Source: Swiss-Protein Bank.

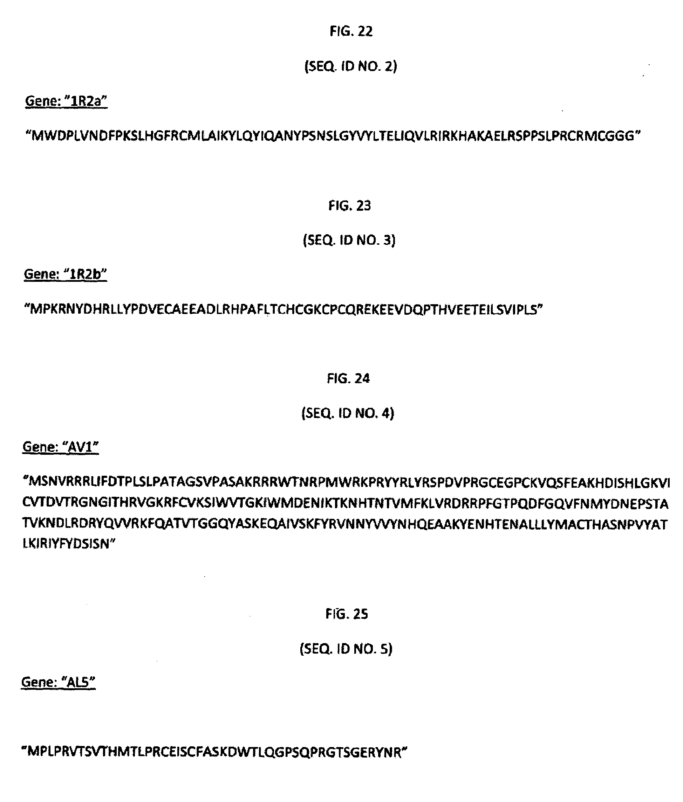

**FIG. 22** illustrates SEQ ID NO: 2, the entire polypeptide sequence of gene "1R2a" in Mungbean yellow mosaic, *geminivirus, begomovirus* DNA A acronym MYMGV; Gene Bank accession numbers gene ID: 991130; from positions 242 to 466. Protein id = "NP 077085.1".
**FIG. 23** illustrates SEQ ID NO: 3, the entire polypeptide sequence of gene "1R2b" in Mungbean yellow mosaic, *geminivirus, begomovirus* DNA A acronym MYMGV; Gene Bank accession numbers gene ID: 991131; from positions 402 to 593. Protein id = "NP 077086.1".
**FIG. 24** illustrates SEQ ID NO: 4, the entire polypeptide sequence of gene "AV1", in Mungbean yellow mosaic, *geminivirus, begomovirus* DNA A acronym MYMGV synonym: CP; gene ID:991132; from positions 442 to 1176. Protein id = "NP 077087.1. product = "COAT PROTEIN".
**FIG. 25** illustrates SEQ ID NO: 5, the entire polypeptide sequence of gene "AL5"; in Mungbean yellow mosaic, *geminivirus, begomovirus* DNA A acronym MYMGV, gene ID: 991133; from positions 570 to 707. Protein id = NP "0770877.1".
**FIG. 26** illustrate SEQ ID NO: 6, the entire polypeptide sequence of gene "AC5"; in Mungbean yellow mosaic, *geminivirus, begomovirus* DNA A acronym MYMGV, gene ID: 991134; from positions 820 to 1071. Protein id = "NP 077089.1".

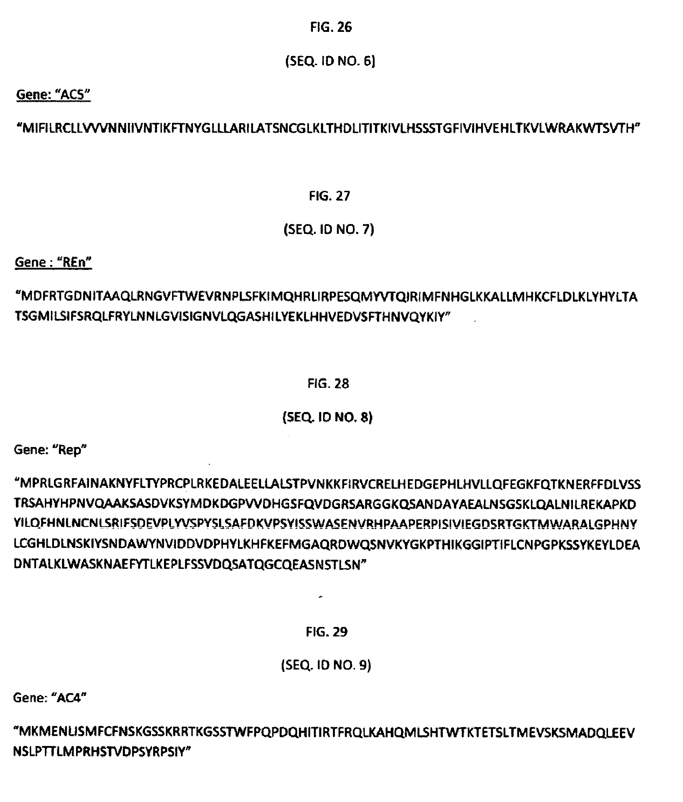

**FIG. 27** illustrate SEQ ID NO: 7, the entire polypeptide sequence of gene "Ren"; in Mungbean yellow mosaic, *geminivirus, begomovirus* DNA A acronym MYMGV, gene ID: 991135 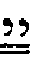 ; synonyms: AC3, AL3; from positions 1173 to 1577). Product = "AC3 PROTEIN"; Protein id = "NP 077090.1". *Note=* "AB017341 has pentanucleotide CACAG inserted in this position. As the result, ORF for the AC2 homolog, is restored in AB017341. Begomovirus AC2 is an ssDNA-binding protein, probable zinc-binding protein, probable phosphoprotein; localized to the nucleus; activates expression from the viral coat protein and BR1 movement gene promoters."; 1613 to 1614.
**FIG. 28** illustrate SEQ ID NO: 8, the entire polypeptide sequence of gene "Rep"; in Mungbean yellow mosaic, *geminivirus, begomovirus* DNA A acronym MYMGV, gene ID: 991136; from positions 1620 to 2705; Protein id = "NP 077091.1 "; product = "Potential NTP - binding protein", site specific endonuclease-ligase. "Required for the synthesis of the plus strand."
**FIG. 29** illustrate SEQ ID NO: 9, the entire polypeptide sequence of gene "AC4"; in Mungbean yellow mosaic, *geminivirus, begomovirus* DNA A acronym MYMGV, gene ID: 991136 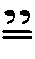 ; from positions 1620 to 2705; Protein id = "NP 077091.1".

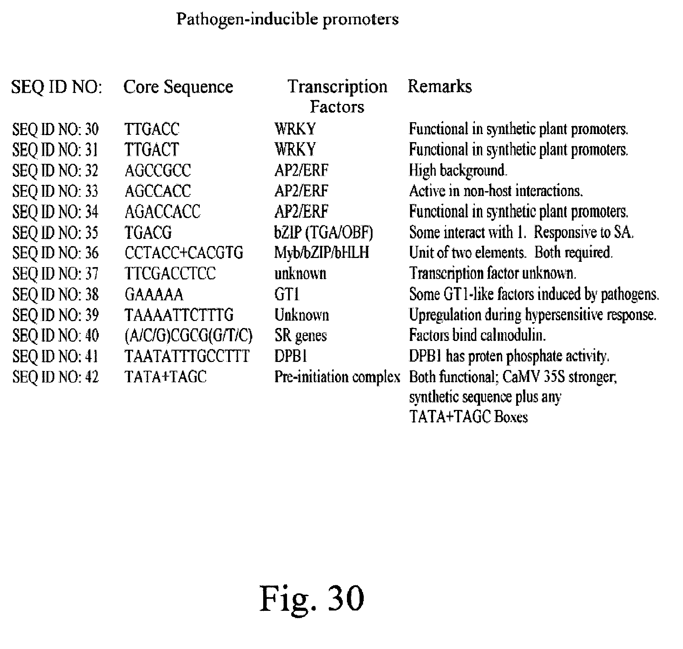

**FIG. 30** illustrate SEQ ID NO: 30 to SEQ ID NO: 42, corresponding to synthetic pathogenic transcription factors-Inducible promoters.

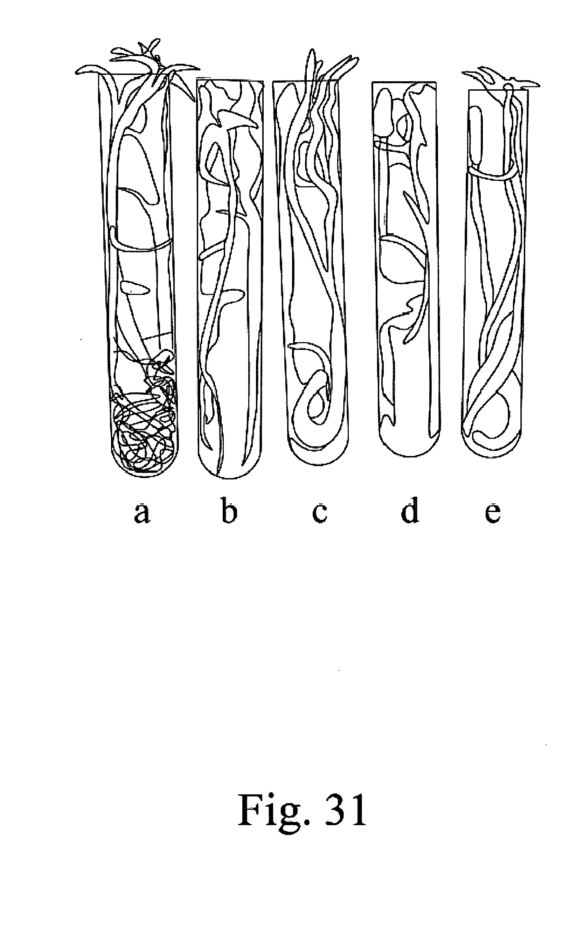

**FIG. 31** illustrates the induction by Picolinic Acid (PA) of *Portulaca-defence* related genes and subsequent resistant enhancement by PA against pathogenic fungus present in the test tube water. The PA-stimulated defense-response involves first, the hypersensitive response (HR), consisting in rapid generation of Reactive Oxygen Species (ROS). HR is a primary process of plant defense against pathogenic agents such as fungus, bacteria and other microbes. Following the HR, systemic acquired resistance (SAR) develops to prevent infection by virulent pathogens in roots or distal tissues such as leaves. Disease resistant could be enhanced again by periodic exogenous applications of PA. Pictures (a, c, and e), show fungal toxin disease resistant in Portulaca induced by treatment with picolinic acid for 10 days; note the profuse root system in plants continuously exposed to 6 mM PA. Pictures (b, d): Control, PA untreated plants: Fungal toxin disease developed in roots and leaves, destroying them, in *Portulaca* plants without PA treatment after 10 days. Note the lack of roots or the minimal development of them.

**Table 1**

| **Plant zinc finger proteins** | | | |
|---|---|---|---|
| ***Plant*** | ***Protein Mr, virus*** | ***Zinc finger protein*** | ***Reference No*.** |
| Hydrangea | No ZFP identified | | 79 |
| Prunus | Coat protein, 24.9 kDa,Prunus necrotic ringspot ilarvirus | | 80 |
| Allium sativum | Capsid protein | | 81 |
| Apple | Mosaic virus coat protein; Mr 25,056 | | 82 |
| Cauliflower | Resembles ZFP, highly conserved arrangement of cysteines and a histidine | | 83 |
| | ZFP motif and Zinc in alfalfa mosaic virus coat protein, strong inhibition by chelating agents | | 84 |
| Tobacco, Alfalfa | ZFP motif and Zinc in alfalfa mosaic virus coat protein, strong inhibition by chelating agents | | 85 |
| Tobacco, Alfalfa | ZFP motif in tobacco streak virus coat protein. TSV and AIMV contain 1 zinc atom per 4 protein subunit in TBS, and one zinc atom per 2 proteins subunits in AIMV. | | 86 |
| Apple | | | 87 |
| Phaseolus vulgaris L. | | | 88 |
| | | | 89 |
| Triticum aestivum | | | 90 |
| | | | 91 |
| | | | 92 |
| Garlic | | | 93 |
| Citrus | | | 94 |
| | | | 95 |
| Citrus | | | 96 |
| | | | 97 |
| Lily | | | 98 |
| Tomato | | | 99 |
| | | | 100 |

**Table 2**

| **Genome>*Viruses> East African cassava mosaic Cameroon virus DNA A*** | | |
|---|---|---|
| Lineage : Viruses; ssDNA viruses ; Geminiviridae; Begomovirus; East African cassava mosaic | | |
| Segments: DNA A, DNA B | | |

| **Genome Info** | **Features** | **BLAST homologs:** |
|---|---|---|
| Refseq: NC 004625 | Genes: 6 | COG |
| GenBank: AF112354 | Protein coding: 6 | 3D Structure |
| Length: 2,802 nt | Structural RNAs: None | TaxMap |
| GC Content: 45% | Pseudo genes: None | TaxPlot |
| % Coding: 91 % | Others: None | GenePlot |
| Topology: circular | Contigs: 1 | gMap |
| Molecule: ssDNA | | |

**Table 3**

| **Genome>*Viruses> East African cassava mosaic Zanzibar virus DNA B*** | | | |
|---|---|---|---|
| Lineage : Viruses; ssDNA viruses ; Geminiviridae; Begomovirus; East African cassava mosaic | | | |
| Segments: DNA A, DNA B | | | |

| ***Genome Info*** | ***Features:*** | ***BLAST homologs*** | ***Links*** |
|---|---|---|---|
| Refseq: NC 004656 | Genes: 2 | COG | Genome Project |
| GenBank: AF422175 | Protein coding: 2 | 3D Structure | Refseq FTP |
| Length: 2,763 nt | Structural RNAs: None | TaxMap | GenBank FTP |
| GC Content: 42% | Pseudo genes: None | TaxPlot | BLAST |
| % Coding: 61% | Others: None | GenePlot | TraceAssembly |
| Topology: circular | Contigs:l 1 | gMap | CDD |
| Molecule: ssDNA | | | Other genomes for species: 10 |

**Table 4**

| ***Genome>Viruses> Bhendi yellow mosaic virus, complete genome*** | | | | |
|---|---|---|---|---|
| Lineage : Viruses; ssDNA viruses ; Geminiviridae; Begomovirus; Bhendi yellow vein mosaic virus | | | | |

| **Genome Info:** | **Features:** | **BLAST homologs** | **Links** | **Review Info:** |
|---|---|---|---|---|
| Refseq: NC 003418 | Genes: 7 | COG | Genome Project | Publications: [1] |
| GenBank: AF241479 | Protein coding:7 | 3D Structure | Refseq FTP | Refseq Status: Provisional |
| Length: 2,741 nt | Structural RNAs: None | TaxMap | GenBank FTP | Seq.Status: Completed |
| GC Content 43% | Pseudo genes: None | TaxPlot | BLAST | Sequencing center: Madurai Kamaraj University, Plant Biotechnology, India, Madurai |
| % Coding: 89% | Others: None | GenePlot | TraceAssembly | Completed: 2000/04/13 |
| Topology: circular | Cotigs: 1 | gMap | CDD | Organism Group |
| Molecule:ssDNA | | | Other genomes for species: 1 | |

**Table 5**

| Topology of polypeptide binding and action of chaperone families | | |
|---|---|---|
| **Chaperone** | **Topology of binding** | **Action** |
| Hsp 100 | 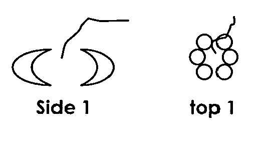 | ATP-dependent disaggregation and unfolding for degradation |
| Hsp 90 | Multiprotein complex | Conformational maturation of steroid hormone receptors and signal transducing kinases |
| Hsp 70 (DnaK) | 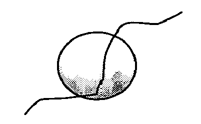 | ATP-dependent stabilization of hydrophobic regions in extended polypeptide segments |
| Hsp 60 (GroEL) | 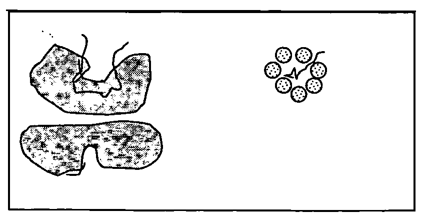 | ATP-dependent facilitation of folding to the native state |
| Small Hsps (Hsps25, etc) | 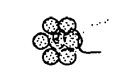 | Stabilization against aggregation during heat shock |
| DnaJ | Details on interaction of DnaJ with DnaK and GrpE can be found in Sebastian Fernandez-Pol and J. A. Fernandez-Pol, inventors, U.S. Patent, October 12, 2004, US 6,803,379 82, *Figure* 12. Substrate binding is located in the C-terminal domain, amino acids 200-376. DnaK interaction is present in amino acids 1-78. G/F-linker motif corresponds to amino acids 78-108. | DnaJ is a metalloprotein of 87 kDa which is essential for stimulation of the Hsp70 ATPasa activity. Amino acids 143-200 region contains one zinc-finger binding domain. |

Data for Table 5 was obtained from: *Schirmer et al.* 1996; Levchenko *et al.,* 1997; Bohen *et al.,* 1996; *Prodromou et al.,* 1997; Lee *et al.,* 1997; Ehmsperger *et al.,* 1997; Sousa and Parodi, 1995; Helenious *et al.,* 1997

### ZINC FINGER PROTEINS

**Table 6**

| **Examples of families of viruses using Zinc Finger Proteins, Zinc Ring Proteins or transition metal ion-dependent enzymes for replication and virulence.** | | |
|---|---|---|
| **Virus, protein, and Mr** | **Location and general characteristics** | **Protein Function and Specific properties** |
| *MPS-1*/*S27 [metallopanstimulin]Toxoplasma Gondii Mr= 10Kd* | Ribosomal and extra-ribosomal | ZFP binds mRNA and RNA |
| ***Rotavirus**, RoXaN, associated with NSP3, Mr=110 Kda* | Cellular protein tetratricopeptide | Five zinc finger motifs. Binds to protein, RNA and DNA. |
| Lambda-1, 140 Kd Rho-3, 41 Kd | Inner capsids Outer capsids | Zinc finger protein Binds dsDNA Zinc finger protein Binds dsDNA |
| NSP1, 53 Kd | Non-structural | Zinc finger protein RNA binding |

| ***Retroviridae*** | | |
|---|---|---|
| NCp7 (AIDS) | Nucleocapsid | Zinc finger protein RNA binding Required for inclusion of RNA in virions |
| 55 amino acids | | |
| TAT (AIDS) 82-101 amino acids | Regulatory protein | Cluster of 7 cystein residues Trans-activator |

| ***Papillomavirus*** | | |
|---|---|---|
| E6 | Regulatory protein | Zinc finger protein Transforming protein of HPVs Continuous cell proliferation Targets degradation of p53 |
| E7 | Regulatory protein | Zing finger protein Tranforming protein of HPVs continuous cell proliferation binds to the retinoblastoma protein, Rb |

| ***Adenovirus*** | | |
|---|---|---|
| | | Zinc finger protein gene expression transforming |
| E1A | Regulatory protein | protein |

| ***Hepatitis C*** | | |
|---|---|---|
| NS2 (+NS3) | Zn-dependent enzyme | Zn-metalloproteinase |

| ***Herpes viruses*** | | |
|---|---|---|
| HSV-1:ICPO protein | Regulatory protein | Zinc finger DNA-binding Trans-activation |
| HSV-2:MDBP protein | Regulatory protein Fe-dependent | Zinc finger protein ssDNA-binding DNA replication Synthesis of DNA |
| ICP6: Ribonucleotide reductase | enzyme | precursors Zinc ring configuration DNA binding Protein/protein |
| ***Equine Herpes virus***-1 ZR protein | Regulatory protein | interactions |
| ***Orthomyxoviriadae.*** Influenza virus matrix protein M1 | Tetrahedral coordination of two Cys residues and two His residues to a Zn(2+) ion in the central part of the peptide | M 1 protein is bound to Zn(2+) in the virion, and the Zn(2+)-bound M1 molecule may play a special role in virus uncoating. |

**Table 7**

| **Filovirus gene functions and relative molecular weights of gene products** | | |
|---|---|---|
| ***MW* (*kd*)** | ***Gene*** | ***Protein function*** |
| 90-104 | Nucleoprotein (NP) | RNA encapsidation |
| 35 | VP35 | Cofactor in polymerase complex |
| 35-40 | VP40 | Matrix protein; virion assembly and budding |
| 150-170 | Glycoprotein (GP) | GP = Virus entry (surface peplomer) |
| 50-60 | | SGP = Unknown |
| 24-25 | VP24 | Unknown |
| 270 | Polymerase (L) | Transcription and replication |
| 27-30 | VP30 | Ebola virus activator of viral transcription, RNA encapsidation |

Additional Information * on VP30 protein:
VP30 is an essential activator of viral transcription;
VP30 is closely associated with the nucleocapsid complex;
VP30 is an unconventional Zinc-binding Cys(3)-His motif comprising amino acids 68 to 95;
VP30-specific Cys(3)His motif;
Table 7 was modified from Fields Virology, 4th ed.; Vol. 1, Lippincott ***et al; Philadelphia.***

**Table 8**

| Examples of plants that can be infected with plant viruses containing ZFPs or use ZFPs from the host plant cells. | | | |
|---|---|---|---|
| ***Genome*** | ***Family*** | ***Genus*** | ***Type member*** |
| dsDNA | Caulimoviridae | Badnavirus | Commelina yellow mottle virus |
| | | Caulimovirus | Cauliflower mosaic virus |
| | | "Soybean chlorotic mottle virus-like" | Soybean chlorotic mottle virus |
| | | Cassava vein mottle virus-like | Cassava vein mottle virus |
| | | "Petunia vein clearing virus-like" | Petunia vein clearing virus |
| | | Rice tungro bacilliform virus-like | Rice tungro bacilliform virus |
| ssDNA | Germiviriade | Mastrevirus | Maize streak virus |
| | | Curtovirus | Beet curly top virus |
| | | Begomovirus | Bean golden mosaic virus |
| | No family | Nanovirus | Subterranean clover stunt virus |
| dsRNA | Reoviridae | Fijivirus Oryzavirus | Fiji disease virus Rice ragged stunt virus |
| | | Phytoreovirus | Wound tumor virus |
| | Partiviridae | Alphacryptovirus | White clover cryptic virus 1 |
| | | Betacryptovirus | White clover cryptic virus 2 |
| | No family | Varicosavirus | Lettuce big-vein virus |
| ssRNA (-) | Rhabdoviridae | Cytorhabdovirus | Lettuce necrotic yellow virus |
| | | Nucleorhabdovirus | Potato yellow dwarf virus |
| | | Unassigned | |
| | Bunyaviridae | Tospovirus | Tomato spotted wilt virus |
| | Nofamily | Tenuivirus Ophiovirus | Rice stripe virus Citrus psorosis virus |
| ssRNA (+) | Bromoviridae | Bromovirus | Brome mosaic virus |
| | | Alfamovirus | Alfalfa mosaic virus |
| | | Cucumovirus | Cucumber mosaic virus |
| | | Ilarvirus | Tobacco streak virus |
| | | Oleavirus | Olive latent virus 2 |
| | Comoviridae | Comovirus | Cowpea mosaic virus |
| | | Fabavirus | Broad bean wilt virus |
| | | Nepovirus | Tobacco ringspot virus |
| | Potyviridae | Potyvirus | Potato virus Y |
| | | Macluravirus | Maclura mosaic virus |
| | | Ipomovirus | Sweet potato mild mottle virus |
| | | Tritimovirus | Wheat streak mosaic virus |
| | | Bymovirus | Barley yellow mosaic virus |
| | | Rymovirus | Ryegrass mosaic virus |
| | Tombusviridae | Tombusvirus | Tomato bushy stunt virus |
| | | Carmovirus | Carnation mottle virus |
| | | Necrovirus | Tobacco necrosis virus A |
| | | Dianthovirus | Carnation ringspot virus |
| | | Machlomovirus | Maize Chlorotic mottle virus |
| | | Avenavirus | Oat chlorotic stunt virus |
| | | Areusvirus | Pothos latent virus |
| | | Panicovirus | Pamicum mosaic virus |
| | Sequiviridae | Sequivirus | Parsnip yellow fleck virus |
| | | Waikavirus | Rice tungro spherical virus |
| | Closteroviridae | Clostervirus | Beet yellow virus |
| | | Crinivirus | Lettuce infectious yellow |
| | Luteoviridae | Luteovirus | Barley yellow dwarf virus |
| | | Polerovirus | Potato leaf roll virus |
| | | Enamovirus | Pea enation mosaic virus-1 |
| | | Unassigned | |
| | No family | Benyvirus | Beet necrotic yellow vein virus |
| | | Carlavirus | Carnation latent virus |
| | | Potexvirus | Potato virus x |
| | | Tobamovirus | Tobacco mosaic virus |

**Table 9**

| ***Geminivirus* gene function Begomovirus** | | | | |
|---|---|---|---|---|
| **Function** | **TGMV,SqlCV, ACMV** | **TYLCV** | **Curtovirus** | **Mastrevirus** |
| **Replication** | Rep (AL1, AC1) | Rep | Rep (C1) | Rep (C1:2) |
| | Ren (AL3, AC3) | (C1) Ren (C3) | Ren (C3) | Rep A (C1) |
| **Transcription** | | | | |
| **Repress** | Rep (AL 1,AC 1), | | | |
| **RepActivate late** | (Al4, AC4) TrAP | TrAP | | |
| **(V) genes** | (AL2, AC2) | (C2) | | Rep A (C1) |
| **Encapsidation and insect** | | | | |
| **Transmission** | CP (AR1, AV1) | CP (V1) | CP (V1) | CP (V1) |
| | MP (BL1, BC1) NSP | MP (V2) | MP (V3) | |
| **Movement** | (BR1, BV1) | CP(V1) | CP (V1) | MP (V2) CP (V1) |
| **Host activation** | Rep (Al1, AC1) | | C4 | Rep A |
| **Symptoms** | MP (BL1, BC1) | C4 | C4 | MP (V2) |
| **Suppress host defenses** | TrAP (AL2, AC2) | | C2 | |
| **Regulate viral** | | | | |
| **ssDNA** | | CP (V1) | | |
| **accumulation** | CP (AR1, AV1) | MP (V2) | V2 | CP (V1) |

**Abbreviations for Table 9:** TGMV, tomato golden mosaic virus; SqLCV, squash leaf curl virus; ACMV, African cassava mosaic virus; TYLCV. Tomato yellow leaf curl virus; WDV. Wheat dwarf virus; MSV, maize streak virus; BCTV, beet curly top virus; PCNA, proliferating cell nuclear antigen. Activation of PCNA transcription, initiation of cell division, and/or bind Rb based on studies with TGMV.

**Table 10**

| | **Picolinic acid carboxylase** | | | |
|---|---|---|---|---|
| 1. | Oxidoreductases | | | |
| 2. | Transferases | | | |
| 3. | Hydrolases | | | |
| 4. | Lyases | | | |
| | 4.1. | Carbon-carbon lyases | | |
| | | 4.1.1 | Carboxy-lyases | |
| | | | 4.1.1.45 | Aminocarboxylmuconate-semialdehyde decarboxylase; **picolinic acid carboxylase; picolinic acid decarboxylase;** alpha-amino-beta-carboxymuconate-epsilon-semialdehyde decarboxylase; 2-amino-3-(3-oxoprop-2-enyl)but-2-enedioate carboxy-lyase. |

**Table 11**

| **Picolinic Acid Analogs** | | | |
|---|---|---|---|
| 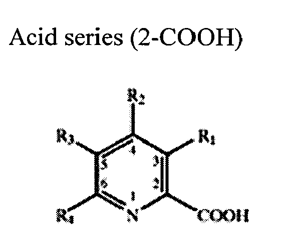 | | | |
| Numbers indicate position of the R residues in pyridine ring. | | | |

| 1 | 2 | 3 | 4 |
|---|---|---|---|
| CO2H | H | H | H |
| H | CO2H | H | H |
| H | H | CO2H | H |
| H | H | H | CO2H |
| CO2H | H | CO2H | CO2H |
| CH3 | H | H | H |
| H | H | H | CH3 |
| H | Et | H | H |
| H | H | n-Propyl | H |
| H | H | n-Bu | H |
| H | H | Et | H |
| H | H | n-Heptyl | H |
| H | H | 4-n-Hexylcyclohexyl | H |
| H | H | cyclohexyl | H |
| H | t- BuOCONHCH2 | H | H |
| H | H | H | F |
| H | Cl | H | H |
| H | H | H | Cl |
| Cl | H | Cl | H |
| Cl | H | H | Cl |
| Cl | Cl | Cl | Cl |
| H | H | H | Br |
| I | H | H | H |
| OH | H | H | H |
| H | H | OH | H |
| H | H | H | OH(=O) |
| nPrO | H | H | H |
| NH2 | H | H | H |
| H | NH2 | H | H |
| H | H | NH2 | H |
| H | NO2 | H | H |
| C6H5CH2S | H | H | H |
| CONH2 | H | H | H |
| H | H | CO2CH3 | H |
| H | H | H | CO2CH3 |
| H | H | H | CO2Et |
| C8H5CO | H | H | H |
| H | OH((C=O) | H | CO2H |
| Cl | NH2 | Cl | Cl |
| H | H | NO2 | CO2H |
| CO2H | H | H | CH3 |
| H | C6H5 | CO2H | H |
| H | CO2H | CO2H | CH3 |

**Table 12**

| ***Ribosomal protein MPS-1*/*S27 in genotoxic stress response in Arabidopsis thaliana*** | | | | | | | | | | |
|---|---|---|---|---|---|---|---|---|---|---|
| A | | 1 | | | | | | | | 86 |
| ARS27 | AF08 | MVLQN | NPPAE | HKLKR | NSFFM | GCFNI | HSQTV | CQTIL | GKAKL | FRRKG |
| A | 3336 | DIDLL | LEKRK | LVQSP | OVKCQ | TTVFS | VVCGN | CQPTG | TEGCS | D |
| ARS27 | T421 | MVLQN | HPPPE | HKLKR | NSFFM | GCFNI | HSQTV | CQTVL | GKARL | FRKK |
| B | 15 | DIDLL | LEKRK | LVQSP | OVKCQ | TTVFS | VVCGN | CQPTG | QEGCS | |
| ARS27 | AA71 | MVLQN | NPPAE | HKLKR | NSFFM | GCFNI | HSQTV | CQTLL | GKAKL | FRRKG |
| | 2867 | DIDLL | LEKRK | LVQSP | OVKCQ | TTVFS | VVMGN | CTPTG | TEGCS | D |
| ARS27 | AF08 | MVLQN | NPPAE | HKLKR | NSYFM | DCINI | HSQTV | CQNVL | GKARL | FRKLT |
| E | 3337 | DIDLL | LEKRK | LVPSP | VVRCS | TTIFS | VVCGK | CQPTG | TVGCS | D |
| Rice | D231 | MVLSN | NPPAE | HKKKR | NSFFM | GCFSI | HSQTV | CQTVL | GKARL | FRRKN |
| | 399 | DIDLL | LEKRK | LVQSP | OVKCQ | TTVFS | WCPG | CQPTG | TEGCS | D |
| Barle | X855 | MVLQN | NPPAE | HKKKR | NSFFM | GCFNI | HSQTV | CQTVL | GKARL | SVARA |
| y | 44 | DIDLL | LEKLK | LVQSP | OVKCQ | TTVFS | VVCPG | CQPTG | TEGSP | TKPVA |
| Rat | X593 | MPL- | HPSLE | HKKKR | NSYFM | GCYKI | HAQTV | CSTVL | GKARL | FRRKQ |
| S27 | 75 | ARDLL | EEKKK | LVQSP | DVKCP | TTVFS | VLCVG | CQPTG | TEGCS | H |
| Human | L197 | MPL- | HPSPE | HKKKR | NSYFM | GCYKI | HAQTV | CSTVL | GKARL | FRRKQ |
| MPS | 39 | ARDLL | EEKRK | LVQSP | DVKCP | TTVFS | VLCVG | CQPTG | TEGCS | H |
| Yeast | Z281 | MVLV- | HPTAA | HKLKT | RSYFL | GCLNI | HAQTA | CSTIL | GKAKL | FRRK |
| RPS27 A | 56 | QDLL | SEARK | LVQGP | DVKCP | TTVFS | VTCES | CTPTG | SEGTS | |
| Yeast | U103 | MVLV- | HPTAA | HKLKT | RSYFL | GCLNI | HAQTA | CSTVL | GKAKL | FRRK |
| RPS27 | 99 | -- | SEARK | LVQGP | DVKCP | TTVFS | VTCES | CTPTG | SEGTS | |
| B | | QDLL | | | | | | | | |
| **CONSENSUS** | | **-----** | **- P-** | **HK-** | **- S-** | **GC- -** | **H- QT** | **C- T-** | **GKA-** | **-----** |
| | | **-----** | **- - -** | **K-** | **F-** | **ITTVF** | **- V-** | **LC-** | **L-** | **-----** |
| | | **--DLL** | **E- -** | **LVQ-P** | **DVKC-** | **S** | **C--** | **PTG** | **EG--** | **-----** |
| | | | **X** | | | | | | | **-** |

Sequence alignment of ARS27A and selected homologs of the ribosomal protein S27. Amino acid sequences were deduced from cDNA sequences [ARS27A, ARS27B, ARS27C A. thaliana mRNAs; rice, Orysa sativa mRNA from callus; barley, Hordeum vulgare root mRNA; rat, Rattus rattus mRNA for ribosomal protein S27 (Chan et al., 1993); human metallopanstimulin (MPS-1) mammary gland carcinoma mRNA (Fernandez-Pol *et al.,* 1993)] or genomic sequences [yeast, S. cerevisiae RPS27A and RPS27B (Baudin-Baillieu *et al.,* 1997) and ARS27Ψ, deduced from the A. thaliana genomic sequence]. GenBank/EML accession Nos indicated.

### DESCRIPTION OF THE INVENTION

Picolinic acid, a metal chelating, naturally occurring, biological compound, inhibits the growth of numerous cultured normal and transformed mammalian cells. It has been shown that short-term treatment with picolinic acid arrests normal cells in G1 (Go) while transformed cells are blocked in different phases of the cell cycle. With longer exposure to picolinic acid cytotoxicity and cell death was observed in all transformed cells whether they were blocked in G1, G2 or at random. In contrast, most normal cells showed no toxic effects from the picolinic acid. Thus, the selective growth arrest and the differential cytotoxicity induced by picolinic acid reveals a basic difference in growth control and survival mechanism(s) between normal and transformed cells.

Kinetic and radioisotopic studies show that picolinic acid both inhibits incorporation of iron into the cells and effectively removes radioiron from the cells. Hence, it is conceivable that the inhibition of cell proliferation in vitro and in vivo by picolinic acid results, at least in part, from selective depletion of iron from the cells.

It has also been shown that picolinic acid may arrest prokaryote and eukaryote cell growth by inhibiting Zn-requiring enzymes. In addition to its chelating ability, picolinic acid has a number of biologic properties including inhibition of ADP ribosylation and ribosomal RNA maturation, modulation of hormonal responses, and macrophage activation. Picolinic acid in combination with interferon gamma can inhibit retroviral J2 mRNA expression and growth in murine macrophages. Thus, picolinic acid and its derivatives can act as *biological response modifiers.*

The inventors have determined that picolinic acid and fusaric acid inhibit the zinc dependent binding of recombinant MPS-1 to DNA, as determined by gel shift assays and the data correlates with the absence of radioactive Zn65 from recombinant MPS-1 protein. MPS-1 is a ubiquitous tumor marker and cell growth stimulator and is described in detail in the inventors' U.S. Pat. No. 5,243,041. MPS-1 has one zinc finger domain of the type CCCC. Picolinic acid and fusaric acid react with the CCCC zinc finger to remove radioactive Zn65 from MPS-1. This is detected by a change in the electrophoretic mobility of MPS-1 under non-denaturing conditions. These experiments indicate that picolinic acid and derivatives should remove zinc and denature various types of zinc finger, zinc ring proteins, or other types of ZF motifs whether known or heretofore undiscovered, including animal or plant viral proteins such as nucleocapsid proteins of retrovirus or the nucleocapsid proteins of *Geminivirus.* Furthermore, the inventors have determined that any chemical compound, whether known or heretofore undiscovered, that will remove the zinc (or other metal) and denature the proteins or that will form a ternary complex (protein-zinc-chelator) can be effective as an antiviral agent, with nontoxic effects, provided that the agent conforms with certain specific structural requirements.

In accordance with the present invention, a composition can be prepared to successfully treat a wide range of viral diseases. The compositions of the invention comprise picolinic acid, fusaric acid, or any pharmacologically acceptable salt or derivative thereof. Fusaric acid is the 5-butyl derivative of picolinic acid. The structure of fusaric acid and many of its derivatives is represented by the structure shown in **FIG**. **16**. Wherein R1, R2, and R4, are selected from the group consisting of a peptide of sixteen amino acids, carboxyl group, methyl group, ethyl group, propyl group, isopropyl group, butyl group, isobutyl group, secondary butyl group, tertiary butyl group, neopentyl group, fluorine, chlorine, bromine, iodine, and hydrogen and R3 is a butyl group.

Fusaric acid (FU) (5-butyl picolinic acid) is an inhibitor of animal and plant cell cancerous growth. In particular, FU is potent inhibitor of virally transformed mammalian cell lines in tissue culture. US Pat. Nos. 5,767,135 and 6,127,393, herein incorporated by reference in their entireties, show the usefulness of fusaric acid as a potent anticancer agent in animal cells in vivo.

Fernandez-Pol *et al* have observed synergistic effects when fusaric acid, and pharmaceutically acceptable derivatives thereof are combined with antiviral drugs.

Fusaric acid is effective against viral infections by HSV-1 and HSV-2 in tissue culture and in animals (Fernandez-Pol, Anticancer Res., 2002). Additional antiviral compounds of the invention may be prepared by methods that are well known in the art.

The relative concentrations of picolinic acid, fusaric acid and derivatives thereof alone or in combination with other drugs are dependent upon the specific use for the compositions of the invention, e.g., the nature or virulence of the virus infecting the plant, such as a *Geminivirus* infecting Cassava crop plants; and the relative age, conditions and degree of development of the plant. The method of administration or carrier employed is also important.

In general, the compositions of the invention are comprised in the range of about 0.01 % to 99% Fusaric acid. In another composition of the invention the range is estimated to be about 0.1 % to about 2% fusaric acid alone. It will be appreciated by those skilled in the art that compositions and concentrations outside the stated ranges are within the scope of the present invention. The foregoing compositions of the invention may also be mixed with other therapeutic compounds to form pharmaceutical compositions (Fernandez-Pol *et al.* US Pat. Dec. 4, 2003). *"Pharmacological agents and methods of treatment that inactivate pathogenic prokaryotic and eukaryotic cells and viruses by attacking highly conserved domains in structural metalloproteins and metalloenzyme targets ".* For application to plants under certain conditions it may be necessary to suspend the antiviral agents in, for example, microcrystalline cellulose, agar-agar, and any combination or mixture of these substances or others not listed here. Any pharmaceutical carrier suitable to permit drug administration and penetration into the plants organs may be used.

### Background on Geminivirus

*Geminivirus is* the most common organism found in plantations of Cassava around the world. It plays a major role in the pathogenesis of Cassava mosaic disease. Presently available topical, irrigation by the roots, and genetic engineering produced preparations of genetic vectors as antiviral agents exert their therapeutic effect through competition, inhibition, and utilization of critical pathways usually controlled by the Wild Type *Geminiviruses* strains. The genetic engineering produced materials are eventually unstable. The instability leads to the return and more virulent invasion by the Wild Type *Geminiviruses* strains. Thus, although the ideas behind these genetic modification techniques appear to be solid and viable, the evidence demonstrates that the selective Darwinian pressures predominate with, in many cases, consequences, such as famine. To complicate the issue, the most virulent wild-type *Geminiviruses* are able to invade other species of plants. Problems arise from limited experimentation in the laboratory and wide-spread use of new genetic materials in large crop fields. Thus, under the existing art, genetically-modified plants can present a biohazard. In contrast the instant invention is such that the likelihood of generating a similar biohazard to the crops is remote. The reason is that the agents used under the invention are naturally occurring compounds that produce lethal destruction of zinc finger motif, thus, preventing any further Darwinian selection of viral species.

Picolinic acid and its derivatives offer a powerful, rational and inexpensive alternative to controlling or treating *Begomoviruses* and essentially any other virus that utilizes either viral or plant zinc finger proteins. Picolinic acid and some of its derivatives and their uses treating certain animal and human diseases were described in Fernandez-Pol, J. A., U.S. Pat No. '393, October 3, 2000, and is hereby incorporated by reference. As the need for new compounds to treat plant viruses becomes apparent, the inventors have created new structures of matter in the last few years (2002-present).
Since that time, novel compounds of Picolinic acid and its derivatives were invented. These compounds are represented by the following structure: 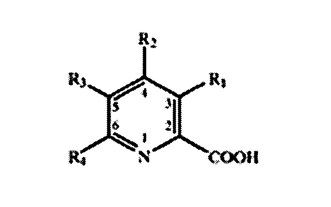 wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen. It is evident for those initiated in the art that novel compounds can be created from the formulas delineated above. For example, substitution of R1 by a peptide of five to 21 amino acids can result in stronger formation of ternary complexes with *Geminiviruses* zinc finger proteins.

The present invention provides pharmaceutical compositions for controlling or treating Begomoviruses and *Geminiviruses* comprising a compound having the following structure: 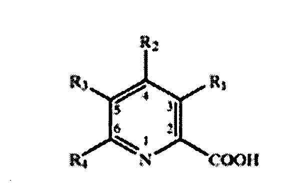 wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen, and wherein the composition reduces, destroys or inhibits viral growth *Begomoviruses* and *Geminiviruses* by at least one to two orders of magnitude, resulting in viral inactivation and Program Cell Death of the plant cell.

In one embodiment, the composition further comprises propylene glycol, ethyl alcohol, hydroxyethyl cellulose, sodium chloride, and water. Preferably, **R₃** [position 5 of the pyridine ring] of the compound is a butyl group. More preferably, the compound is picolinic acid, fusaric acid (5-butyl picolinic acid), or derivatives thereof. In another embodiment of the present invention, the composition comprises about 0.001% to about 100% of picolinic acid. Preferably, the composition comprises about 2 to 10% of picolinic acid. Unless otherwise stated herein, the percentage of a component refers to the component's percent by weight to the total weight of the composition. In still another embodiment, the composition further comprises another compound (s) to enhance the formulation described here (Fernandez-Pol, *et al* US Pat. Dec. 4, 2003).

Picolinic acid drug substance (also referred to herein as PA) is an anti-infective and immunomodulator in animal cells. PA is a metabolite of the amino acid tryptophan. It is produced in approximately 25-50 mg quantities by the body on a daily basis, by the breakdown of tryptophan, assuming normal dietary intake. PA plays a role in zinc transport and also in transition metal (TM) ion transport such as Cu²⁺, Mn²⁺, and other transition metal ions. As a therapeutic agent, the mechanism of action of picolinic acid and derivatives thereof involves disruption of zinc binding in zinc finger proteins (ZFPs), formation of ternary complexes and exchange of iron (Fe ²⁺) by Zn²⁺ in ZFPs transforming them in "iron finger proteins". ZFPs are critically involved in animal cell, plant cell and, in viral replication and packaging of retroviruses and plant viruses such as *Geminiviruses.* Picolinic acid, Fusaric acid, and numerous derivatives thereof, have been shown to be powerful antiviral agents in *vitro* and *in vivo,* and they also modify the immune response in animal and human cells, alone and in conjunction with other cytokines such as interferon gamma. The main target as a biological response modifier is the activation of macrophages, which perform a myriad of functions essential for homeostasis. Plant cells do not appear to posses any immunological system. It is the belief of the inventors that while the characteristics of plant "immunological" system may not resemble the same system in animal cells, there is a degree of functional equivalence.

AFRI-P (Picolinic Acid) product was formulated for the prevention of *Geminiviruses ACMV and EACMV Cassava mosaic virus.* It is estimated that daily spray applications of 1% to 2% Picolinic Acid (AFRI-P) in liquid form would result in delivery of approximately 42ug to 84 ug [3 mM to 6 mM, respectively] to the surface of the plant leaves per application. The result of cumulative pilot studies for seven days in leaves of live plants showed no irritation. To assess the pharmacokinetics of the product, levels of AFRI-P in leaves will be determine in dose and time-dependent studies performed with [H3]-tritiated- Picolinic acid (treated) and [H3][Picolinamide (control, inactive). These studies will allow a more precise pharmacokinetics study of penetration of Picolinic acid as well as safety information on the formulation.

In one embodiment, the therapeutic composition (consisting in a spray of 0.1 % to 0.5% Fusaric acid) inhibits *Geminivirus* lesions which are mild to moderate. Preferably, the new composition reduces at least about 98% (in one week) of the total Cassava lesions induced by the *Geminivirus.* Total Cassava viral lesions comprise every region of the plant that shows evidence of Cassava mosaic virus disease.

In another embodiment, the composition is a spray for topical application containing 0.001 to 1.0 agar plus 10 mM Picolinic acid preparations embedded in the agar. The topical preparation is designed to coat the plant with the formulation to allow longer times of penetration and exposure of the agent with no toxic *effects. It is worth to mention here that Picolinic Acid, Fusaric* acid and *numerous derivatives thereof are devoided **of photosensitization** activity or **antiviral resistance,** which provides an advantage* when higher doses should be used in severe cases of Cassava mosaic virus disease. Moreover, the compositions do not prevent respiration by the leaves. Thus, the composition does not contain any chemical at concentrations that would be effective to block any physiological function of the plant organs. More preferably, the compositions are rapidly absorbed and *destroy the Geminivirus in dividing cells.*

Pharmaceutically acceptable salts of picolinic acid and its derivatives thereof may also be used, and can be prepared from pharmaceutically acceptable non-toxic acids or bases including, but not limited to, inorganic and organic acids. Buffering agents for picolinic acid or its derivatives may also comprise non-toxic acids or bases including, but not limited to inorganic or organic acids. Examples of such inorganic acids include, but are not limited to hydrochloric, hydrobromic, hydroiodic, sulfuric and phosphoric. Organic acids may be selected, for example, from aliphatic, aromatic carboxylic, and sulfonic classes of organic acids. Examples of suitable organic acids include but are not limited to formic, acetic, propionic,succinic, glycolic, gluronic, maleic, furoic, glutamic, bezoic, anthralinic, salicylic, phenylacetic, mandelic, embonic (pamoic), methanesulfonic, ethanesulfonic, pantothenic, benzenesulfonic, stearic, sulfanilic, algenic and galacturonic acids. Examples of such inorganic bases for potential salt formation with the sulfate or phosphate compounds of the invention include, but are not limited to monovalent, divalent, or other metallic salts made from aluminum, calcium, lithium, magnesium, potassium, sodium and zinc. Appropriate organic bases may also be selected from N₂N-dibenzylethylenediamine, chloroprocaine, choline, diethanolamine, ethylenediamine, meglumaine (N-methylglucamine), procaine, ammonia, ethylenediamine, N-methyl-glutamine, lysine, arginine, orithine, choline, N,N'-dibenzylethylene-diamine, chloroprocaine, diethanolamine, procaine, N-benzylphenethylamine, diethylamine, piperazime, tris (hydromethyl)aminomethane and tetramethylammonium hydroxide. Carboxylic acid and its derivatives are also contemplated as being within the scope of the invention. Further experimental information will be presented in Examples.

The examples described herein provide guidance for determining the efficacy of various *formulations. Some formulations may comprise within the range of about 0.001% to about 20%* of the composition, although higher concentrations may be useful in certain formulations. Preferably, the concentration of the composition ranges about 0.0 1 % to about 10%. More preferable, the formulations comprises within about 1% to about 10% of the composition. For certain compounds such as picolinic acid, the formulation may be solid and applied directly to the plant prior to neutralization of the acidity of PA to physiological pH.

Depending on the particular intended use for control of **Cassava mosaic virus** disease, the composition may be formulated into a variety of media such as, but not limited to, a cream, gel, paste, aerosol, solution, soap, shampoo, powder, liquid, or any other formulation capable of delivering the active agent to the affected area of the plant. Preparations to be absorbed by the roots are also contemplated. The affected area may be any part of the Cassava plant.

The present invention further provides a method for treating, controlling, destroying or disintegrating *Geminiviruses* infecting Cassava, comprising administering to the plants afflicted with these viral diseases a therapeutically effective amount of a composition comprising a compound having the following structure: 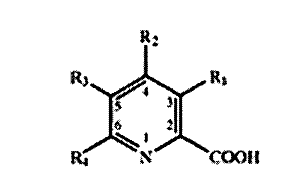 or a pharmaceutically acceptable salt thereof, wherein R₁, R₂, R₃ and R₄ are selected from a group consisting of a carboxyl group, methyl group, ethyl group, propyl group, isopropyl group, butyl group, isobutyl group, secondary butyl group, tertiary butyl group, pentyl group, isopentyl group, neopentyl group, fluorine, chlorine, bromine, iodine, and hydrogen and wherein the composition reduces, disintegrates or inhibits viral growth of *Geminiviruses.*

Treating or treatment, as used herein, refers to preventing a disease or symptom from occurring in plants or plant cells, inhibiting a plant disease or symptom from further development, or relieving the disease or symptom. Also disclosed are methods for treating *Geminiviruses* and other viruses which use viral zinc finger proteins by the administration of a composition comprising picolinic acid or derivatives thereof. The composition may be administered by any known means used in agriculture or biotechnology, including administration directly to the affected area by simple techniques such as spraying or more complex procedures such as electrostatic fields to facilitate the transfer of the pharmaceutical agents into the circulatory system of plants or plant cells.

In one embodiment, the method comprises administering the composition at least once daily to the *geminivirus infecting cassava.* Preferably, if the viral infection is severe, the composition can be administered to the plant or plant cells twice daily. More preferably in this case, the composition comprises about 10% picolinic acid and is administered to the plant or plant cells twice daily. Depending upon the extension of the cassava viral disease, the composition may be administered for any number of weeks, preferably for at least four to eight weeks.

### CONTROL OF GROWTH BY PICOLINIC ACID AND DERIVATIVES THEREOF IN PLANT AND ANIMAL CELLS: DIFFERENTIAL RESPONSE OF NORMAL AND TRANSFORMED PLANT AND ANIMAL CELLS

The antiviral agent Picolinic acid (PA) is a metal-chelating agent. It is structurally related to nicotinic acid, a precursor in the biosynthesis of NAD⁺. Cell transformed by different viruses responded differently to picolinic acid, and cell lines from different species transformed by the same virus blocked in similar manners (For details see Fernandez-Pol et al Proc. Natl Acad. Sci. USA 774 (1977).

Picolinic acid is an endogenous metabolite of tryptophan synthesized by the kynurenine pathway, like quinolinic acid and nicotinic acid (FIG. 17). The functions of both picolinic acid and nicotinic acid are essential for controlling growth and to produce energy for charging the cells membranes in both plant and animal cells, respectively (**FIG. 17**). In a simplified sense, they have opposite effects. While picolinic acid is an inhibitor of growth and modulator of hormonal responses, nicotinic acid by its participation in NAD+ nucleoside, controls the flow of energy in all cells and thus is essential for life (**FIG. 17**).

**FIGURE 17** shows that in the catabolism of tryptophan there is a branch point that leads either *to picolinic acid or quinolinic acid.* Picolinic acid is an end product whereas quinolinic acid is further metabolized to nicotinate nucleotide, an important enzymatic cofactor (NAD+. Nicotinate nucleotide is converted to nicotinate (nicotinic acid: 3-pyridine carboxylic acid) by the enzyme nicotinate phosphoribosyl-transferase. Nicotinate is converted to nicotinamide and after a series of enzymatic steps is converted to Nicotinamide Adenine Dinucleotide-P (NADP).

The results show that Picolinic acid has several simultaneous actions all related to the cell cycle: 1) PA binds to transcriptionally active species of protein-metal ion complex in a manner similar to the binding of nicotinic acid to leghemoglobin, a plant protein; 2) PA alters the activity of the ADP-ribose moiety of (NAD+) polymerase, the enzyme that catalyzes the polymerization of the ADP-ribose moiety in NAD+ in both animal and plant cells; 3) PA alters the response to growth factors in animal serum and plant culture media. For example, prostaglandin E1 is two to five times more potent in elevating cyclic AMP if the cells are pre-treated with Picolinic acid. Cyclic AMP is the mediator of the action of beta adrenergic agents.

In recapitulation, the results of these extensive studies (Fernandez-Pol, Cancer Genomics and Proteomics, 2003) established that PA has effects on the cell cycle that are viral-transformation dependent, may involved NAD+ metabolism and more significant can interact with essential proteins denoted ZFP which are integral parts of at least two thirds of all the viruses. Pathogenic viruses that do not possess ZFP, induce cellular ZFP by activating the cellular promoters with viral enhancer promoter proteins. The results of this study clearly shows that PA has effects in the cells that are viral-transformation dependent. Furthermore, picolinic acid is a component of a *normal physiological regulatory mechanism* that is altered by viral transformation in both animal and plant cells.

Picolinic acid is produced by activated macrophages which have important functions in the body. Picolinic acid is a co stimulus with interferon gamma for the induction of reactive nitrogen intermediate (NO₂⁻) in macrophages. In addition to other therapeutic actions, the biological effects exerted by picolinic acid in vitro and in vivo indicate that picolinic acid can have potential therapeutic applications in stimulation of macrophage functions such as phagocytosis. Extensive research in being carried out in these areas.

Zhang et al (Acta Botanica Sinica; 2004, Vol. 46, No.10, P. 1200-2005) presented data on Alpha-picolinic acid related to the diverse defense responses of Salicylic acid, Jasmonic acid/ethylene- and Ca²⁺ -dependent pathways in *Arabidopsis* and Rice suspensions of cells. Zhang et al found that in plants, Alpha-picolinic acid (2-pyridine carboxylic acid); can elicit the **hypersensitive response (HR)** *in rice,* a monocotyledonous model plant and also a plant of enormous economic value. Thus, PA is an HR inducer in plants. In fact, PA is an HR inducer in essentially every plant in which it was properly tested. It also induced HR in *Arabidopsis* thaliana, a dicotyledonous model plant, including the oxidative burst and cell death. *Zhang et al.,* investigated in detail the defense signal which was transduction activated by Picolinic acid. For this purpose, marker genes of particular defense pathways were studied in *Arabidopsis.*

The results of experiments with *Arabidopsis* indicated that both the salicylic acid-dependent and jasmonic acid/ethylene-dependent pathways were strongly activated by Picolinic Acid. In these experiments the marker defense genes PR-1, PR-2 and PDF1.2 were all induced by picolinic acid in a dose-dependent and time dependent manner. In rice suspension cells, PA induced reactive oxygen species (ROS) which was Ca²⁺ - dependent. Zhang et al, confirmed and extended previous studies of PA-induced defense activation in rice. The researchers concluded that PA acts as a nonspecific elicitor/stimulator in plant defense gene systems, and that has the potential utilization in cellular models for the establishment of systemic acquired resistance (SAR) activation in a systematic basis. Furthermore, the results strongly suggest that Picolinic acid may be used in rice fields to increase the resistance of rice crops to pathogenic prokaryote or eukaryote life forms attacking rice.

*Zhang et al* (2004) identified Picolinic acid as a fungal toxin (in rice culture media) and an enhancer of disease resistance in rice (Zhang et al, Cell Research (2004); 14:27-33). PA has been reported to be a toxin produced by certain plant fungal pathogens and is used for screening of disease resistant mutants. PA is an efficient apoptosis agent triggering cell death of hypersensitive-like response in plants. These results were confirmed by Fluorescence Activated Cell Sorter. Rice suspension cells and leaves showed apoptosis induced by PA. The PA-induced cell death was associated with accumulation of reactive oxygen species and was NADPH-oxidase dependent.

In summary, *Zhang et al* demonstrated the induction of rice defense-related genes by picolinic acid and resistance enhancement by PA against the rice blast fungus *Magnaporthe grisea.* Thus, in PA treated plants, the resistance against *M. grisea* is enhanced. The doses of PA utilized were in the same range used in mammalian tissue culture (0.5 mg/mL).

Fusaric acid is a potent inhibitor of cancer cell growth and viral proliferation. Fusaric acid, a picolinic acid derivative, metal ion chelator, shows an effect on the growth and viability of normal and cancerous cells in tissue culture. Examples presented here show that fusaric acid has potent anti-cancer and anti-viral activity in vitro.

Moreover, *fusaric acid can be useful in the treatment of virally-induced plant diseases such as the Cassava mosaic disease (CMD)* in vivo without substantially damaging living normal plant cells. CMD is caused by viruses belonging to the genus *Begomoviruses* of the family *Geminiviridae.* The genomes of the *Geminivirus* that produced CMD disease code for zinc finger proteins that are critical for viral proliferation.

Fusaric acid is the 5-butyl derivative of picolinic acid. Its structure is shown in Table 11. Fusaric acid was recognized in the early 1960s to have activity as an antihypertensive agent in vivo. Fusaric acid and its properties can be summarized as follows. The drug interacts with various metalloproteins and metal ion-requiring enzyme systems. Fusaric acid is noted to be an inhibitor of a wide variety of seemingly unrelated enzyme systems. These include poly ADP ribose polymerase, a Zn-finger enzyme, and other Zn-finger proteins. Cu-requiring systems are also affected by fusaric acid. These enzymatic systems are important in growth control mechanisms. It has become increasingly clear that fusaric acid, by virtue of its *butyl group penetrates the cell interior much more easily than picolinic acid, and works at least in part as a Zn*/*Cu chelating agent.*

As mentioned above, the *Geminivirus* infecting cassava is a family of viruses that are dependent upon viral proteins having a zinc finger domain for replication of the virus. The early signals leading to successful infection by *Geminivirus* and resulting disease are poorly understood. From the point of view of eliminating the *Geminiviruses* from the plants, it would be convenient to have an antiviral agent that rapidly penetrates the biochemical barriers present in the plant leaves (which are devoted to photosynthesis and *transpiration* [H₂O exchange with the environment]. After penetrating the circulatory system of the plant, and the cells containing the infecting virus, the antiviral agent could exert a role in the initial destruction of the virus. Furthermore, the same agent could elicit various plant defense responses against the virus. The inventors believes that at non-toxic concentrations (e.g. exposure of the plant cells at 50 to 300 uM), Fusaric acid (5-butyl picolinic acid) could have the required biochemical properties to perform all of the following: 1) The presence of the lipid soluble butyl group of Fusaric acid (FU) indicates that this antiviral agent can rapidly penetrate the leaves and, when in contact with the virus irreversibly disrupt ZFPs of *Geminivirus;* 2) FU could elicit various plant defense response at 100 uM without toxic effects to the plant cells; and 3) FU could also decrease the overproduction of DnaJ protein, MPS-1/S27 ribosomal protein, and other ribosomal zinc finger proteins; 4) All these proteins induced and overproduced by viral infection will decrease by the presence of FU; thus, the capacity of the virus to control the key steps of transcriptional and translational cellular functions will be shut off; and finally 5) the virus will be unable to continue with its hypercycles of disassembly, replication, assembly, viral movement mechanisms to invade the circulatory system of the plant and invade all the plant cells. Thus, one of the antiviral agents of the instant invention, FU can be very useful to eliminate *Geminivirus* infections of Cassava plants.

There are a number of papers that indicate that early physiological responses of several plants to fusaric acid and picolinic acid result in biocontrol of parasitic invasion of the plant by the induction of numerous genes that prevent parasitic infection as a result of exposure of the plants to FU or Picolinic acid. For example, Bouizgarne *et al* (101) demonstrated that fusaric acid can control the parasitic angiosperm *Orobanche* ramose. The authors showed that FU can elicit various plant defense responses at 100 nM without toxic effects. Large FU concentrations (>500 uM) reduced root growth and induced a rapid transient membrane hyperpolarization, followed by a large depolarization, compatible with the induction of a signal transduction pathway. As a result of this rapid response of the plant *Arabidopsis thaliana* which was pre-treated with FU, the germination of the angiosperm *parasite Orobache ramosa* was inhibited. These data indicates that FU at nontoxic concentrations can activate complex signal transduction components necessary for plant-defense responses that contribute to the control of invading parasites. Similarly, Cassava plants pretreated with FU could activate defensive cellular signal transduction pathways which can participate in conjunction with FU, in the inhibition of the infection and proliferation of *Geminivirus.*

Novel substituted derivatives of picolinic acid and related compounds can be used systemically to treat plant viral infections. The novel substituted derivatives of picolinic acid, Fusaric acid and related compounds also work by disrupting the binding of zinc atoms in zinc finger proteins, zinc ring proteins or other structures heretofore unknown that depend upon the inclusion of zinc or other metal ions such as transition metal ions, for stability, packaging or replication. The novel substituted derivatives are stable and retain their zinc chelating properties even when introduced systemically by superficial spray of a plant, injection, electrostatic, magnetic fields, root administration, trans-leave, or other routes of administration.

**Table 11** illustrates the chemical structure of novel derivatives of picolinic acid for external or systemic use in plants infected with viruses. Computer modeling indicates that such derivatives can interact with zinc atoms and disrupt its binding to the zinc finger protein. Substitutions at positions 3, 4, 5 and 6 on the 2-pyridine carboxylic acid (picolinic acids) have the proper configuration to prevent interference with zinc finger protein backbone. For example R1, R2, R3 or R4 can be a methyl, ethyl, propyl, isopropyl, butyl, isobutyl, secondary butyl, tertiary butyl, pentyl, isopentyl, neopentyl or similar group. Further, substitution with halogens such as fluorine, chlorine, bromine and iodine can result in effective, systemically active agents. The systemic compounds can be prepared by methods generally known to the art and include pharmacologically acceptable salts thereof.

Figure 16, and 16.1-16.4 illustrate the binding of picolinic acid or fusaric acid to zinc which is bound to the zinc binding domain of a zinc finger peptide. Furthermore, **Table 11** shows that analogs of picolinic acid can be substituted at pyridine ring positions 3, 5 or 6 without affecting the binding of the analogs to zinc. Therefore, the moieties listed in **Table 11**, that can be substituted at various positions in the pyridine ring, result in picolinic acid derivatives that not only are more stable for spraying of plants with the purpose of systemic administration of the plant systems (leaves, roots, etc), but also one that has even greater affinity and specificity for, and binding potential with, various zinc finger proteins of viral or plant cell origins at pM concentrations.

It will be appreciated that substitutions at the 3, 4, 5 and 6 positions can be made with a peptide of sixteen amino acids or more with either basic or acid amino acid residues predominating. The substituted picolinic acid would have an increased molecular weight and a substantially *increased half-life in the vascular system of the plant* being treated for diseases such as CMD. Such compounds penetrate virus-containing plant cells more effectively due to the amphipatic nature of the peptide residues.

The systemic compounds can be administered to plants by any means that produces contact of the active agent with the target viral protein, such as spraying, transdermally, by root absorption, or any other method for obtaining a pharmacologically acceptable intracellular level. In general, a pharmacologically effective daily does can be from about 0.01 mg/kg to about 25 mg/kg per day or any other pharmacologically acceptable dosing. It will be appreciated that picolinic acid derivatives referred to herein as the **"systemic compounds"** can be employed in the hereinafter described topical preparations as well as employed systemically. Furthermore, the claimed invention is intended to include any other chemical compounds, either derivatives of picolinic acid, compounds with structural relationships to picolinic acid, or heretofore unknown compounds that function to chelate, attach to, or modify metal ions in proteins structures, including, but not limited to transition metal ions found in proteins structures of viruses, proliferative cells (plant or animal) or even as components of fungi and bacteria.

It has previously been discovered that Np7 nucleoprotein is required for correct assembly of newly formed virus particles during the viral life cycle, as explained above. By modeling, the inventors has discovered the activity of picolinic acid in disrupting zinc finger Np7 proteins in retroviruses (Fernandez-Pol, JA; US Pat. '393, Oct. 3, 2000).

**FIGURE. 7** illustrates the role of ZFPs in *Geminivirus* infection; FIGURE 16 and 16.1-4 illustrate the NMR spectra of the binding of fusaric acid and picolinic acid to the zinc atom of a zinc finger peptide; **FIGURE 14** **A,** illustrates the disruption of zinc finger binding domains in a viral nucleoprotein with two zinc finger caused by picolinic acid; and **Table 11** illustrates the wide spectrum of antiviral activity of picolinic acid and derivatives thereof.

As mentioned above, the *Geminivirus infecting cassava* is a family of viruses that are dependent upon viral proteins having a zinc finger domain for replication of the virus.

It will be appreciated that various changes and modifications may be made in the preparations and methods described and illustrated without departing from the scope of appended claims. For example, suitable preparations, other than topical preparations of metal chelating compounds may be employed for the treatment of plants systemically infected by *Geminivirus.* The preparations may be used alone or in combination with other antiviral chelating agents related or unrelated to picolinic acid that interact with zinc finger proteins of the virus. The combination of chelating agents antiviral agents having different targets on the zinc finger proteins may be more effective (Fernandez-Pol *et al* US Pat. 2003). Furthermore, the preparations may be used to treat a wide spectrum of proliferative and viral diseases mediated by zinc finger proteins or metal ion dependent proteins or enzymes. Therefore, the foregoing specification and accompanying drawings are intended to be illustrative only and should not be viewed in a limiting sense.

### DESCRIPTION OF TRS SYNGENIC VIRUS-RESISTANT PLANTS BY GENERATION OF INTRACELLULAR PICOLINIC ACID OR DERIVATIVES THEREOF IN THE PRESENCE OF A PATHOGENIC VIRUS.

In the present invention, the generation of virus-resistant plants, is obtained by the tightly control expression of picolinic acid carboxylase (PAC), an enzyme from animal cells transferred and integrated in the genome of the plant cells to be protected from viral damage. The PAC in the presence of an appropriate substrate generates Picolinic acid or derivatives thereof (depending on the substrate substitutions). The antiviral acting systems are introduced into the plants and plant cells by means of DNA vectors, and when the product (PA) is generated under controlled conditions, Picolinic acid disrupts viral ZFP and plant cell ZFP such as DnaJ (a zinc finger protein), ribosomal proteins with zinc finger domains, and other metalloproteins that are involved in the assembly, movement and proliferation of the plant viruses.

This invention represents a completely different strategy to eliminate plant cell viruses such as *Geminiviruses* and numerous other viruses using ZFPs. According to the present invention a non-toxic agent, Picolinic acid (or derivatives thereof), when generated by safe and effective **Cassette TRS construct** methods of this invention, by the introduction of a suitable **strong gene promoter,** such as the enhancer promoter proteins of the invading infectious virus in tandem repeated units (10 to 100 or more), the rapid generation of Picolinic acid by viral promoter protein activation of PAC, can disrupt the ZFPs of the virus and accessory ZFPs produced by the plant cells, resulting in the disintegration of the plant virus and PCD of the infected plant cell. After the disintegration of the invading virus and the attenuation of the overproduction of proteins (DnaJ, MPS-1, Ribosomal ZFP, etc) under the previous control of the enhancers or promoters of the infectious viruses, the absence of protein enhancers or strong promoters of PAC, the production of Picolinic acid is immediately arrested and the levels of PA in plant and plant cells decrease to zero levels. Subsequently, either the few infected cells enter into PCD or if viable utilize their energy chemicals to degrade the viral and cellular picolinic acid-denatured proteins **(****FIG. 15A and 15B****).**

Picolinic acid and derivatives thereof are wide-spectrum antiviral agents with non-toxic properties. These antiviral agents interact and disrupt viral ZFP components which are of critical importance to the replication, assembly and dissemination of the virus by the circulatory system of the plant.

The present invention provides the basic concepts and techniques to produce TRS syngenic plants with increased resistance to any pathogenic plant virus. Since picolinic acid is non-toxic for normal plant cells, the plant cells infected with plant viruses may be prevented from entering into PCD if the treatment is applied at early stages of the viral infection. The inhibition by PA of the plant cell ZFPs can result in a transitory and reversible conformational impairment of the host cell ZFPs participating in viral interactions, with a potential functional recovery of the cellular ZFPs, after elimination of the virus by PA, and a decrease in intracellular the picolinic acid levels. Thus, in cases of early treatment of viral infection, the functional integrity of cellular plant systems involving ZFPs and interacting with viral proteins can be preserved..

The disruption by Picolinic acid of certain ZFPs produced by plant cells which are of critical importance for the replication, assembly and dissemination of the pathogenic virus provides an additional therapeutic opportunity for this wide-spectrum antiviral agent. For example, the plant DnaJ proteins which are involved in numerous essential protein processing functions can be a valuable target for picolinic acid and derivatives thereof. As the inventors previously demonstrated, DnaJ ZFP is an important target for PA in animal cells, reducing inflammation when its functions are arrested by PA (Fernandez-Pol, JA; US Pat. '393, Oct 2, 2000).

Plant viruses are dependent for viral protein processing of many of the functions of DnaJ proteins such as active folding of viral proteins, inhibition of viral protein aggregation, and protein distribution into specific cellular compartments (Harti et al., 2002, Science, 295, 1852-8 and 1996, Nature, 381, 571-9). (Kelley, 1999, Current Biology, 9: 305-308 and Kelley, 1998, TIBS, 23: 222-227).

The presence of the J domain, allows these proteins to bind to the DnaK component critical for the function of the DnaJ proteins. Thus, inhibition of the functions of the ZFP DnaJ should disrupt and prevent the assembly, movement and proliferation of virus such as *Geminiviruses and other viruses using plant ZFP* and requiring DnaJ for their pathogenic effects.

Researchers have found that viruses are able to activate **heat shock proteins** to be used for specific viral functions (**FIG. 7**). For example DnaK (Hsp 70) is induced by animal viruses such as adenovirus, herpes virus, cytomegalovirus, and other viruses. DnaJ proteins are ZFPs, cysteine rich, defined by the J domain, which is essential for stimulation of the **Hsp 70 DnaK ATPase activity.** The DnaK interacts with zinc finger ribosomal proteins to either refold some of the unfolded ribosomal proteins or by solubilizing the denatured ribosomal proteins to increase the turnover rate.

As in the case of animal cells, viruses which attack plants control and use the DnaJ - mediated coupling of ATPase with substrate binding by activating the ATPase domain of DnaK, which activates the complex **Chaperone Cycle (Table 5).** Some plant viral proteins bind directly to DnaK (Hsp70) proteins. Other viruses produce proteins that contain sequences with J domains such as SV-40 oncogenic viruses. Numerous other pathogenic viruses contain sequences resembling the J domain. Thus, these viral proteins use and control the cellular Chaperone Cycle to perform the following functions essential for viral survival: disassemble or assemble viral protein polymers or other complexes, denature and render useless cellular protein components that prevent the movement of viruses within and in-between the plant cells; and prevention of precipitation of viral proteins with exposed hydrophobic regions.

There are numerous examples of the use by plant viruses of the Chaperone Machine. The movement protein NSm of the *tomato spotted wilt virus* binds to DnaJ protein from *Arabidopsis* thaliana and other experimental plants (Von Bargen et al., 2001, Plant Physiol. Biochem., 39: 1083-1093 and Soellick et al., 2000, PNAS, 97: 2373-2378). A plant virus of the family *closteroviruses* has a gene that codes for DnaK protein. In this family of viruses, DnaK is essential for the assembly and dispersion of the viruses among the plant cells (Alzhanova et al., 2001, EMBO J., 20: 6997-7007). Moreover, the capsid protein (CP) of several viruses such as *Geminiviruses, potyviruses,* and other plant viruses also interacts with a DnaJ proteins.

The data described above clearly shows that pathogenic plant viruses subvert and use the plant cellular Chaperone Machine for numerous viral survival functions. Because DnaJ proteins are zinc finger proteins, these proteins can be neutralized and render non-functional by using picolinic acid and derivatives thereof. DnaJ is essential for protein-related cellular functions in both non-infected normal plant and animal cells. In normal animal cells, the use of picolinic acid did not lead to the suppression of DnaJ-like proteins or other proteins of the Chaperone system, confirming that picolinic acid is non-toxic to normal cells. In contrast, in virally infected cells, picolinic acid and derivatives thereof have pronounced toxic effects which may include suppression of DnaJ proteins. Virally infected cells were killed by picolinic acid and derivatives thereof by apoptosis. In plant cells, this process is denoted as PCD.

The responses of both the Chaperone system and ribosomal protein response to viral infection are part of the organism (animal or plant) defense against viruses. The compounds and methods of the present invention can be used to block both the action and overproduction of the DnaJ zinc finger proteins when excessively expressed by virus infection. This partial blocking of excessive DnaJ ZFP expression in virally infected cells required for enzyme activity will reduce the stress reaction in virally infected cells by reducing the availability of the components of the Chaperone system for viral functions, effectively neutralizing the virus infecting plant or animal cells. Thus, the present invention, can show that the suppression of the function of DnaJ proteins by neutralizing the DnaJ zinc finger domain by Picolinic acid or derivatives thereof *can produce plants which have increased virus resistance with no effects on the normal plant cells.* The syngenic plants overproducing picolinic acid and having lower levels of DnaJ can have essentially the same phenotype which will be indistinguishable from the wild type homologous plants.

The present invention can show that inhibition of the expression by picolinic acid of DnaJ proteins, which are no longer able to interact with viral proteins because of the binding of picolinic acid to the zinc atom in the zinc finger domain of DnaJ proteins, will result in a DnaJ protein with *conformation changes which prevents its binding to chaperone partners.* The inhibition of the zinc finger domain by picolinic acid can result in the production of plants which are modified in a way that results in increased virus resistance due to unavailability of spare DnaJ molecules. The method according to the invention, by means of which plants with increased virus resistance are produced by inhibiting the function or binding of DnaJ to other partners by *endogenous or exogenous picolinic acid,* offers a considerable advantage of neutralizing an additional zinc finger protein required for virus survival and propagation in plants cells.

In general, the person skilled in the art can use appropriate algorithms to determine the proportion of plant intracellular inhibition of different zinc finger proteins such as DnaJ, ribosomal proteins, zinc finger viral proteins, induced by increasing the doses of endogenously plant produced or exogenously added picolinic acid or derivatives thereof on zinc finger protein activity produced by viruses or plants. In general, the concentrations of Picolinic acid and derivatives thereof that interact with ZFPs are in the pM range.

The TRS syngenic plants of the present invention are particularly resistant to the *Geminivirus that produces the Cassava mosaic disease.* The reason for this specificity is that the Cassette TRS construct is design with a tandem repeated DNA encoding the sequence for binding a strong *Geminivirus* protein promoter.

According to the instant invention, syngenic plants in which several specific viral proteins are silenced by picolinic acid produced by the syngenic plants are resistant to all virus classes, groups and strains possessing zinc finger proteins in their structures. Syngenic plants infected by viruses which contain no zinc finger proteins but require use of zinc finger proteins produced by the syngenic plants, will be resistant to these viruses, if the viral proteins interact with the plant zinc finger proteins such as DnaJ proteins, ribosomal zinc finger proteins (e.g. MPS-1/S27) during the infection cycle.

The present invention provides methods which either produce plants with permanent resistant to viruses or are modulated to express transient resistance. The syngenic plants that permanently produce picolinic acid can help, along with other methods, to produce plants which eventually can eradicate the virus simply by lack of susceptible plants. The syngenic plants producing picolinic acid as an antiviral agent are characterized by more stable virus resistance, *without having any negative effect upon the environment, animal and other plants, and in the phenotype of the novel Cassava syngenic plant resistant to all strains of Geminivirus and other potentially infectious viruses that utilize viral or plant cell zinc finger proteins for replication.*

The methods according to the invention can also be put into practice to produce any TRS syngenic plant and plant cells which have increased virus resistances.

The invention also encompasses the crop products and seeds of syngenic plants, and the plant cells from any tissue with increased virus resistance. More specifically, the materials resulting from this invention include in general: fruits, seeds, tubers, cuttings, leaves, etc. Moreover, they include portions of these plants, such as cells, protoplasts, tumors, mitochondria, Golgi apparatus, microtubules, DNA, RNA, etc., from which a new syngenic plant can be originated.

The invention also includes the nucleic acid molecules containing the viral resistant genes and controlling sequences (strong promoters, enhancers, etc.), described below.
By **Cassette TRS construct** technology, these nucleic acid molecules are stable integrated into a non-coding region of the plant genome. In addition, the same nucleic acid molecules designed as autonomously replicating molecules in the plant cell cytoplasm or nuclear fluids are also included. Autonomously replicating virus vectors are well known in the art.

There are no restrictions to the method of the invention to incorporate into a plant the tandem repeated gene or genes sequences that will produce picolinic acid or derivatives thereof. In this particular invention we will focus on agricultural plants, and in particular Cassava which is prone to acquire viral infections from *Geminiviruses.* A few examples of plants in which the method can be used are Triticum (wheat), Secale (rye), Oryza (rice), Sorghum (millet), and Zea (maize). Other plants that can be transformed into TRS syngenic plants producing PA are listed (partially) in **Table 8.**

To determine that plants are TRS syngenic, the nucleic acid containing the sequences of interest (Picolinic Acid Carboxylase) will be transferred to the plant by using vectors well know in the art, such as a plasmid, which can replicate inside the plant cells, the isolated plant cells or a plasmid or nucleic acid that can be integrated into the plant genome permanently such as the DNA sequences present in a **Cassette TRS construct**.

In one of the embodiments of this invention, the DNA vectors used include, in 5'-3' orientation, **a strong promoter,** an operatively linked DNA sequence which includes the sequence encoding the Picolinic Acid Carboxylase (PAC) enzyme which produces picolinic acid in the presence of an appropriate enzyme substrate [e.g: *2-amino-3-carboxy-muconate semialdehyde]* in the tissues or circulatory system of the plant, and a termination sequence. When the transcription of these DNA vectors occurs in the plant cell, RNA molecules coding for PAC are produced. By translation in the plant ribosomes, the PAC protein emerges and is ready to act on the corresponding substrate molecule which generates picolinic acid, or if the substrate is a derivative of PA, generates the corresponding derivative of PA. The enzyme substrate [2-a-3-c-mu/ s-hyde] can be introduced in the plant by various routes such as roots, leaves or in solid particles-self-dissolving water soluble forms (e.g., such as the common dishwashing particles). The zinc finger proteins targets produced by the plant cell or the virus infecting the plant can be completely disrupted by picolinic acid. After interacting with picolinic acid, DnaJ proteins, ribosomal proteins and viral zinc finger proteins can be degraded by proteases due to the change in configuration.

In another embodiment of this invention, the nucleic acid tandem repeated sequences encoding PAC are transferred to the plant cells and can be place under the control of a strong viral promoter which will function in plants immediately after viral infection and disassembly of the virus. For the purposes of this invention, these plant promoters can be constitutive or inducible promoters. Furthermore, they can also be virus-specific promoters such as *Geminivirus* specific promoters present in Cassava plant cells. From these **Cassette TRS constructs**, it can be inferred that TRS syngenic plants can be produced which, under normal circumstances, express no picolinic acid production, but if attacked by a virus, *the virus-specific DNA promoter in the cells first affected by the viral infection will be activated by the viral protein promoter and the PAC- integrated in the plant genome- will rapidly start the production of picolinic acid which will neutralize and disrupt the zinc finger proteins of the invading virus.*

It is well known in the art that there are numerous strong promoters that can be used in the construction of vectors. Typically, the constitutive strong promoter for the ribosomal protein 35S can be used as a promoter for vectors. Furthermore, other promoters selected from different sources, such as plants or plant viruses, can be useful for the expression of PAC in plants or plant cells. The selection of the strong promoter, and of regulatory sequences such as enhancers, will determine the dose-dependent and time-dependent expression pattern of PAC, and thus the degree of production of picolinic acid. In turn, the time- and dose-dependent increase of picolinic acid inside the plant cells will determine the disruption of the zinc finger proteins of the virus and the plant DnaJ and ribosomal proteins in syngenic plants. A summary of useable control sequences can be found, in Zuo et al., 2000, Curr. Opin. Biotech., 11:146. The inducible strong promoters are numerous and are well known in the art.

The term **"Cassette TRS construct"** as used herein includes a DNA sequence capable of controlling the expression of a designated nucleotide sequence in a compatible host plant cell. The Cassette TRS construct consist of a strong viral promoter operable linked to the nucleotide sequence of interest which is functionally linked to the termination sequences. The DNA sequences are arranged so that proper transcription and translation of the nucleotide sequence of interest will occur. The coding regions codes for the proteins of interest of this invention, but fusion components which will remain fused to the sequence of interest may be added. Furthermore, the gene of interest, such as the gene for PAC will contain no introns in their coding configuration.

For the purposes of this invention, "plant" denotes any plant at any developmental stage, such as embryos, seeds, meristematic areas, leaves, roots, gamethophytes, sporophytes, pollen, spores, microspores, and derivatives thereof. "Plant tissues" refers to plant cells, organs, seeds, callous tissue, cells in culture, and protoplasts. There is no limitation on the type of plant that can be used to modulate picolinic acid and derivatives thereof gene expression by PAC. Although emphasis is placed in the plant Cassava (*Manihot esculenta*), the invention is not limited to any type of plant in particular.

The term **"TRS syngenic plant"**, refers to a plant containing within its genome a heterologous polypeptide sequence introduced by using *Agrobacterium* transfer technology (**FIG. 18**). The heterologous polypeptide sequence, in the case of this invention, is PAC and the associated regulatory nucleotides. *The heterologous sequence is stable and capable of being passed from generation to generation as a "stable genomic integrated Cassette of genes* ".

The use of inducible strong promoters allows the production of plants and plant cells which only transiently express, and thus transiently neutralize and destroy the virus infection of the plant when the sequences coding for viral promoters are enhanced by binding of the virus protein promoters. The transient resistance to a virus may be useful if the risk of virus infection of the plants is cyclic and therefore the plants need to resist the virus for only a specific time period.

The vectors utilized in this invention can also include enhancer elements as regulatory elements. In addition they can contain, replication signals, DNA signals to propagate the vectors in bacteria such as E. coli. *Moreover, it is desirable that the DNA vector contains regulatory sequences that allow stable integration of the vector into the plant's host genome. These types of vector construction techniques are well known in the art.*

Moreover, termination sequences ensure that the transcription or the translation is properly terminated. In the case of PAC the transferred nucleic acids need to be translated. Thus, they should contain stop codons and regulatory sequences. Standard cloning methods can be obtained from, e.g., Sambrook et al., 2001 (Molecular cloning: A laboratory manual, 3.sup.rd edition, Cold Spring Harbor Laboratory Press).

To illustrate the reader on the specific objectives of this portion of this invention and non-obvious tandem repeated DNA Cassette TRS constructs technology used in this invention, a brief description of the steps for the creation of TRS syngenic plants follows. The DNA vectors used include in the specific orientation 5'-3', a promoter, an operatively linked DNA sequence which *includes the sequence encoding the Picolinic Acid Carboxylase (PAC)* which generates the product picolinic acid in the presence of an appropriate substrate, which is supplied to the plant externally by the roots or by other desirable means. The PAC gene also includes a termination signal. Thus, the non-toxic substrate circulates continuously in the vascular system of the plant and is always available to produce picolinic acid product. When the plant cells are infected by pathogenic viruses such as *Geminiviruses,* the viral transcriptional- promoters proteins, bind to the strong tandem repeated promoter of PAC, activating the tandem repeated PAC system to destroy the virus by production of picolinic acid. In summary, plants infected by *Geminivirus* are destroyed by their own viral promoter proteins which activate the PAC gene system leading to the production of picolinic acid.

When the transcription of these DNA vectors occurs in the plant cell, RNA molecules coding for PAC are produced. By translation of the mRNA in the plant ribosomes, the PAC protein molecules emerge and are ready to act on the corresponding substrate molecule to generate picolinic acid (PA) or if the substrate is a derivative of PA, the enzyme generates the corresponding derivative of PA. The ZFPs targets produced by the virus infecting the plant or induced by the virus to be overproduced by the plant, are rapidly disrupted and destroyed by picolinic acid. According to NMR studies carried out by the inventors, after interaction of picolinic acid or fusaric acid with DnaJ ZFPs, ribosomal ZFPs, and/or viral ZFPs, all these ZFPs proteins are incompatible with existence in a normal functional configuration. Thus, the infecting virus is permanently damaged and destroyed.

There are numerous techniques for the introduction of DNA into a plant host cell. Examples of these techniques are: the transformation of plant cells with *T-DNA by using Agrobacterium tumefaciens or Agrobacterium rhizogenes as a transformation agent* (FIG. 18), the electroporation of DNA, and the introduction of DNA by means cell fusion. These plants are distinguished by increased and permanent resistance to the viruses various classes, groups and strains specified above.

Examples of chemical structures of broad-spectrum antiviral agents, including picolinic acid, substituted picolinic acid derivatives, as well as the practical application of those agents will be presented in the following paragraphs. Furthermore, the present invention provides methods which either produce plants with permanent resistant to viruses or are modulated to express transient resistance upon viral infection by the production of picolinic acid and derivatives thereof as it will be presented now.

### DEFINITIONS

### FUNCTIONAL COMPONENTS OF CASSETTES EXPRESSION VECTORS

The plant viral resistant system of the present invention involves stable and silent expression of transfected DNA in plant chromosomes in the absence of an invading pathogenic virus, fungus, bacteria, protozoa, eukaryotic or prokaryotic agents.

The presence of controlling elements (CE) in the transfected DNA (cDNA or synthetic DNA) can only be expressed if the CE are correctly placed in a vector that contains a specific promoter and other elements such as enhancers, cis-acting elements, polyadenylation signals and other regulators.

The resistant death genes of this invention will work normally in all tissues and will be able to replicate, most remarkable in merystematic cells (stem cells of plants) able to supply all the viral replicating factors absent in the pathogenic invading virus. In the absence of the pathogenic virus, the cassettes expression units will be suppressed and thus silent.

Promoter and enhancers consist of short arrays of DNA sequences that interact and bind specifically with viral and cellular proteins involved in transcription (McKnight and Tjian 1986). The combination of different recognition promoter sequences and the amounts of the complementary (cognate) transcription factors determines the efficiency with a given death gene is transcribed.

In general promoters contain two types of recognition sequences : the TATA box and the upstream promoter elements. Enhancer elements can stimulate transcription up to 1000-fold from linked promoters (homologous or heterologous).

A number of enhancers are specifically activated by the presence of an inducer, such as a hormone (steroid or thyroid hormone) or transition metal ions (copper or iron).

Many enhancer elements produced by pathogenic viruses have a wide-spectrum host range and are active in many tissues. For example, the following promoters are active in many cell types: (1) the SV40 early gene enhancer; (2) the long terminal repeat (LTR) of the Rous sarcoma virus genome; and (3) the LTR from the cytomegalovirus.

RNA polymerase II transcribes through the site that initiates polyadenylation. DNAs encoding the foreign proteins of interest are cloned as cDNAs, thus lacking all of the controlling elements required for expression. Furthermore, plasmid DNA expression vectors can contain regulatory elements from Eukaryotic viruses such as SV40, BPV-1 encoding eight early gene products (E1-E6), or Epstein-Barr virus origin P (EBV oriP).

As defined previously, a promoter is a regulatory region of DNA located upstream of a gene, providing a control point for regulated gene transcription. The promoter contains specific DNA sequences that are recognized by proteins produced by the plant pathogenic agents as transcription factors. These factors bind to the promoter sequences, recruiting RNA polymerase that synthesizes the RNA from the coding region of the gene.

Several transcription factors produced by *Geminiviruses* are targets for the cassette constructs of the instant invention. The *Geminiviruses* transcription proteins specifically interact with the inducible viral promoters present in the cassette constructs and are denoted: AC1(Rep) [replication associated proteins], AC2 (TrAP) is an strong promoter [transcriptional activator protein, also involved in suppression of silencing], AC3 (REn) [Replication enhancer], AC4 [synergism and suppression of PTGS], AV1 (CP) [encoding coat protein (CP)], AV2 [bidirectional promoter], BC1 (MP) [Movement protein], BV1 [encoding nuclear shuttle protein (NSP)], BV2 [silencing response]. Key activators of viral transcription, AC 1, AC2 and AC3 *Geminivirus* viral proteins are early proteins and their transcripts are the most abundant species in early infection. They are involved in transcriptional activation and in silencing of plant genes.
AC2 protein is the key for activation and suppression of silencing and thus a mayor target for this invention. The promoters for AC1, AC2 and AC3 viral transcription factors are targets for activation of death genes numbers 1,2 and 3 of the instant invention.

### EXAMPLE 1

### REPRESENTATIVE EXAMPLES OF ANIMAL AND PLANT VIRUSES CONTAINING ZINC FINGER PROTEINS.

Because the zinc finger domain is essential for nucleic acid (DNA and RNA) binding, viral resistant mutants will not occur. Picolinic acid can be used, therefore, for prevention of *Geminivirus and other plant viral diseases* by, for example, inhibiting replication of the virus in early stages or by chemically inducing a non-infectious virus (devoided of zinc finger proteins). Furthermore, any chemical entity, either known or unknown at this time, that functions in the same manner as picolinic acid or its derivatives, is intended to be encompassed by the instant invention. Representative viruses which include zinc finger, zinc ring proteins or other types of motifs are included in **Table 1, 2, 3-9.** These tables illustrate animal and plant viruses containing zinc finger proteins that can be utilized as targets for the antiviral agents of this invention.

**Tables 1 and 8** provide Examples of families of viruses using ZFPs, Zinc Ring Proteins or Transition Metal Ion-Dependent Enzymes.

### EXAMPLE 2

### NMR Studies of Ligand Binding by a Zinc Finger Peptide From HIV-1: Inhibition of HIV-1 infectivity by Zinc-binding 2-pyridine carboxylic acids

The experiments with NMR presented here with retroviral zinc finger peptide from HIV-1 are relevant to the inhibition/disruption of zinc finger proteins from *Geminiviruses* and other plant viruses, as shown below.

Retroviruses of animals contain special zinc finger proteins denoted Nucleocapsid proteins. Nucleocapsid proteins of all known retroviruses have one or two copies of a highly conserved amino acid sequence consenting of ten variable residues and *four invariant residues.* The invariant residues function to coordinate zinc, forming "zinc fingers" that provide gag precursors and mature nucleocapsid proteins with highly organized structures that have high affinity and facilitate specific nucleic acid binding during almost every stage of the HIV replication hypercycle.

Viruses of plants are also highly dependent on the zinc finger motifs of nucleocapsid and other proteins for most of its biological functions. In general, *Geminiviruses* and other plant viruses have one or two copies of a highly conserved amino acid sequence consisting of several variable residues and four invariant residues having functions similar to those of zinc finger proteins the retroviruses mentioned above.

The great majority of both animal and plant viruses are under great selection pressure to maintain the zinc ligand residues. The conservation of nucleocapsid proteins, capsid proteins, specific transcription factor proteins, which are all zinc finger proteins, among animal and plant viruses and their essential role in infection, replication, assembly, packing of RNA, and other critical functions makes these viral zinc finger proteins attractive targets for therapeutic intervention.

The interaction between two of the ligands of this invention (Fusaric acid and picolinic acid) and their binding by a Zinc Finger Peptide from HIV-1 containing a zinc finger motif were studied by NMR spectroscopy (FIGS. 16, and 16.1-4).

Retroviral zinc fingers with the sequence Cys-X₂-Cys-X₄-His-X₄-cys (CCHC) bind zinc stoichiometrically and with high affinity [dissociation constant K_{d} = 10⁻¹² M (Rice et al, Nature, 361:473-475, 1993). The peptide sequence used in the instant experiments was synthesized as reported (Fernandez-Pol, 1994).

The peptide sequence **VKCFNCGKEGHIARNCRA**, corresponds to the N-terminal CCHC region of the HIV-LA1 *gag* polyprotein (amino acids 390-470). The peptide containing the complex of zinc atom bound to the zinc finger domain of the peptide was named: Zn (HIV1-F1). A synthetic oligonucleotide with sequence corresponding to a region of HIV-1 psi-packaging signal, d (ACGCC) was complexed with the peptide (South and Summers, 1993).Fusaric Acid [4-butyl-picolinic acid] was denoted FSR-488 and picolinic acid [2-pyridine carboxylic acid] was denoted PCL-016. Interactions of FSR-488 and PCL-016 with the zinc finger peptide containing Zn [Zn (HIV1-F1)] were studied by NMR spectroscopy. The results of these studies indicate that both small molecules (Fusaric acid and picolinic acid) bind Zn (HIV1-F1) at a hydrophobic cleft where the peptide binds an unpaired Guanosine base, its natural substrate (described by South and Summers, 1993). Titration experiments with each of the two molecules caused shifts in the ¹H resonances of Zn(HIV1-F1) that are similar to those observed when the peptide binds analogs of its natural substrate. Two dimensional NOE correlation spectroscopy (NOESY) of the solution containing Zn (HIV1-F1) and FSR-488 gave cross peaks at frequencies that are consistent with contacts between FSR-488 and hydrogen nuclei in the hydrophobic cleft of the peptide.

### Titration of a Zn (HIV1-F1) with FSR-488 (Fusaric acid) [FIGURE 16].

A sample containing peptide (1.63 mM) and Zn²⁺ (2 mM) was titrated with a solution of FSR-488. The experiment was monitored by one dimensional ¹H NMR spectroscopy. Under conditions in which the free zinc in the buffer was minimized, titrations with FSR-488 were conducted. Addition of FSR-488 yielded spectral shifts in the spectrum of the peptide (FIGURES 16, and 16.1-4). These results indicate that FSR-488 competitively binds both Zn²⁺ and the Zn²⁺/Peptide complex. This experimental finding and conclusions led the inventors to run a second 2 D NOESY experiment, this time at the higher concentration of FSR-488.

### The two dimensional NOESY spectrum of the peptide/FSR-488 (Fusaric acid; FIGURE 16, and 16.1-4).

The NOESY spectrum at the high concentration of [FSR-488]. was considerable different than that measured at low concentrations of [FSR-488]. At high concentrations [FSR-488], the sign of the NOE contacts involving either the drug or the peptide were positive, indicating that NOEs involving the ligand arose from the ligand / peptide complex. The NOESY spectrum shows cross peaks between an aromatic FSR-488 at approximately 8 ppm and resonances at 4.06 (peptide C-alpha-H), 2.60, 141, and 0.70 ppm (FIGURE 2). The presence of the cross peaks and tentative assignments for these peaks are presented in FIGURES 16 and 16.1-4). Interpretations for these assignments are presented in detailed below.

The shapes of the crosspeaks involving the FSR-488 resonance at 8.03 ppm indicate that these interactions involve a doublet resonance (either HC5 or HC6) and not with the singlet (HC3) FIGURES 16 and 16.1-3. Literature reference values indicate that pyridine hydrogen in the 3 and 6 positions have higher resonance frequencies than those in the 3 and 5 positions (example: 3-bromo pyridine, H6, 8.52 ppm; H5; 7.16). The 8 ppm resonance was therefore assigned to be that of the HC6 hydrogen. The assignment was supported by considering the first NOESY spectrum, where conditions did not favor Zn (HIV1-F1) binding. Under this condition, crosspeaks (with negative sign) are observed between the HC3 singlet and the resonance of the HC7 alkyl group (at 2.86 ppm), and between the doublet at 7.90 ppm and the HC7 alkyl group (at 2.86 ppm), and between the doublet at 7.90 ppm and the HC7. *This confirms that the 7.9 ppm resonance is attributable to HC5 and the 8.05ppm resonance contains the contribution from HC6.*

While the resonance frequencies of the alkyl (H2C7, H2C8, and H3C10) resonances correspond closely to the 2.6, 1.4, and 0.7 ppm cross peaks, neither the HC3 nor HC% shows a cross peaks at these frequencies. Since both HC3 and HC5 are closer to the alkyl group than HC6, the cross peaks most likely arise from contact between the HC6 of FSR-488 and hydrogen nuclei of the peptide, and not those of the FSR-488 alkyl chain.

The peak at 1.4 corresponds to the second FSR-488 alkyl chain hydrogen frequency, but also covers the Ala 13 methyl resonance. The inventors note that this is one of the resonances that underwent a substantial shift in the 1D titration experiment.

A number of the peptide beta-hydrogens have chemical shifts in the region of the 2.6 ppm cross peak (C3;279, F4, 2.61, N5; 2.80, C6; 2.53, N15; 2.56). The Phe 4 hydrogen assignment is favored because of its spatial proximity to other hydrogens whose resonant frequencies are affected by FSR-488 (this residue is in the hydrophobic cleft).

### EXAMPLE 3

### Identification of analogs of Picolinic Acid that disrupt Zinc finger proteins in plant cells.

The purpose of these experiments is to identify Picolinic acid analogues for exogenously testing in plant or cell plants for anti-viral properties that may be more potent than picolinic acid or fusaric acid in disrupting critical zinc finger proteins of *Geminivirus* that infect Cassava plants.

The inventors conducted a computer atomic substructure search utilizing the structure shown in **Table 11**, as the basis for the analogue search. All variations of positions **1,2,3,4,5, and 6** were investigated. Addition of covalently bound peptides, lipids and fused-rings were not extensively investigated in this study. Nevertheless, since both some fused-rings and peptides were tested in cultured animal cells, a brief description of their properties, which may also be useful as anti-viral in plant cells, will be mentioned. The carboxylic group at position **2** is fixed, as this carboxylic acid group defines the family of picolinic acid together with the pyridine ring. The nitrogen at position **1** remained without any substitution, to preserve its chelating ability of the two delocalized electrons. Furthermore, holding the **2** position inviolate as carboxylic acid, and the Nitrogen at position **1** unsubstituted, the number of analogues available is 112. These compounds are listed in Table 10. Group A compounds are highly likely to show anti-viral activity in appropriate plant cells bioassays. It is pertinent to mention here that the addition of covalently bound peptides (e.g of 16 amino acids) or lipids (e.g. 5 to 15 C atoms) will lead to more potent compounds.

### EXAMPLE 4

### ANIMAL AND PLANT CELLS RESPOND TO DAMAGING GENOTOXIC AGENTS AND PATHOGENIC VIRUSES WITH INDUCTION OF VIRAL AND CELLULAR STRESS SIGNALS THAT MODULATE GENE EXPRESSION

The zinc finger ribosomal protein MPS-1/S27 functions as an extra ribosomal protein to repair DNA damage and in particular degradation of mutated mRNA produced by genotoxic agents and pathogenic virus infecting animal and plant cells (**FIGS. 8****,****9****,****10** **and** **11**). The important function of the enzymatic degradation of the deleterious mutated mRNA by MPS-1/S27 is to prevent the production of defective proteins [by mutated cellular mRNA ] which can transform the cells in cancerous or induce apoptosis. Methods to neutralize overproduction of MPS-1/S27 which is induced by plant viruses and is deleterious for plants and plant cells, will be presented in the following paragraphs.

### Unexpected Functions of ribosomal protein MPS-1/S27 in preventing animal virus genotoxic stress: mRNA Degradation triggered by genotoxic damage to mRNA.

Fernandez-Pol *et al* have previously cloned, sequence and isolated one human ribosomal gene that because of its properties and various extra ribosomal functions was denoted Metallopanstimulin (MPS-1) (Fernandez-Pol, JA; US Pat. '041; Sep. 7, 1993; *"DNA vector with isolated cDNA gene encoding Metallopanstimulin";* **FIGS. 5** **and** **6**). MPS-1 is a ubiquitous 9.4 kDa multifunctional ribosomal S27/cytoplasmic/nuclear "zinc finger protein" which is expressed at high levels in a wide variety of cultured proliferating cells and malignant tumor tissues. The human MPS-1 gene and its relationship to human cancer cell growth was discovered in 1989, using breast cancer cells stimulated with specific growth factors such as EGF and TGF beta. MPS-1 tumor antigen is a ubiquitous tumor marker which is increased in malignant cells and in the serum. It has been shown to be useful in the detection and prognosis of various types of malignant conditions. Other experiments indicate that MPS-1 is involved in protein synthesis, repair of DNA, anti-apoptosis and rapid cell proliferation. Notably, in human melanomas, the MPS-1 protein is increased at least 10-fold more in melanomas than in melanocytes. **FIGURE 11** shows the genotoxic response of MPS-1/S27 in human Xeroderma pigmentosum, a genetic disease in which DNA repair is impaired.

MPS-1 was isolated using differential hybridization to screen a cDNA library derived from a human mammary carcinoma cell line (MB-468) that was stimulated with TGFB1 in the presence of cycloheximide (Fernandez-Pol, 1993). MPS-1 has 84 amino acids and an unmodified molecular mass of 9,460 Da. The MPS-1 protein contains a zinc finger domain of the C4 type (CCCC). Immunofluorecent studies demonstrated that in human melanomas MPS-1 is located in the nucleus, nucleolus, and cytoplasm. Fernandez-Pol *et al* (1994) showed that recombinant protein MPS-1 expressed in Sf9 insect cell lined was phosphorylated, can bind to DNA specifically, and participates in degradation of mutated mRNA induced by genotoxic stress. *UV light is a ribotoxic stressor and a damaging genotoxic agent, supporting the notion that neutralizing the response to UV light may be generated in the ribosomes which rapidly participate in protein synthesis.*

### Unexpected function of ribosomal protein MPS-1/S27 in genotoxic stress responses in Xeroderma Pigmentosum fibroblasts resembles responses in plant cells (Arabidopsis thaliana).

The inventors has examined the effect of UV light C radiation (254 nm) on MPS-1/S27 gene expression in culture XP (Xeroderma Pigmentosum Fibroblasts) skin cells (**FIG. 11**)for the following reasons: 1) cells from patients with this condition are hypersensitive to UV radiation, because of a defect in DNA repair system; 2) UV radiation has been shown to induce complex responses in human skin and any other tissue studied, including plants and plant cells; 3) the complex reactions include expression of repair genes and oncogenes; and 4) the homology of MPS-1 to many proteins involved in DNA repair and genotoxic responses that respond to UV light indicate that MPS-1/27 may be essential to protect animal cells such as XP skin fibroblasts from UV light and plant cells such as *Arabidopsis thaliana* from a series of genotoxic responses including UV light tissue destruction and mutagenesis, formation of tumors, oncogenesis, and susceptibility to viral infection of plant cells (**FIGS. 8****,****9****,** **10** **and** **11****; Table 12**). (Fernandez-Pol, et al; Cell Growth and Differentiation; 5:811-825, 1994; Revenkova, et al; 9th International Conf. on Arabidopsis thaliana, U of W, Madison, 1998; S27, MPS-1; mapped the S27-MPS-1 to gene chromosome 1; Fernandez-Pol et al 1998). **FIGURE 8** illustrates the role of MPS-1/S27 in genotoxic responses to chemical agents, radiation and viruses such as *Geminivirus.* **FIGURE 11** shows the effects of UVC radiation on the expression of MPS-1/S27 protein in human Xeroderma Pigmentosum (XP) cells. **FIGURES 9 and 10** show a series of experiments that demonstrate that ribosomal MPS-1/S27 protein is essential for resistance to carcinogenic agents in plants such as *Arabidopsis thaliana.* Therefore, MPS-1/S27 is required for the elimination of damaged transcripts after UV irradiation and mutagenic agents in both animal and plant cells.

### Unexpected function of ribosomal protein S27 (SRS27A of Arabidopsis thaliana in genotoxic stress responses in plants: Arabidopsis thaliana ribosomal protein S27/MPS-1 is involved in mRNA degradation triggered by genotoxic stress (FIGS. 9 and 10; Table 11).

To genetically dissect DNA damage responses in plants, Revenkova et al (Friedrich Miescher Institute, Switzerland) screened a collection of a *syngenic Arabidopis thaliana* plant lines with random T-DNA insertions for the mutant sensitive to genotoxic treatments. Revenkova et al isolated a mutant sensitive to methylmethanesulfonate (MMS) and UV-C, and the mutation co segregated with the T-DNA insert. The T-DNA insertion disrupted one of three genes for ribosomal protein **MPS-1/S27** (identified by Revenkova as ARS27A for *A. thaliana; [previously isolated from animal cells Fernandez-Pol identified an homolog denoted S27*/*MPS-).* Surprisingly, seedlings of the ARS27A developed normally under standard growth conditions. The growth was strongly inhibited in the presence of MMS, in comparison with the wild type. This inhibition was accompanied by the formation of tumor-like structures on the main root instead of auxiliary roots. Importantly, wild type seedlings treated with increasing doses of MMS up to the lethal dose have never displayed such developmental abnormalities, nor was this phenotype observed in ars27A plants in the absence of MMS nor under other stress conditions. *Thus, it can be concluded that the hypersensitivity and tumorous growth is ars27A specific response to the genotoxic MMS treatment, UV irradiation and chemical mutagens introduced lesions not only into DNA but also into **RNA,*** however little is known about the fate of the damaged transcripts. Revenkova et al observed rapid degradation of transcripts after UV light treatment in the wild type plants and this process was clearly affected in the ars27A mutant. The genomic fragment containing the ARS27A gene was introduced to the ars27A mutant by *Agrobacterium* mediated transformation. Treated with MMS , the complemented lines were indistinguishable from the wild type and they also regained wild type ability of rapid RNA degradation after exposure to UV irradiation. Therefore, they proposed that this isoform (ARS27A) of ribosomal protein S27 (MPS-1) is indispensable for the function of ribosomes, but is also required for the elimination of damaged transcripts after UV irradiation. [summarized from the 9th International Conference on Arabidopsis Research, Madison WI, 1998]

Therefore, ribosomes appear to incorporate several proteins with possible functions beyond protein synthesis, including responses to DNA damage (For reviews, see Fernandez-Pol, 2002; Fernandez-Pol et al 2005). An important characteristic of a number of ribosomal proteins is that they possess zinc finger motifs similar to those found in DNA-binding proteins or numerous animal and plant viruses. The ZFP are highly conserved in distant organism such as yeast, animals and plants (Wool, 1993). Loss-of-function mutations in the ZF motif are usually lethal.

*Arabidopsis* mutants were identified by Revenkova et al in a search for individuals hypersensitive to DNA-damaging treatments. The T-DNA insert was found to generate a null allele of one of three active genes coding for ribosomal protein S27. Fernandez-Pol et al, 1993, described the S27 as a ribosomal protein which displays DNA-binding activity due to the C2-C2 ZF binding domain similar to those involved in response to DNA injury. Fernandez-Pol et al cloned, isolated and sequence the same gene from human cells induced to proliferate by growth factors (1993). The characteristic features of S27 ribosomal protein isolated after stimulation by growth factors led Fernandez-Pol to name this gene Metallopanstimulin-1 (MPS-1). Highly elevated levels were shown to be one of the characteristics of MPS-1 in human melanomas.

Revenkova *et al* determined that one isoform of Arabidopsis mutant corresponding to ribosomal protein S27/MPS-1 is dispensable for translation. However, the same S27/MPS-1 acts as a regulator of transcript stability in response to genotoxic treatments. They showed that this isoform of S27/MPS-1 is involved in the degradation of damaged RNAs. Similarly, Fernandez-Pol et al showed that the S27/MPS-1 of human melanomas is also involved in the degradation of damaged RNAs.

Alignments of S27 ribosomal protein sequences of different species [GenBank/EMBO] showed high conservation of ARS27A and ribosomal protein S27 from rice, barley, rat S27, human MPS, yeast RPS27B and other selected homologs.

### EXAMPLE 5

### REQUIREMENTS FOR PENETRATION OF ANTIVIRAL AGENTS INTO PLANTS AND PLANT CELLS

The presence on the surface of plants of waxes, cutin and pectin protect the plants from viral infection. The underlying cells are protected by cellulose walls. All these substance form protective barriers to the invading viruses and vectors that must passed if infection will occur. Wounds are ports of entrance of choice. Wounds on leaves are also ports of entrance for the viruses. When the virus penetrates the cytoplasm of plant cells, in particular when the plant cells are in the process of healing and rapid cell [S] division, the viral genome is then uncoated and the sequential chemical steps results in the complex mechanisms of viral replication, possibly ending in apoptosis, and finally the transmission of the complete virus to other areas of the plant. Some of the mechanisms are similar to those of animal viral replication.

The antiviral agents of this invention are able to penetrate the plant and plant cells by various routes. The agents which are soluble in water can be absorbed by the roots and distribute through the circulatory system of the plant. Antiviral agents such as Fusaric acid (5-butyl picolinic acid) and derivatives thereof can penetrate more demanding barriers such as leaves. The 5-butyl group of fusaric acid increases the lipid solubility and thus can penetrate lipid bilayers of plant cells.

It is germane to mention here that both plant and animal virus utilize zinc finger proteins as structural DNA/RNA binding proteins or as replicating proteins. Moreover, mutation of the zinc finger domain renders the proteins unable to perform any function, because **mutations in the zinc finger domain are lethal**. Thus, these proteins are extremely important targets to control animal or plant viruses with the appropriated antiviral agents.

The importance of ZFP in the treatment of viral diseases in humans, animals and plants has been defined in detail by The USA Federal Register; August 10, 1995: 60, No. 145 which identified based on the research done at the National Cancer Institute that a new type of proteins, denoted zinc finger proteins, are critical targets for the prevention and control of viral diseases involving all types of viruses, including plant viruses. Since that time efforts have been made to specifically target ZFP. Fernandez-Pol (Cell, 1978) conceived a novel idea: to destroyed the zinc finger motifs of ZFP with antiviral chelating agents. The destruction of the zinc finger motifs by these agents can be lethal for the virus in question. As far the inventors can determine we are the only company that has achieved such preliminary goal of producing various preparations of non-toxic agents for numerous important human and animal viral diseases.

### EXAMPLE 6

### MULTIPLICATION OF BEGOMOVIRUS WITH SPECIAL REFERENCE TO GEMINIVIRUS THAT ATTACK CASSAVA,

**FIGURE 13** shows the genetic configuration of *Geminivirus* consisting of the Met binding site and five ORF. Of particular interest for this invention is the protein TrAP. TrAP is a phosphoprotein that binds and requires zinc for effective interaction with ssDNA. Molecular modeling shows that TrAP is a ZFP. The TrAP protein C-terminal domain acts as a transcriptional activator for other *Geminivirus* genes as well as for host plant cell genes, required in *Gemininivirus* replication. When the TrAP gene is fused to the DNA of the binding domain of yeast transcriptional activator GAL4, it strongly and specifically activates expression of a CAT reporter gene driven by an adenovirus E1B promoter. Thus, it is evident that TrAP of *Geminivirus,* like the human adenovirus E1A or human herpes simplex virus VP16, act as transcriptional coactivator interacting with various zinc finger cellular transcription factors bound to the sequence-specific promoter elements to cooperatively assembly and recruit the basal transcriptional machinery which is composed by critical non-structural (NSP) Zinc Finger Proteins.

When the ZFP are neutralized, destroyed or form ternary complexes with the pharmacological agents of this invention, the virus is disintegrated and the plant cells undergo apoptosis, eliminating the pathogenic virion. Similarly, in some of the syngenic plants cells selected for resistant to *Geminivirus,* if the viral infection is too advanced, the agents of the invention disintegrate the virus and also the infected cell (apoptosis). In animal cells infected by viruses (e.g. Herpes genitalis) a similar phenomenon of viral destruction and apoptosis occurs when the infected cells are treated with the agents of the instant invention (Fernandez-Pol, J.A.; Anticancer Res. 2002).

*Thus, encapsidation of ssDNA of geminivirus cannot occur and the viral cycle is interrupted in dividing cells only. Since encapsidation is required for propagation of the viruses by Aphids as well as nematodes, the virus is eliminated not only from the plant but also from its natural cycle in Aphids, nematodes and propagation by less common means such as cutting of the plant.*

The B ssDNA component of *Begomavirus* encodes two movement proteins that allow the virus to transit by various cell compartments such as the nucleus, nucleolus and cytoplasm. The *Geminivirus* encodes two transit-movement proteins which like in essentially all pathogenic animal DNA viruses, *Geminiviruses* replicates in the nucleus. Thus, the NSP (non-structural proteins) such as zinc finger proteins of the virus and the ZFP of infected cells) and the MP are strictly required under natural conditions to cooperate to move the ssDNA *Geminivirus* genome across the nuclear envelop, first through the cytoplasm, and previously across the cell wall of the plant cell.

### EXAMPLE 7

### GEMINIVIRIDAE: A CONSERVED ZINC FINGER MOTIF IN THE COAT PROTEIN OF TOMATO LEAF CURL BANGALORE VIRUS IS RESPONSIBLE FOR BINDING TO ssDNA: A SPECIALLY VULNERABLE AND ACCECCIBLE "TARGET" TO DISRUPT ZFPs WITH THE AGENTS OF THIS INVENTION

As efficient therapeutic agents, picolinic acid and derivatives thereof, the molecules must be able to attack zinc finger domains in ZFP and disrupt or neutralize zinc binding to ZFP of plant cells involved in viral replication. The N-terminal helix of the Coat Protein (CP) of the Tomato leaf curl Bangalore virus [ToLCBV] is involved in viral ssDNA encapsidation, virus movement and nuclear localization (Kirthi and Savithri, Arch Virol (2003) 148:2369-2380). Sequencing showed that CP is a zinc finger protein.

**FIGURE 14** shows the results of a computer search for DNA binding motifs in ToLCBV CP and numerous other *begomovirus* CP which found the following characteristics in all the CP proteins studied: 1) A stretch of 25 amino acid residues conserved in all *begomoviruses;* 2) a zinc finger motif showing 75% homology with other CP zinc finger proteins; 3) Begomovirus CP sequences in this region show that the motif corresponds to residues 65 to 85 in the TolCBV-[Ban 5] CP sequence; and 4) the CP sequence contains two cysteines and two histidines, typical of ZFPs. In summary, the results shown in **FIGURE 14** (Kirthi, N. and Savithri, H.S., Arch Virol (2003) 148:2369-2380) demonstrate that a zinc finger motif presents in the N-terminus region of begomovirus CP (coat protein) is required for the binding of ssDNA. Such interaction is essential in viral ssDNA encapsidation and in viral ssDNA movement and localization.

Although the main purpose of this invention is to treat Cassava diseases with the antiviral agents of the instant invention, the sequence characteristics of the plants listed in **FIGURE 14** and the analysis of the Gene Bank data, show the presence of a zinc finger motif in the CP proteins which binds ssDNA. Thus, the antiviral agents presented here can be highly efficient at pM concentrations in neutralizing and disrupting many *begomovirus functions* of the CP protein such as binding to ssDNA, encapsidation, movement and localization.

As an efficient therapeutic antiviral agent, picolinic acid and derivatives thereof, the antiviral molecules must be able to penetrate the plant cells and attack zinc finger domains in ZFP, disrupting or neutralizing zinc binding to ZFPs involved in viral replication, encapsidation, movement and localization in various cellular compartments. The Coat Protein (CP) of the *Begomovirus* ToLCBV-[Ban 5] rCP virus binds to ssDNA specially for encapsidation (and other functions) and can be therapeutically approached or entered into contact in the plant cells by the agents of this invention to prevent the binding of ssDNA to CP and disrupt the zinc finger domain of CP. Table 10 shows a number of selected derivatives of Picolinic acid and Fusaric acid that can be tested for the purposes delineated above,

### EXAMPLE 8

### MECHANISMS OF SURVIVAL OF PLANTS TO VIRAL INFECTIONS AND PENETRATION OF PHARMACOLOGICAL ANTIVIRAL AGENTS IN PLANT CELLS.

Wax, cutin and pectin are protective materials distributed in certain areas of the plants. The underlying living cells contain thick cellulosic walls. The antiviral agents of the present invention are able alone, or when properly mixed with water or lipid soluble agents in specific miscelle forms or inert detergents, or mixed with an innocuous fine-mesh abrasive substance to create the necessary peeling (as is done very commonly in dermatology in human beings without any significant consequence for the skin). Although random passage of viruses from cell to cell is prevented to some degree by the cell walls of plant cells contain channels. These channels are utilized by plant pathogenic viruses to invade the entire plant. The transport of the *Geminivirus* to all sectors of the plant is a extremely rapid process.

**Micelles:** The inventors experimented with penetration of active antiviral agents into the plant and plant cells. Micelles were quite effective in penetrating the plant cell barriers as they form amphipatic molecules of various predesigned forms. Micellar systems have the unique property of solubilizing both hydrophobic and hydrophilic compounds. The colloidal aggregates prepared contain a standard number of amphipatic molecules (50 to 100). This type of micellar preparation may be ideal to spray large fields of crops with high efficiency of penetration into the plants and plant cells. It is worth mentioning here that amphipatic peptides of 16 to 21 amino acids attached, for example, to positions 4, 5, and 6 of the pyridine ring of picolinic acid and derivatives thereof can also function as agents with high degree of penetrability in the treated plants, depending on the ratio of hydrophilic to hydrophobic amino acids in the peptide.

It is well established that the final distribution, dilution and replication of *Geminivirus* systematically infecting a plant such as Cassava is not uniform or systematic. In the leaves there is a display of "mosaic symptoms" and the concentration of the *Geminivirus* in the leaves is unrelated to the color produced by the infection of the Gemini virus.

The evidence of disease in a Cassava infected plant is the result of viral interference with vital activities of the plant such as photosynthesis, respiration, nutrient deprivation, hormonal regulation of growth and production of small areas of cellular transformation (**cancer of the plant cells**) which are induced by the *Geminivirus* which activates the nuclear oncogenes, initiating the process of malignant plant cell transformation. This usually, but not always occurs in the S [synthetic] phase of the cell cycle. In the S phase of the cell cycle is where picolinic acid, fusaric acid or derivatives thereof usually destroy cancer cells in plants and animals. Similarly, virally transformed cells which utilized ZFP for replication and are exposed to the agents of this invention, undergo apoptosis and viral destruction of transformed cells (Fernandez-Pol, J.A., Anticancer Res.; 2002). The inventors consider that there is no evidence that the process that occur in the animal cells (apoptosis of virally infected cells) does not and will not occur in *Geminivirus* or other type of plant pathogenic virus. The reason is simple: Apoptosis is produced when the configuration of Zinc Finger Proteins of viruses is altered and that event results in the exposure of sites susceptible to proteolysis in the ZFPs that lead to a cascade of events to eliminate an abnormal protein and the cell from an animal of plant cell.

### EXAMPLE 9

### LIMITED NATURAL PLANT RESISTANCE TO VIRUSES IS ENHANCED BY NOVEL AND EFFECTIVE EXOGENOUSLY ADDED ANTIVIRAL AGENTS

In contrast to the control of fungal plant diseases, no chemical or pharmacological agents are presently available for use as antiviral agents in plants. Control of viral vectors (such as whiteflies) is the most commonly used measure to prevent propagation of viral diseases. The plant's resistance response to viruses is rather limited. Important crop plants have no resistance to viruses that then cause serious diseases.

The novel and non-obvious therapeutic plant antiviral agents such as picolinic acid and derivatives thereof, are disclose *for plant and cell plant uses* for the first time in the instant application. The molecules are designed to penetrate the plant cells and attack zinc finger domains in ZFPs of plant viruses. The disruption of zinc binding to ZFPs of plant cells involved in viral replication, encapsidation, movement and localization renders the ZFP unable to perform functions critical to viral infection, reproduction and other survival functions.

It will be appreciated by those skilled in the art that the inventors have disclosed the best mode presently known for picolinic acid and its derivatives to disrupt plant viruses by neutralizing ZFPs. The scope of the appended claims include, however, other mechanisms of action, both presently known and unknown, including the use metal ions containing proteins as mediators of critical plant viral functions.

Furthermore, the claimed invention applies to other pathogenic viral infections produced by plant viruses with or without zinc finger proteins in their structure, both presently known and unknown. It is also applies to other pathological conditions of plants where "plant" refers to any plant or plant cells at any stage of development, including seeds, suspension cultures, embryos, meristematic tissues, callus tissue, leaves, roots, shoots, gametophytes, sporophytes, pollen, and microspores, and progeny thereof. Including also cuttings, and cell or tissue cultures derivatives of cloned cells. The term "plant tissue" includes, but is not limited to, plant cells, plant organs (e.g., roots, meristems, etc.) plant seeds, protoplasts, callus tissue, and any set of plant cells systematically organized into structural or functional units.

The present novel antiviral agents can be used to modulate gene expression, alter genome structure, and silence specific genes. The plant types are preferable the class of higher plants amenable to transformation techniques, such as monocots and dicots.

The antiviral agents of the present invention can also be used in species from the following genera: Gramineae including Sorghum and Zea. Cucurbita, Rosa, Fragaria, Lotus, Medicago, Onobrychis, Juglans, Olium, Trigonella, Raphanus, Sinapis, Atropa, Capsicum, Datura, Vigna, Citrus, Linum, Geranium, Manihot, Daucus, Arabidopsis, Vigna, Citrus, Linum, Geranium, Manihot, Daucus, Brassica, Hyoscyamus, Lycopersicon, Nicotiana, Solanum, Petunia, Digitalis, Majorana, Clahorium, Hellanthus, Pennisetum, Ranunculus, Senecio, Salpiglossis, Cucumis, Browaalia, Glycine, Pisum, Phaseolus, Lollum, Oryza, Avena, Hordeum, Secale, Lactuca, Bromus, Asparagus, Antirrhinum, Heterocallis, Nemesis, Pelargonium, Panieum, and Triticum.

Plant cells of great commercial value which are frequently infected by plant viruses and which can be treated by the novel antiviral agents of the instant invention includes: those from rice (Oryza sativa), rye (Secale cereale), corn (Zea Mays), canola (Brassica napus; Brassica rapa ssp.), alfalfa (Medicago sativa), sorghum (Sorghum bicolor, Sorghum vulgare), sunflower (Hellanthus annus), wheat (Triticum aestivum), soybean (Glycine max), tobacco (Nicotiana tabacum), Gossipium (hirsutum), sweet potato (Ipomoea batatus), potato(Solanum tuberosum), peanuts (Arachis hypogaea), cotton (Gossypium barbadense), cassava (Manihot esculenta), coffe (Coffea spp.), coconut (Cocos nucijra), pineapple (Anana comosus), citrus trees (Citrus spp.), cocoa (Theobroma cacao), tea (Camellia sinensis), banana (Musa spp.), avocado (Persea americana), fig (Ficus casica), cashew (Anacardium occidentale), macadamia ( Macadamia integrifolia), almond (Prunus amygdalus), sugar beets (Beta vulgaris), sugarcane (Saccharum spp.), duckweed (Lemma spp.), oats, barley, vegetables, ornamentals, guava (Psidium guajava), mango (mangifera indica), olive (Olea europaea), papaya (Carica papaya), and conifers.

Vegetables which can be treated by the methods of the disclosed invention, include tomatoes (Lycopersicon esculentum), lettuce (e.g., Lactuca sativa), green beans(Phaseolus vulgaris), lima beans (Phaseolus limensis), peas (Lathyrus spp.), and members of the genus Cucumis such as cucumber (C. sativus), cantaloupe (C cantalupensis), and musk melon (C. melo). Preferred ornamentals include azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbia pulcherrima), and chrysanthemum), are also treatable

Conifers that further research may demonstrate can be treated with the agents of this invention include, for example, pines such as loblolly pine (Pinus taeda), slash pine (Pinus elliotil), ponderosa pine (Pinus ponderosa), lodgepole pine (Pinus contorta), and monterey pine (Pinus radiate), Douglas-fir (Pseudotsuga menziesil), western hemlock (Isuga Canadensis), sitka spruce (Picea glauca), redwood (Sequoia sempervirens), true firs such as silver fir (Abies amabillis) and balsam fir (Abies balsamea), and cedars such as Western red cedar (Thuja plicata) and Alaska yellow-cedar (Chamaecyparis nootkatensis). Most importantly, the present invention can be used to treat diseases of crop plants such as corn, alfalfa, sunflower, canola, soybean, cotton, peanut, sorghum, wheat, and tobacco.

### EXAMPLE 10

### SUPPRRESSION OF PLANT VIRAL INFECTIONS BY ZFPs SPECIFICALLY AND IRREVERSIBLY BOUND TO HOST PLANT CELL DNA RESPONSE ELEMENTS (DNA-RE).

R. van Wezel, et al (J. Virol, Jan. 2003, Vol.: 77: 696-700) explored the contributions of the zinc finger motif, its interaction with the zinc atom and viral DNA Binding by a suppressor of Post-transcriptional Gene (PTGS) Silencin. Postranscriptional gene silencing (PTGS) in plants, RNA interferencing in animals, and gene quelling in fungi share a common molecular mechanisms in which a target RNA is trans-inactivated by homology-dependent RNA degradation. In plants, PTGS protects the host against virus infection. Consistent with the active role of PTGS in antiviral defense, plant viruses contain counterattack functions by encoding proteins that are capable of suppressing PTGS. PTGS suppressors often enhance viral pathogenicity. A number of suppressor proteins that have been characterized. The AC2 and C2 proteins of *African cassava mosaic virus and Tomato yellow leave virus of China* (TYLCV-C) are of particular interest for the instant invention. The results of many studies and mutagenesis analysis have indicated that the altered biochemical behavior of the three zinc-finger mutants in zinc and DNA binding correlates with a loss of biological function in inducing necrosis and suppressing PTGD in plants.

In plants, PTGS protects, the plant cells, to a certain extent, against virus infection by neutralizing the deleterious effects of the virus on PTGS. Some pathogenic viruses contain proteins that suppress PTGS and enhance pathogenicity. There is a large variation in the extent and effectiveness of PTGS suppression by different viruses which express different virus-encoded suppressor proteins, and thus the pathogenicity is variable.

Van Wetzel demonstrated that the *Geminivirus* TYLCV-C C2, *a protein of genus Begomovirus,* is *a zinc finger protein that induces plant cell necrosis and suppresses PTGS.* In plants, PTGS is a relatively effective plant defense mechanism against infection by RNA and DNA viruses, but not against every virus. For example, in plants, at the transcriptional level, the C2 protein enhances pathogenicity of *Begomoviruses* and thus the *"plant defense mechanism based on PTGS" is useless* for the plant.

The results of the experiments with PTGS described above, strongly suggest that more powerful methods will have to be designed and developed to address the challenges of agricultural biotechnology and the invasion of plants and plant cells by numerous pathogenic viruses. One promising methodology to control gene expression to prevent viral infection in plants is the use of synthetic zinc finger protein transcription factors to manipulate the expression of endogenous genes in plants to prevent the infection by various types of plant viruses (Stege, et al; Plant J. 32:1077-1086, 2002).

The antiviral agents of the instant invention are capable of disrupting cellular ZFP such as ribosomal proteins, etc., depriving the invading virus with the ability to control viral replication at the translational ribosomal level [a critical cellular machine for survival]. Furthermore, the inventors has isolated and cloned numerous proteins which can be used to silence *DNA response elements* (**DNA-RE**), in animal and plant cells. The Response Elements are specific DNA sequences that control critical genes for the host plant cell. Many of the invading viruses contain ZFP that can be disrupted by the agents of this invention.

Ribosomal proteins with zinc finger motifs which bind to DNA and RNA (Wool, 1996). Essentially identical results were obtained with cloned ribosomal cDNA of human breast cancer cells cloned and isolated by Fernandez-Pol, (1989). These proteins have similar **DNA/RNA** binding properties than ZFPs of plant and animal viruses involved in transcription and viral replication. Thus, ribosomal proteins with zinc finger motifs can control genomic activity as well as interfere with invading viruses.

Ribosomal zinc finger protein is multifunctional (Fernandez-Pol, 1995). The multifunctional ribosomal protein Metallopanstimulin-1 (MPS-1/S27), and other proteins were used to perform the following experiments: 1) Some genes can be suppressed and silenced after the MPS-1/S27 protein is dimerized, phosphorylated, specially prepared with iron, and then specifically bound to its corresponding **DNA Response Element** (**DNA-RS**) (Fernandez-Pol, Anticancer Res., 2002, 2003).

The inventors exchanged the zinc in MPS-1/527, which cannot be oxidized and is a non-toxic transition metal, for another non-toxic metal ion [Fe2+] which produces specific damage on DNA-RE *on site* because of its ability to perform Redox cycles (Fe2+/Fe3+). After binding to the DNA-RE, and making contact with the DNA bases, the dimerized-phosphorylated MPS-1/S27 protein binds specifically to the DNA-RE of the gene which is controlled by MPS-1/S27 and other proteins. After the redox reaction is initiated by addition of Ascorbic acid, the gene RE is destroyed in a **non-random fashion**. The metal selected for redox was a salt of iron (Fe2+) because the large redox potential.

The Zn ²⁺ of the MPS-1/S27 was exchanged by the Fe2+ and thus an *"iron finger protein "MPS-1*/*S27* [Fe ²⁺] was created to specifically bind to the DNA-RE corresponding to MPS-1/S27. The DNA-RE is a controlling/promoter region for one or more, specific genes for cell division. The plant viruses can also used the sequence DNA-RE to control specific protein synthesis of the corresponding gene. In fact, ribosomal protein synthesis is the critical step in the production of proteins from amino acids and is the only synthetic system that correlates precisely with both cell and viral division. Thus, to irreversibly silence, or eliminate plant cell survival genes such as ribosomal genes, for a pathogenic virus, destruction of ribosomal genes will result in lethal consequences for the virus. The plant cells will enter into Program Cell Death (PCD) but the virus will be disintegrated together with PCD process. It is likely that the cell, depending upon how fast the treatment was initiated, will enter PCD, but propagation of the virus will be eliminated. This sophisticated and highly specific method of silencing DNA-RE presented here, combined with the novel antiviral agents of this invention, can prevent the control of PTGS by any virus using this system.

More specifically, when the **Iron-Finger Protein (Fe-FP)** is bound to the specific control sequence of the DNA, denoted RE for enhancers (REEP) or suppressor protein (RESP) if the genes are stimulatory or inhibitory, respectively, in the presence of added Ascorbic acid there was production hydroxyl radical [OH], the most damaging oxidative agent known for cells. The recombinant protein utilized was identical to MPS-1/S27. It was denoted "iron finger protein" [Iron-FP] of the class RESP because it binds to the RE in the DNA that controls one gene involved in PTGS.

The binding of MPS-1 to its RE, in the presence of ascorbic acid, released the powerful hydroxyl radical [OH], peroxidase and superoxide and **destroyed non-randomly** the DNA-RE double-stranded plant cell DNA which controls PTGS. The destruction of the DNA was not random but consistent with an algorithm that the inventors call the "**Ladder form**", the destruction occurs only in the DNA area where MPS-1/S27 protein was bound to the DNA-REM. DNA electrophoresis showed that this was the case and that the MPS-1/S27 gene controlled by DNA-REM that is essential to counteract many *genotoxic* and *viral infections* [ protective activities] and the controlled gene it behaves as a PTGS. As a consequence, viral infection of plant cells cannot occur because the PTGS have lost the DNA-RE and thus it was permanently silenced and cannot function anymore. This is particularly compelling for ribosomal zinc finger proteins because the cell's survival is dependent on their integrity. The inventors believe the method described here can be used in any plant or plant cells to prevent viral invasion in an effective fashion. Furthermore, in conjunction with the novel antiviral agents of the instant invention, the zinc finger proteins of the virus should be disrupted and the virus render inactive to neutralize the protective mechanisms of plant cells such as PTGS.

In summary, by exchanging Zinc (which cannot be oxidize) by Fe2+, which could redox between 2+ and 3+ and generate OH and other deleterious oxidative agents, it can specifically bind to the exact site denoted DNA Response Element which is destroyed by the Fe 2+/3+ upon contact with DNA. Unlike other methods, this approach permanently suppresses the DNA-RE utilized by viruses. If the DNA-RE corresponds to the Posttranscriptional gene (PTGS), this gene will be silenced, and the control element (RE) eliminated, rendering the PTGS useless. The proteins of the pathogenic virus which normally control PTGS can be rendered useless, and as the disorder increases by lack of regulation of **PTGS**, the gene cannot function properly. Thus, if the virus invades the plant cells it will be unable to control critical plant cell genes whose control has been eliminated by the method of this invention. The therapeutic applications of MPS-1 [Fe2+/3+] in which the Zinc was replaced by Fe2+ was described by Fernandez-Pol (Anticancer Research, 2001)

There are many approaches to obtain the goal of silencing genes. The inventors believe that the approach disclosed is the most specific (a recombinant protein with Fe ²⁺), the DNA-RE is cut in DNA ladders by the OH generated by the Redox reaction and the cell is rendered non-functional, because it cannot fight the deleterious genotoxic effects, which is one of the properties that characterize the ribosomal ZFP MPS-1 (Fernandez-Pol, 1995). When this methodology is properly combined with the antiviral agents of this invention, viral replication, movement, and encapsidation do not occur.

### EXAMPLE 11

### PLANT DISEASE RESISTANCE: TANDEM REPEATED CASSETTE TRS CONSTRUCTS INCREASE RESISTANCE OF TRS SYNGENIC PLANTS TO VIRAL INFECTION.

For convenience in identifying the essential elements of the invention in the area of TRS syngenic plant and plant cells and resistance to pathogenic viruses, the methodologies used are: (1) Enhanced natural resistance of wild-type plants by using exogenously added picolinic acid; and (2) Enhance resistance in plants using **cassette TRS construct** technologies to endogenously increase picolinic acid production.

**Natural resistance of plants:** The resistance mechanisms of plant and plant cells to viruses are not well understood. Some individual plants become resistant after chemical, physical or environmental treatments, but the mechanisms of this phenomenon are poorly understood and are not uniform. In one scenario, viral disease development in plants and plant cells is a dynamic process that activates initially a few genes to counteract the virus. The second reaction of the plant to the virus is by the action of many genes (amplification of resistance) that coordinate a response to the invader. In most cases, however, the virus takes over control of the plant cells' transcriptional machine with its powerful promoters and transcriptional factors. Then, the virus reproduces rapidly and the plant cells overproduce proteins required by the virus for replication such as DnaJ proteins, MPS-1/S27 ribosomal protein and other proteins. The result in this case is PCDs for the plant cell infected by the virus. The methodology of external exposure to antivirals is quite acceptable and effective to control the virus *but not to eliminate it,* because a small percentage of *Geminiviruses* hide in regions of the cells not reach by external antiviral agents. Thus, the methods of this invention disclose ways of eliminating the virus and the unrecoverable infected cell.

### Plant Resistance to viral infection using Cassette TRS construct technologies.

Resistance to viral disease using the novel technologies disclosed in this invention involves the following: 1) introduction of a segment of DNA containing the desired genetic encoding sequence material that will be inserted in the genome of the plant cell without altering any other plant gene. In this case, the protein produced by the DNA construct is designed to interfere with some specific function of the pathogenic virus mediated by a viral protein. In the last 15 years numerous experiments were done and the results with proteins produced with the DNA construct to control plant viruses were mixed: 1) Some proteins were partially or fully successful in protecting plants against viruses of limited virulence and laboratory experimental conditions; 2) Some genes that were used as controls unexpectedly had an effect on the viral disease that resulted in partial protection of the plant; and finally, 3) Other genes that were predicted to confer resistance did not. It appears that all these experiments had some degree of **genetic** instability and that not all the factors involved in neutralizing and destroying a plant virus were either considered or known.

### EXAMPLE 12

### DEVELOPMENT OF NON-TOXIC ANTIVIRAL AGENTS FOR TREATMENT OF VIRAL DISEASES IN PLANTS AND PLANT CELLS

The inventors took a more direct approach. Rather than exposing the plants to external antiviral agents, it would be more convenient and deathly to the virus to have the plant cells themselves producing intracellularly the naturally occurring antiviral agent picolinic acid or analogs thereof. We know that small molecules can be used as antiviral agents [*specific bullets*] for plant viruses and also we know in detail that the zinc finger motif [*specific targets* for the bullets] is attacked by the small molecules such as picolinic acid and analogs. Thus, in this case, there is no survival or possibility of mutation for the pathogenic virus containing ZFPs, since the picolinic acid at pharmacological doses, produced by the plant and plant cells will permeate all plant compartments disrupting the zinc finger protein domains and effectively terminating with the possibility of viral survival.

In the first phase of the development of antiviral agents for agricultural uses, the inventors studied the application of naturally occurring antiviral agents such as picolinic acid and fusaric acid and they were applied topically to the plants infected with common viruses. The inventors decided that to really solve the problem and eliminate *Geminiviruses* and other viruses containing ZFPs, one should pursue a radically new concept of treating viruses with small molecules antiviral agents, *destroying the virus in any protective cell compartment in plants and plant organs.* For all those reasons, the inventors decided to generate syngenic plants and plant cells that can produce endogenous picolinic acid or analogs thereof, intracellularly, only in the presence of the pathogenic virus.

### EXAMPLE 13

### TRS SYNGENIC PLANTS PRODUCE ENDOGENOUS PICOLINIC ACID AS AN ANTIVIRAL AGENT THAT ELIMINATES THE INVADING VIRUS.

As used herein, "TRS *syngenic plant"* or *"genetically TRS modified plant"* includes a plant which comprises within its genome a heterologous DNA polynucleotide. Generically, and preferably, the heterologous DNA polynucleotide is stably integrated within the plant genome such that the polynucleotide is passed on to successive generations of plants. In the case of the instant invention, the heterologous DNA polynucleotide [containing all the functional units] can be integrated into the genome alone. "TRS syngenic" is used herein to include any plant cell or tissue culture plant cell line, callus tissue, a portion of a plant.

The genotype of the syngenic plant has been modified only by the addition of a heterologous DNA nucleic acid (**Cassette TRS construct**) inserted in a non-coding region of chromosomal DNA. Subsequent TRS syngenics can be created by sexual crosses or asexual propagation from the initial TRS syngenic. The term "TRS syngenic" as used herein does not encompass the alteration of the plant genome (chromosomal or extra-chromosomal) by conventional plant breeding methods or other methods well known in the art. In summary, *the phenotype of the wild type plant and plant cells and the TRS syngenic plant and plant cells are indistinguishable by standard examination.*

### EXAMPLE 14

### Creation of plants and plant cells that produce wide-spectrum antiviral agents by using Agrobacterium tumefaciens.

There are numerous methods for the introduction of gene (s) of interest into the plants and plant cells. **FIG. 18** illustrate the use of ***Agrobacterium tumefaciens*** to transfer the genes of interest assemble in a TRS Cassette construct. The selected DNA sequence can be placed into the plasmid vector at an appropriate restriction site. A large number of versatile vectors are commercially available. The vectors contain replication signals, generally from E. *coli,* and a marker gene for selection of E. *coli* containing the transfected gene. The sequence of interest can be inserted into the vector in one of various restriction sites. The plasmid with the gene incorporated is used in subsequent steps for the transformation of E. *coli* cells. There are numerous plasmids that can be used as vectors, such as pBR322, M13, pcDNA II, pBlueBac/pJVETL, etc. After transformation of E. *coli* cells containing the plasmid and the desired sequence, the cells are grown, lysed and the plasmids purified. The plasmid DNA is characterized as follows: Restriction enzyme analysis, gel electrophoresis and transfer to a membrane. The DNA fragment is cleaved and the inserted segment is identified and sequenced.

The plasmid of interest with the verified sequence of interest can be cloned or subcloned in other plasmids to be used for specific tasks, such as production of protein (e.g. Baculovirus). Standard cloning methods can be found in "Molecular Cloning", a laboratory manual, Maniatis et al, Cold Spring Harbor Laboratory Press. More detailed methodology preparation of a cDNA library, preparing probes and screening of cDNA library, sequencing of cDNA, computer correlation with Picolinic acid carboxylase, viral and cellular zinc finger proteins, HPLC measurement of picolinic acids, PCR of generated products and other constructions can be found in Fernandez-Pol, USA Patent 5,243,041, Set. 7, 1993)

There are numerous well-established versatile techniques for the introduction of plasmid DNA into a plant cell. Typical vectors useful for expression of genes in plants, include vectors derived from the **tumor inducing (Ti) plasmid** of *Agrobacterium tumefaciens* (**FIG. 18**). These vectors can integrate a portion of the vector DNA into the genome of the plant cells. Commonly utilized *A. tumefaciens* vectors are denoted plasmids pKYLX6 and pKYLX7. These methodologies provide an immediate phenotypic effect.

More specifically, *A. tumefaciens* is a gram-negative soil bacterium with the unique ability to infect plants utilizing a process that involves the delivery of a specific segment of its genome to the nuclei of susceptible plant cells (**FIG. 18**). The transferred DNA (T-DNA) is a discrete region of the bacterial genome which contains repeated border sequences (**FIG. 18**). Both types of transfers can be obtained: stable and transient transformants. Although *Agrobacterium* only forms tumors on dicotyledonous plants, T-DNA transfer does occur in monocots, including Cassava, but does not lead to tumorigenesis.

The construction of **TRS syngenic plants** involve the *Agrobacterium* binary vector system, in which a binary vector is introduced into a *disarmed Agrobacterium* strain with a Ti plasmid lacking the T region (helper plasmid) (**FIG. 18**). The binary vector contains a plant-selectable marker (either an antibiotic- or herbicide - resistance gene under the control of plant cell expression signals). It also contains a multiple cloning site between the left and the right border repeat in which the gene(s) of interest can be cloned in E. *coli.* The resulting construct -plasmid/gene selected- vector is then transferred to an *Agrobacterium* helper strain. Subsequently, the T-DNA can be transfer into plant cells. The genome *of Agrobacterium* consists of two chromosomes, one circular and one linear, and often multiple large plasmids. **FIG. 18** illustrates the process of transferring genes from *Agrobacterium* into to a plant cell. This system has been utilized to produce countless of syngenic plants with unique phenotypes, such as resistance to herbicides and pests.

In the case of the instant invention, the creation of TRS *syngenic Cassavas* with versatile promoters that are activated by the transcription factors produced by *Geminiviruses* (and other viruses) after plant cell infection, will significantly advance the protection of the Cassava plants as follows.

It is useful to describe here a few points about promoters and viral enhancer which will help to clarify the subsequent experimentation with plants and plant cells. For convenience in identifying essential elements of the regions of DNA vectors responsible for the biological activity of the preparations, a brief description of the different promoters and enhancers that can be used in DNA vectors of this invention, is briefly described as follows [Maniatis et al (Molecular Cloning , CSHL, 1982].

A promoter is a DNA sequence that directs RNA polymerase to bind DNA and to initiate RNA synthesis. The efficiency of the promoters depends on various factors. Strong promoters cause mRNAs to be copied at high frequency. Weak promoters control and direct the synthesis of uncommon transcripts. The only true test of the efficiency of a promoter is to measure the frequency with which the synthesis of the mRNA of interest is initiated. This value is difficult to obtain from in vivo studies. Thus, the efficiency of a promoter is frequently deduced indirectly from the level at which the protein product of interest is expressed in the cells.

An example relevant for this invention is that **E. *coli*** genes are controlled by weak promoters. The expression of such genes can be increased by placing them downstream from an strong promoter (e.g. trp, tac, etc). Efficient expression of eukaryotic proteins can be achieved only when the coding sequence is placed under the control of a strong E. coli promoter.

The most useful promoters for expressing eukaryotic genes in *E. coli* have two properties: they are strong and also they can be regulated. Obviously, coupling a gene that cannot be regulated is not appropriate; (2) Strong promoters also require the presence of a strong downstream termination signal to maintain the inserted gene on a plasmid.

### EXAMPLE 15

### IN SYNGENIC PLANTS CONTAINING GENOMIC PICOLINIC ACID CARBOXILASE (PAC) THE PROMOTER OF PAC IS ACTIVATED BY TRANSCRIPTIONAL FACTORS RELEASED BY VIRAL INFECTION AND AS A RESULT THE PROMOTER OF PAC PRODUCES INCREASED LEVELS OF THE ANTIVIRAL PICOLINIC ACID

The DNA promoter of the Picolinic Acid Carboxylase (PAC) cloned in the same plasmids, have been designed by the inventors as the targets for activation by the *Geminivirus* transcriptional activators. Thus, as the viruses uncoat their proteins and genetic materials inside the plant cells, the viruses release *Geminivirus* transcriptional factors (GTF). After cellular processing, the GTF can activate the PAC promoter which will lead to the formation of the enzyme mRNA for PAC. After processing of the mRNA by the ribosomes and proper folding by the Chaperone System, the resulting PCA enzyme is ready to produce Picolinic acid in the presence of an adequate substrate. In the presence of the substrate of PCA, the PCA enzyme converts the substrate into picolinic acid. The PCA is classified Internationally (IUC) as follows (**Table 10**) : 1. Oxidoreductase; 2. Transferase; 3. Hydrolase; 4. Lyases; 4.1 Carbon-carbon lyases; 4.1.1 Carboxy-lyases. 4.1.1.45.: Aminocarboxymuconate-semialdehyde decarboxylase; picolinic acid carboxylase; picolinic acid decarboxylase.

Transformed plant cells can be selected by co-transfer of an antibiotic resistant marker and regenerated into fertile syngenic plants. T-DNA integrates into the plant nuclear genome by a process termed "illegitimate" recombination.

Transformation of Monocots has developed into the efficient, reproducible and stable transformation of rice, corn, wheat and other monocots such as Cassava. The key factor in the success of monocots protocols was the use of actively dividing cells such as immature embryos. The availability of strategies to deliver foreign DNA genes to specific sites in the plant genome where there are strong promoters is critical for the successful production of significant quantities of endogenous picolinic acid by PAC.

*A. tumefaciens* is readily manipulated in such a way that *plasmids carrying foreign genes of interest are easily introduced into appropriated bacterial strains for delivery to plants.* First, the strains for delivery to plants are **"disarmed"**, that is deleted of oncogenic T-DNA but still possessing intact Ti plasmid and chromosomal vir genes. Foreign genes such as PAC, destined to be introduced in the plant cells, can be cloned into a plasmid that carries a single T-DNA border sequence or two T-DNA border sequences that flank numerous restriction sites for cloning. Adjacent to the restriction site is an antibiotic resistance gene to select for transformed plant cells. The plant and plant cells obtained by this technology can be examined for the presence of the DNA introduced. (Extensive details can be found in Willmitzer: Syngenic Plants in Biotechnology (1993).

In summary, the finding that oncogenes can be deleted from T-DNA and replaced with genes of interest resulted in the industry of plant genetic engineering. Monocotyledonous plants or their cells can also be transformed by means of vectors based on *Agrobacteria* (Chang et al, 1993). The resulting transformed cells grow within the plant in a way that is undistinguishable from normal plant cells. The resulting hybrid has characteristic phenotypical properties. Plant cells that contain the nucleic acid sequences and express the protein of the invention (PAC) can be cultivated indefinitely.

The person skill in the art, with conventional gene technology, can integrate the new nucleic acid molecules coding for PAC into the plant genome. Thus, stable transformants can be produced and the nucleic acid molecules containing the encoded PAC are replicated with the plant genome. With a different vector system, it is possible to produce plants which act as an independent replicating system in the plant cytoplasm. This is accomplished by having DNA sequences that allow the replication of the plasmids within the cells.

The full-length cDNA clone contains the PCA enzyme. The encoded protein, Picolinic Acid Carboxylase, is a critical enzyme in the pathway of picolinic acid and quinolinic acid (which leads to nicotinic acid and NAD+). PAC is the enzyme that the inventors selected to produce Picolinic acid in the presence of an adequate substrate when it is stimulated by a *Geminivirus* strong protein promoter.

In summary, when *Agrobacteria* are used for the transformations that will lead to the syngenic plants, the DNA to be transfected in the plant cells must be cloned in special plasmids, denoted intermediary or binary vectors (**FIG. 18**). The use of the *Agrobacteria* for transformation of cells has been extensively investigated and is well known in the art.

### EXAMPLE 16

### GENERATION OF GEMINIVIRUS RESISTANT CASSAVA PLANTS BY SIMULTANEOUS DISRUPTION OF BOTH GEMINIVIRUS ZFPs AND PLANT ZFPs INDUCED BY GEMINIVIRUSES

To make the Cassava plants resistant to infectious viruses and disrupt the use by plant viruses of *virus-induced overproduction* of DnaJ ZFPs, ribosomal ZFPs, and other ZFPs required for viral replication, disassembly, assembly and movement, and thus making the plant cells partially or completely resistant to infection by *Geminiviruses,* a plasmid can be constructed to produce the enzyme Picolinic Acid Carboxylase (PAC), which after transcription and translation of PAC mRNA in the plant cells will result in the production by PAC of Picolinic acid in the presence of the appropriate substrate. This type of PAC enzyme, once properly folded by the Chaperon System generates the following products: 1) In the presence of the natural substrate of PAC, 2-amino-carboxy-muconate semialdehyde; 2) the substrate is transformed by PAC in 2-aminomuconate semialdehyde; and 3) in a non-enzymatic reaction loses H₂O, and is converted into Picolinate (2-pyridinecarboxylic acid). The 2-amino-3-carboxy-muconate semialdehyde can be converted in picolinic acid if the PAC is active and the substrate accumulates in Quinolinate (precursor: 3-hydroxy-anthranilate; enzyme: hydroxyl anthranilate). Quinolinate, after a series of enzymatic steps ends as Nicotinate and nicotinamide, finally producing NAD and NADP, two essential energy producing molecules for redox potential of cellular membranes..

The induction of the PAC system by the syngenic plants cells containing genomic PAC *produces picolinic acid only when the strong promoter of PAC is activated by transcriptional viral proteins that are released by the Geminiviruses during plant cell infection.* As a result, the promoter of PAC is activated by the viral proteins and produces increased levels of the antiviral agent picolinic acid intracellularly. The production of high levels of Picolinic acid by the virus-activated PAC enzyme can disrupt the expression of numerous virus-induced-overproduced ZFPs such as *Geminivirus* ZFPs; and both DnaJ, and ribosomal proteins with zinc finger domains. The syngenic plant production of picolinic acid induced by the *Geminivirus* transcriptional proteins acting on the strong promoter of PAC produces picolinic acid under the control of the pathogenic virus. By using this feed-back control system [promoter-virus], *the instant invention controls the viral infection at several different levels such as disassembly, viral protein synthesis, assembly, encapsidation and movement.* The result of the production of endogenous picolinic acid at high levels will result in the destruction of the virus and possibly the cure of the Cassava plant cells infected by the *Geminiviruses.* After the *Geminiviruses* are disrupted and disintegrated in the plant cells, the PAC system stops the production of picolinic acid because of the absence of stimulation of the strong promoter of PAC by the lack of viral encoded proteins stimulation.

### EXAMPLE 17

### GENERATION OF THE PLASMID pPAC-1 ENCODING PICOLINIC ACID CARBOXYLASE

Typical vectors useful for expression of genes in higher plants are well known by molecular biologists and include vectors derived from tumor-inducing (Ti) plasmid of *Agrobacterium tumefaciens* (Rogers et al., Methods in Enzymol., 153:253-277, 1987). The main characteristic of these vectors is that they are plant integrating vectors in that on transformation, the vectors integrate a portion of the DNA vector into the genome of the host plant. For example, *A. tumefaciens* vectors useful for this invention are the plasmids pKYLX7, and pKYLX6; and the plasmid pBI101.2. The use of these plasmids should provide an immediate phenotypic effect concerning PAC *[only production of picolinic acid*] but not in other respects. Thus, *Geminivirus* replication can be inhibited, and the virus destroyed by at least one or all of the following disrupting actions of picolinic acid on zinc finger domains of: (1) *Geminivirus* and other viruses containing intrinsic ZFPs which are released intracellularly to initiate the process of replication; and (2) Plant cell ZFPs induced by the viral proteins acting on plant cell genes to overproduced ZFPs proteins such as DnaJ, ribosomal ZFPs, and other plant cell ZFPs utilized by the virus. The target sequences (zinc finger motifs) can be disrupted by picolinic acid and derivatives thereof generated by the virus-induced PAC enzyme production in the presence of the adequate enzyme substrate.

Cassava plant cells can be transformed with plasmid pPAC-1 [containing PAC DNA sequence] enclosed in *Agrobacteria* according to the method described above and in Hooykaas, P.J.J, Agrobacterium, Encyclopedia of Microbiol. Vol. pp78-85; 2000). A number of kanamycin-resistant primary transformants can be tested for expression of the construct. Northern blot analysis, Southern blot analysis and HPLC can be used to test for the PAC DNA gene sequence inserted into the genome of the syngenic plant cells, after restriction enzyme cutting. The resulting cDNA fragment of PAC can be used as a probe for the RNA hybridization.

### EXAMPLE 18

### ANALYSIS OF SYNGENIC PLANTS

The following description of general methods which can be used in conjunction with the present invention is intended for illustrative purposes only. Other alternative or more sophisticated methods and embodiments will be apparent to those skill in the art after reviewing in detail this disclosure.

Numerous analyses are required to determine the stability and the degree of success of the syngenic plants generated as described in the instant invention. The present example describes the techniques required to characterize the clonal colonies containing the cDNA sequence of the invention (PAC). Capillary zone electrophoresis (CZE); release of radioactive Zinc-65 from labeled zinc finger proteins in the presence of picolinic acid or analogs; High Pressure Liquid Chromatography (HPLC) for characterization of Mr of ZFPs and small molecules such as picolinic acid or analogs; detection of disulfide cross-linked zinc finger proteins-Picolinic acid -Zinc by gel mobility shift assays; Nuclear Magnetic Resonance (NMR) detection of zinc loss or formation of ternary complexes by zinc-protein-picolinic acid or analogs. Analysis of Transcription of the Encoded PAC sequence Using "Northern Blot" Analysis to detect the production of PAC mRNA by the pPAC-1 plasmid. The techniques delineated here can be used as described in detail by Fernandez-Pol (DNA vector with isolated cDNA gene encoding metallopanstimulin; US Patent No. 5,243,041, 1993).

Total bacterial and plant cell RNA can be isolated, with minor modifications by techniques described in US Patent '041, 1993. Analysis of Syngenic Plants with RT-PCR can be done as described (Xynos, Anticancer Res., 1983). RT-PCR will be used to confirm the results of Northern blot analysis, with respect to mRNA expression of *Geminivirus* intrinsic zinc finger proteins, mRNA of DnaJ proteins, mRNA of MPS-1/S27 ribosomal protein, and other zinc finger ribosomal proteins expressing mRNAs by viral induction. The inventors expect that the syngenic plant cell lines examined will not be phenotypically different from non-transformed wild plant types (WT).

### EXAMPLE 19

### GEMINIVIRUS INFECTION OF SYNGENIC CASSAVA PLANTS AND PLANT CELLS: METHODS OF TESTING RESISTANCE OF PLANTS AND PLANT CELLS TO THE VIRUS

Testing of viral infection by *Geminivirus* in Cassava syngenic plants can be done to demonstrate the efficacy of the method of the instant invention as follows. The syngenic plants and the respective controls (Wild Type Cassava) can be examined at random and the PAC protein levels induced by the viral infection tested in purified plant extracts. Similarly, the Picolinic acid (or analogs if used as substrates) production can be tested by HPLC and quantified. After 30 min, 60 min, 3h, 6h, 12h, and 24h for a period of four days after the initial exposure of the plant to infection by inoculation of the leaves by inoculation of *Geminivirus* or whitefly vector carrying *Geminivirus* infected lymph inoculated in the leaves, syngenic plant samples will be obtained for the analysis of plasmid pPAC-1 promoter activation by the viral transcriptional proteins, restriction enzyme analysis, increased picolinic acid levels in plant cells extracts, and plant cell extracts in which PAC enzyme activity will be stimulated in vitro by viral promoter proteins. The ZFPs of *Geminivirus,* DnaJ and MPS-1 ribosomal protein will be also analyzed by methods previously utilized by Fernandez-Pol in US Pats. '041, Sept. 7, 1993; and '393, Oct. 3, 2000. Similarly, the Control Wild Type plants and control WT plant cells will be identically analyzed as the syngenic plants and cells. Wild type Cassava plants can be examined at random and the PAC protein levels induced by the *Geminivirus* infection tested in purified WT plant cell extracts. In the case of the WT Cassava plants unprotected by PAC enzyme, we expect all the results on the presence of both PAC and Picolinic acid to be negative even after viral infection.

We expect that the results will conclusively show that the interaction of the syngenic plant cells producing picolinic acid or analogs, can neutralize and disrupt the ZFPs of *Geminivirus* generated in the first few hours of the infection, resulting in the destruction of the virus ability to proliferate and assemble new virions. We estimate that after 1 to 3h of the infection by the virus, the picolinic acid levels will increased from 0 to 100-fold picolinic acid (PA) concentrations intracellularly. We expect that the PA concentrations will rapidly reach, within 3h, 0.5 mM Picolinic acid [normal universal concentration for the majority of enzymatic products inside animal and plant cells] and it will reach a peak or plateau of 3 mM to 6.0 mM for as long as the *Geminiviruses* are infecting the syngenic cells and releasing their viral products. Then, within 3h of virus destruction, and lack of PAC gene promoter enhancer activity, the levels of picolinic acid will decreased rapidly to basal levels of <0.5 mM in syngenic plants. However, if the viral PAC gene promoter proteins that act and enhanced PAC strong promoter gene activity are not quickly degraded by syngenic plant cell proteases, the levels of picolinic acid may be elevated by longer periods (a few days) which will be useful for subsequent plant cell viral infections which should be eliminated rapidly.

To verify the disruption of DnaJ proteins, and certain ZFPs of ribosomal origin such as MPS-1 which are induced by the *Geminivirus* in the host plant cells, these proteins, which are overproduced by viral induction, will also be analyzed as described above by Fernandez-Pol (1993; 2000). The production of picolinic acid can neutralize the *Geminivirus* zinc finger capsid proteins which are essential in establishing infection in plants and the plant cell ZFPs virus-induced accessory proteins (DnaJ, MPS-1, and various zinc finger ribosomal proteins) which are overproduced by viral infection and that perform important roles in the disassembly, assembly, proliferation, propagation and movement of the virus in the plant cells.

In general, and according to the model of the inventors of PAC production of picolinic acid, after 72 to 96 hours of exposure the hybrid plant cells producing picolinic acid should be *Geminivirus*-free and non-infectious to other plants, and whitefly vectors effectively controlling the viral propagation and eventually eliminating *Geminivirus* Cassava disease.

The inventors believe that both external exposure to picolinic acid or derivatives thereof and the use of syngenic plants with increased virus resistance producing endogenous picolinic acid can be a key factor in eliminating the *Geminivirus* pandemic in Cassava plants.

### EXAMPLE 20

### MISCELLANEOUS

The invention also encompasses the crop products and seeds of syngenic plants, and the plant cells with increased virus resistance. More specifically, the materials resulting from this invention include in general: Fruits, seeds, tubers, cuttings, leaves, etc. Moreover, they include portions of these plants, such as cells, protoplasts, cytoplasm, protoplasm, etc., from which a new syngenic plant can be originated.

The invention also includes the nucleic acid molecules containing the viral resistance genes and controlling sequences, which are described above in this invention. These nucleic acid molecules are specifically integrated into the plant genome.

There are no restrictions to the method of the invention to incorporate into a plant the gene or genes that will produce picolinic acid and apoptotic molecules that will destroyed the infected plant cells. In this particular invention, we focused in particular in the Cassava plant which is prone to acquire viral infections from *Geminiviruses.* However, the method can be use in any plant. To determine that the plants are syngenic, the nuclei acid containing the sequences of interest will be transferred to the plant by using vectors well known in the art, such as a plasmid, which replicates inside the plant cells, or a plasmid or nucleic acid that can be integrated into the plant genome permanently.

### EXAMPLE 21

### USE OF PROGRAM CELL DEATH (PCD) AND TRS CASSETTE CONSTRUCTS TO ELIMINATE PATHOGENIC PLANT VIRUSES

**APOPTOSIS**. Apoptosis is a morphological pattern of cell injury and an important mode of cell death (Robbins, Pathological Basis of Disease, 1999, 6th Ed). Apoptosis shares and overlaps common mechanisms with necrosis. The rapidity of the cell death process and the extent of ATP depletion undergone by the cell. This form of cell death is designed to eliminate defective, unwanted and damaged cells. For example, the inventors have shown that cells infected by Herpes Simplex Virus 1 (HSV; labialis) and HSV-2 genitalis are destroyed together with the host cells that they infected by apoptosis when exposed to pyridine carboxylates (Fernandez-Pol, Anticancer Res. 2002). Similarly, the inventors showed that HIV-1, Hepatitis C and numerous other viruses as well as the host cells have the same fate: Death by apoptosis after treatment with picolinic acid, fusaric acid or derivatives thereof. Thus, the inventors believes that when host cells of animals or plants are infected by pathogenic viruses, unless the treatment is extremely quickly, the host cells and the virus will be destroyed. In fact, host cells defective, mutated, and reservoirs for the novo synthesis of viruses are unwanted host cells and must be eliminated in many instances to allow the survival of the organism.

A set of evolutively selected apoptotic and anti-apoptotic genes and gene products sense ATP levels and controls the physio-pathogical state of the individual host cell to maintain a healthy host cell *versus* a damaged, unwanted host cell. For example, *if the infecting virus injuries the host cell and the host cell cannot recover, apoptotic cell death occurs.*

The inventors will briefly describe the morphological and biochemical events that characterize apoptosis. The morphological features are: 1) Chromatin condensation; 2) cell shrinkage; 3) cell surface membrane shows blebs and apoptotic bodies; 4) Phagocytosis of apoptotic cells by adjacent physiologically intact cells.

**The Biochemical basis of apoptosis** is complex and not well understood. It is unclear if plant cells possess apoptotic pathways that resemble animal cell apoptosis. However, it is clear that plant cells contain proteases that can degrade individual cells in a process that resembles animal cell apoptosis. Other data is in accordance with the concept that apoptosis do occur in plant cells.

**Protein Cleavage.** Protein hydrolysis is a specific characteristic of apoptosis which involves the activation of many members of the family of cysteine proteases denoted *Caspases .* Caspases cleave the cytoskeletal proteins, nuclear proteins and activate endonucleases. **Protein Cross-linking.** Transglutaminase activation covalently links cytoplasmic proteins. **DNA Breakdown in a characteristic ladder pattern**. Apoptotic cells show a peculiar breakdown of DNA of 50- to 300-Kb portions. Ca²⁺ and Mg²⁺ - dependent endonucleases continue to degrade the DNA in smaller units which are multiples of 180 to 200 base pairs forming a DNA ladder. **Activation of Apoptosis.** Apoptosis is activated by a myriad of conditions, drugs like picolinic acid and derivatives thereof, deprivation of growth factors , etc. The most relevant form of apoptosis for the instant invention are the actions of the antiviral agents of this invention such as picolinic acids and the specific injurious agents attacking cells represented by viruses such as *Geminivirus* and other virus using ZFPs as transcription factors.

One key control factor in apoptosis is that apoptotic death signals result in increased permeability of mitochondria with release of cytochrome c. The release of cytochrome c consistently occurs early in the process of apoptosis. It is mainly control by the gene Bcl-2 which is anti-apoptotic and thus prevents the release of cytochrome c.

The final phase of apoptosis has been termed "The Execution phase" which *is a proteolytic cascade of events.* The proteases that trigger and mediate the degradation phase are highly conserved throughout evolution and across species in animal cells. They belong to the *Caspase family of proteolytic enzymes. They are denoted Caspases to reflect the following actions: 1) the "c " refers to a cysteine protease mechanism; and 2) the "aspasa" reflects their ability to cleave after aspartic acid residues.* They can be divided in functionally in initiator and execution Caspases. Of interest for this invention is Caspase 9 that is an initiator which binds to Apaf-1, and Caspase 8, which is triggered by Fas-Fas Ligand interactions. The apoptotic death program initiated by rapid and sequential activation of caspases.

Recently, Cai et al (US Pat. Appli. Pub. No. : US 2004/0235846 A1, Nov. 25, 2004) found that substituted nicotinamides and analogs can activate caspases and are inducers of apoptosis. According to the inventors the compounds may be used to induce cell death in a variety of clinical pathological conditions. Nicotinamide was used by Fernandez-Pol (1977, PNAS) and was found only to flatten SV40-transfromed Balb 3T3 cells and had no effect on cell growth or apoptosis in the same cells at 48h. It appears that certain transformed cells may be resistant to apoptosis by nicotinamide (an analog of picolinic acid) while they were extremely sensitive to apoptosis by picolinic acid and derivatives thereof (Fernandez-Pol, 1977, PNAS)

It is anticipated by the inventors that caspase 9 can be incorporated into the genome of a syngenic plant in a "cassette construct" and when a virus infects the plant cell, the viral protein promoter can activate the strong, genetically designed caspase 9 promoter, constructed in a tandem gene of about 10 to 50 copies of caspase 9, with a strong stop codon, and produce the enzyme to eliminate the last traces of virus by initiating simultaneous the inactivated of their ZFPs transcription factor by picolinic acid or derivatives thereof and the direct or indirect cleavage of the promoter proteins of the invading virus. Like many proteases capases exist as zymogens and must undergo activation cleavage for apoptosis to be initiated. Interestingly, caspases can be hydrolyzed by other caspases and also autocatalytically. After the caspase 9 is activated the death program is initiated and irreversible, destroying the cell by apoptosis. The process is so efficient that the death cells disappear without leaving any sign of their existence and there is no detectable inflammation.

In summary, picolinic acid and derivatives thereof such as fusaric acid can initiate apoptotic cell death in virally transformed cells by damaging the mitochondria and subsequently allowed the release of cytochrome c which initiates apoptotic pathways, as shown by the inventors in 1977-1978 (Fernandez-Pol, Mol. And Cell. Pathology, '1978; PNAS, 1977). The process is highly efficient, as shown in many published papers by Fernandez-Pol *et al.* In order to make sure that the viral protein promoters are completely eliminated and the protein irreversible degraded , the inventors believes that an additional system such as the caspase 9 initiator enzyme or other zymogen should be able to irreversible degrade the entire plant cell and the infecting *Geminivirus* in conjunction and simultaneously with the actions of the antiviral agents picolinic acid and derivatives thereof which are able to induce rapid and efficient apoptosis of plant and animal cells. If for any structural and/or functional reason of plant specificity, the animal caspase 9 or 8 is unable to degrade the viral promoters, the inventors will use the cloned sequence of a conventional enzyme such catepsin D, constructed with strong viral promoters, in tandem, with a strong stop signal and the "cassette construction" will be inserted in several chromosomes of the host plant cells to prevent polymerization of viral protein promoters which could overcome the hindrance of the tandem construction by bypassing the non-coding regions of the tandem constructs contained in the "Cassette Construction". The inventors believes that this can be an efficient and rapid "doubled hit" method to eliminate without any doubt the *Geminivirus* and other viruses that infect valuable commercial crops around the world.

### EXAMPLE 22

### ELIMINATION OF GEMINIVIRUS FROM CASSAVA PLANT CELLS: GEMINIVIRUS PROTEIN PROMOTERS AS ACTIVATORS OF PLANT CELLS TRS ENCODED PROTEOLYTIC ENZYMES INDUCED PCD IN VIRALLY INFECTED PLANT CELLS

Apoptosis (in animal cells) or Program Cell Death (PCD; in plant cells) is an important feature of plant regulation of cells that are injured or unwanted. However, the mechanisms in plants utilize different proteases than those used by animal cells. In the majority of the cases, PCD in plants is manifested by the formation of a large vacuole, which ruptures and releases hydrolytic enzymes that degrade the cell contents. As in the case of animal cells, DNA degradation and activation of proteases also occurs. If caspase-like activity occurs, that happens in a minority of the cases. In the majority of plant systems, there is convincing evidence for the involvement of mitochondria and the release of cytochrome c. Plant proteolytic enzymes are numerous and are involved in PCD. The best known proteinases from plants are: subtilisin, papain, and metalloproteinases. Serine, cysteine, aspartic acid and threonine proteinases. Previous studies by the inventors demonstrated the involvement of mitochondria in apoptosis in SV40 transformed BALB-3T3 cells treated with 3 mM picolinic acid (Fernandez-Pol et al; Molecular and Cellular Pathology, 1978). The mitochondria of SV40 transformed cells first released cytochrome c and under observation by electron microscopy showed irreversibly damaged both the inner and outer membranes, clearly showing their incapacity to carry out their normal functions. The SV40 cells entered apoptosis within 3 to 48 h.

*On the molecular basis ofprevious Examples reported here, the inventors has design a system in which the activation of metacaspases can be used to eliminate pathogenic viruses in plants by utilizing the activation of proteolytic enzymes by viral promoter proteins.*

It is of interest to note here that overexposure of *Arabidopsis thaliana* to Ultraviolet-C light induces PCD mediated by caspase-like proteases (Danon, et al J. Biol. Chem. 2004;779-87). The protease cleaving the caspase substrate Asp-Glu-Val-Asp (DEVDasa activity) was induced within 30 min. These enzymes of *Arabidopsis thaliana,* other plants, fungi and protozoa are denoted **Metacaspases**. *It is conceivable that the inventors can used the activation of metacaspases to eliminate pathogenic viruses in plants.*

Fernandez-Pol *et al* studied the effects of Ultraviolet-C light (a strong mutagenic agent) on the expression of MPS-1/S27 protein in human Xeroderma Pigmentosum (XP) fibroblasts **(****FIG. 11****)**. The XP fibroblasts are extremely sensitive to cancer due to propagation of mRNA containing mutations. In plants, using *Arabidopsis thaliana* the effects of Ultraviolet-C light on MPS-1/S27 ribosomal protein and its mutants was also studied (**FIG. 9 and 10****; Table 12**). Revenkova demonstrated that the MPS-1/S27 protein was extremely important as a defense against PCD. The function of the MPS-1/S27 protein was to eliminate defective mRNAs by the enzymatic actions of MPS-1/S27 which functions as an *endonuclease,* and prevents the propagation of defective mRNAs which will produce unwanted mutations in *A. thaliana.* When the MPS-S27 gene is knock-out, numerous genetic defects appear in *Arabidopsis thaliana, some of these defects lead to cancer* (**FIGS. 9 and 10**). In numerous instances, these unwanted defects lead the cells to carcinogenesis or to PCD. In summary, in both Xeroderma Pigmentosum fibroblasts and Arabidopsis thaliana, Ultraviolet-C light induces mutations which in both XP fibroblasts and A. thaliana result in either cancer or apoptosis of the cancerous, unwanted or damage cells.

### EXAMPLE 23

### TANDEM REPEATED DNA VIRAL PROMOTER SEQUENSES FOR PICOLINIC ACID CARBOXYLASE (Fist death gene), AND FOR PROTEOLYTIC ENZYMES (Second death gene), INDUCED PCD IN VIRALLY INFECTED PLANT CELLS

*The essential bases of all systems reported here by the inventors to eliminate the pathogenic viruses from wild type or syngenic plants is the construction of two specific DNA units ["Cassette Units" A and B] with characteristics that can be summarized* as *follows:*

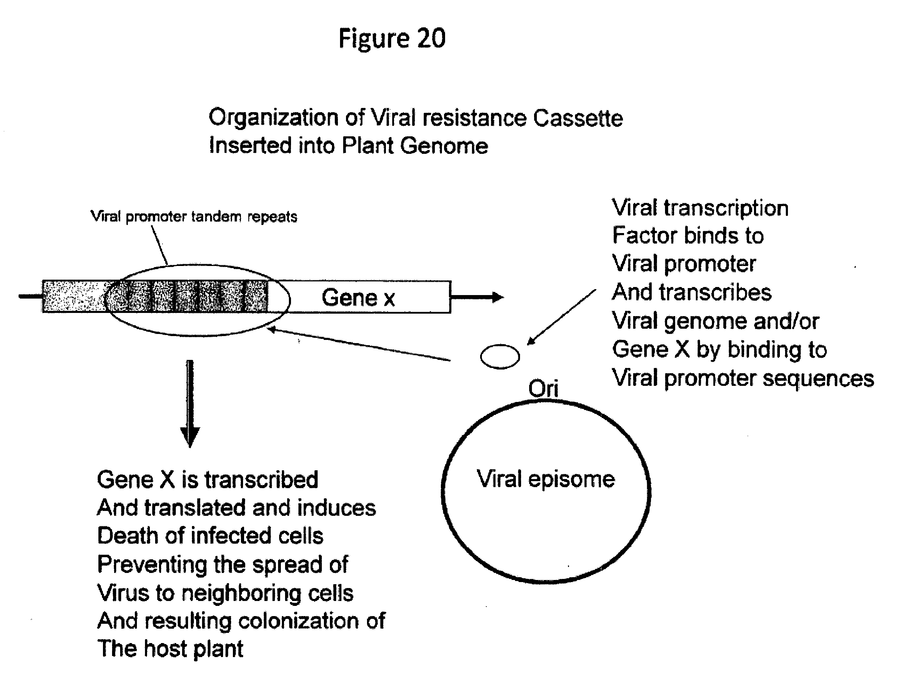

**FIGS. 9** **and** **20** illustrate that genes that exist as multiple adjacent copies are said to be in **tandemly repeated units**. *The extensive repetition reflects the requirement to produce large amounts of the protein product.* The repeating unit with a specific sequence is identical to all the other units. The transcription unit produces the protein while the non-transcribed spacer is the region that separates the identical transcribing units. The unique purpose of the cluster is the production of the mRNA coded by the transcription unit in large quantities. The transcribed proteins can associate among themselves in large amounts and with the DNA segment that functions as a promoter or enhancer of the gene of interest, the DNA sequence of the promoter or enhancer of the gene of interest, recognize the viral protein synthesized by the tandem gene. Excess viral protein is produced in a short time period of less than 30 min. Thus, large overproduction of viral proteins that activate the promoter/enhancer of the gene of interest is a characteristic of this system. A single class of tandemly repeated cluster lack introns.

In summary, *a large number of tandemly repeated identical DNA sequences can be made to function as activators or enhancer for early viral promoter protein transcription of a given gene.* Thus, the plant viruses such as *Geminiviruses,* when infecting the cell and disassembly, in the presence of the tandemly repeated DNA genes will bind to the promoters or enhancers of the tandemly repeated genes and will overproduced viral promoter proteins which will produced a large amount of viral promoter proteins. In turn, the viral promoter or enhancer proteins will bind the promoter of PAC, the enzyme that produces picolinic acid, and disrupts ZFPs of *Geminiviruses.*

If a second gene is placed in the plant genome, and this is desirable to prevent any virus from escaping to other adjacent cells, the gene will have PCD ability, the activation of the PCD activity, represented by a plant proteolytic enzyme will ensure the destruction of the plant cell infected by the *Geminivirus* or other plant virus. The following paragraphs will explain in more detail examples of this invention which are critical to the elimination of plant viruses by disruption of ZFPs and also by PCD.
1. This type of tandemly repeated construct that respond to the viral *Tandemly repeated sequences that function* as *pathogenic virus activators or enhancers of early transcription whose function is to early and continuously promote or enhance the promoter for other gene promoter such* as *PAC, or a proteolytic enzyme designed to induced apoptosis, or any other type of construct to destroy the viral infected cell, to prevent the escape of viruses to adjacent cells, and thus, perpetuating the infection of the plant.*
2. promoter or enhancer of the infecting *Geminivirus, will assure the continuous large overproduction of the viral protein promoter which will stimulate the apoptotic substances* such as picolinic acid, specific proteolytic enzymes, etc, [controlled by the viral promoter/enhancer of the infecting virus], which will result in the destruction of the infected plant cell by PCD, and thus prevent the possibility that the viral promoter proteins, as they decrease by destruction of the virus, will not have the opportunity to escape to other cells.
3. For example, the gene encoding the enzymatic function of a proteolytic enzyme [e.g. metalloprotease-Ca²⁺ dependent] was inserted into a compatible vector with the enhancer sequences of Cassavavirus and was placed adjacent to the early SV40 promoter. The SV40 was placed in a tandem repeat mode to respond to the activation of PAC gene expression. Computer models show that the levels of picolinic acid will be higher than 15 mM, a quantity sufficient to destroy the ZFPs of *Cassavavirus* transcription factors ZFPs.

It is entirely possible that the tandem repeat DNA elements may interact with host - specific molecules related to *Cassavavirus,* which the virus uses in its normal hypercycles. Since the purpose of the syngenic plants is to defend the plant against the invading *Cassava virus* and to produce apoptosis or PCD in the individual infected cell, the host range of these eukaryotic viruses should decrease, as they are destroyed in numerous cells of numerous species of plants by PCD which is enhanced by the viral enhancers/promoters.

The use of the **TRS** method described above is intended not only for Cassava. It is intended for any plant or plant cell that can be infected by a pathogenic virus. Furthermore, *it is also part of the instant invention that the inventors can used the activation of metacaspases to eliminate pathogenic viruses in certain plants in which those enzymes are expressed.*

The **Cassette TRS construct** (**CTRSC**) includes a DNA sequence capable of controlling the expression of a designated nucleotide sequence in a compatible host plant cell. The EC consist of a strong promoter operably linked to the nucleotide sequence of interest which is functionally linked to strong termination sequences. The DNA sequences are arranged in a way that proper transcription and translation of the nucleotide sequence of interest will occur. The coding regions codes for the proteins of interest of this invention and links to fusion components which will remain fused to the sequence of interest may be added.

**"Cassette TRS Construct" of Caspases incorporated in Plants.** It is anticipated by the inventors that caspases 9 can be incorporated into the genome of a TRS syngenic plant in a CTRSC and when a virus infects the plant cell, the viral protein promoter can activate the strong, genetically designed caspases 9 promoter, constructed in a tandem repeated gene of about 10 to 50 copies of caspase 9, with a strong stop codon, and produce the enzyme to eliminate the last traces of virus by initiating simultaneous the inactivated of their ZFPs transcription factor by picolinic acid or derivatives thereof and the direct or indirect cleavage of the promoter proteins of the invading virus. Like many proteases, capases exist as zymogens and must undergo activation cleavage for apoptosis to be initiated. Interestingly, caspases can be hydrolyzed by other caspases and also autocatalytically. After the caspase 9 is activated the death program is initiated and irreversible, destroying the cell by apoptosis. The process is so efficient that the death cells disappear without leaving any sign of their existence and there is no detectable inflammation.

**Other chelating agents:** It will be appreciated that various changes and modifications may be made in the preparations and methods described and illustrated without departing from the scope of the appended claims. For example, suitable preparations, other than picolinic acid produced by PAC, of metal chelating compounds may be employed for the disruption of ZFPs of plant viruses. The preparations may be used alone or in combination with other metal chelating agents. Furthermore, the preparations may be used to treat a wide spectrum of plant viral diseases mediated by zinc finger proteins or other metal ion dependent proteins or enzymes. Therefore, the foregoing specification and accompanying drawings are intended to be illustrative only and should not be viewed in a limiting sense.

### EXAMPLE 24

### AS THE VIRAL PROMOTER PROTEINS AND TRANSCRIPTION FACTORS (ZFPs) INTERACT WITH THE PAC PROMOTER DNA, THE PAC SYSTEM DECREASES THE PRODUCTION OF PICOLINIC ACID AND EVENTUALLY PA PRODUCTION STOPS.

*Geminiviruses* that attack Cassava cells *induced overproduction* of DnaJ ZFPs, ribosomal ZFPs, and numerous other proteins. Transcriptional factors of *Geminivirus* and other ZFPs required for viral replication, disassembly, assembly and movement, rapidly control the host cell and the virus is free to assemble, create new viruses and invade other plants and plant cells.

The inventors have designed a system in which a plasmid can be constructed to produce the enzyme Picolinic Acid Carboxylase (PAC), which after transcription and translation of PAC mRNA in the plant cells it will result in the production by PAC, in the presence of the appropriate substrate of Picolinic acid. In summary: 1) In the presence of the natural substrate of PAC, 2-amino-carboxy-muconate semialdehyde; 2) the substrate is transformed by PAC in 2-aminomuconate semialdehyde; and 3) in a non-enzymatic reaction looses H₂O, and is converted into Picolinate (2-pyridinecarboxylic acid) .

Production of endogenous Picolinic Acid at high levels will result in the destruction of a large proportion of the virus, but possibly not all the viral particles. The decreased in viral promoter proteins may clearly result in the arrest of the PAC DNA promoter system for lack of stimulation with viral promoter/enhancer proteins and thus, the PAC gene will not produce any more picolinic acid in sufficient quantities to destroy the *Geminivirus.*

The induction of the PAC system by the syngenic plants cells containing genomic PAC *produces picolinic acid only when the strong promoter of PAC is activated by transcriptional viral proteins that are released by the Geminiviruses during plant cell infection.* As a result, the promoter of PAC is activated by the viral proteins and produces increased levels of the antiviral agent picolinic acid intracellularly.

In summary, by using this feed-back control system [promoter-virus], *the instant invention controls the viral infection at several different levels such as disassembly, viral protein synthesis, assembly, encapsidation and movement.* The result of the production of endogenous picolinic acid at high levels will result in the destruction of a fraction of the virus population and the cell may remain viable. More likely the virus-infected cells with significant number of viruses will enter into PCD and *Geminiviruses* will be destroyed in the process. The absence of stimulation of the strong promoter of PAC by the lack of viral encoded promoter protein stimulation will result in a decrease of PA, which will not be necessary after viral disintegration.

*Thus, the tandem system described above in combination with PAC should assure the complete elimination of Geminivirus or any other virus infecting Cassava or other plant cells.*

### EXAMPLE 25

### GENERATION OF THE PLASMID pPAC-1 ENCODING PICOLINIC ACID CARBOXYLASE

An important consideration for the practice of this example is that the DNA sequence encoding picolinic acid carboxylase (PAC) must be prepared via a method of synthesis that eliminates the introns in the DNA sequence of PAC. The reason for this requirement is that plant cells may be unable to eliminate the introns and thus the PAC protein product(s) may be non-functional. Thus, the PAC will be transcribed as an intron-free mRNA and the protein translation product will be an active PAC ready to produce PA.

Typical vectors useful for expression of genes in higher plants are well known by molecular biologists and include vectors derived from tumor-inducing (Ti) plasmid of *Agrobacterium tumefaciens* (Rogers et al., Methods in Enzymol., 153:253-277, 1987) (**FIG. 18**). The main characteristic of these vectors is that they are plant integrating vectors in that on transformation, the vectors integrate a portion of the DNA vector into the genome of the host plant. For example, *A. tumefaciens* vectors useful for this invention are the plasmids pKYLX7, and pKYLX6; and the plasmid pBI101.2. The use of these plasmids should provide an immediate phenotypic effect concerning PAC [*only production ofpicolinic acid*] but not in other respects. Thus, *Geminivirus* replication can be inhibited, and the virus destroyed by at least one or all of the following disrupting actions of picolinic acid on zinc finger domains of: (1) *Geminivirus* and other viruses containing intrinsic ZFPs which are released intracellularly to initiate the process of replication; and (2) Plant cell ZFPs induced by the viral proteins acting on plant cell genes to overproduced ZFPs proteins such as DnaJ, ribosomal ZFPs, and other plant cell ZFPs utilized by the virus. The target sequences (zinc finger motifs) can be disrupted by picolinic acid and derivatives thereof generated by the virus-induced PAC enzyme production in the presence of the adequate enzyme substrate.

Cassava plant cells can be transformed with plasmid pPAC-1 [containing PAC DNA sequence] enclosed in *Agrobacteria* according to the method described above and in Hooykaas, P.J.J, Agrobacterium, Encyclop. of Microbiol. Vol. pp78-85; 2000). A number of kanamycin-resistant primary transformants can be tested for expression of the construct. Northern blot analysis, Southern blot analysis and HPLC can be used to test for the PAC DNA gene sequence inserted into the genome of the syngenic plant cells, after restriction enzyme cutting. The resulting cDNA fragment of PAC can be used as a probe for the RNA hybridization.

### EXAMPLE 26

### Development of TRS Syngenic Plants Resistant to Viral Pathogens by Insertion of a Virally Controlled Death Inducing Gene

The following concepts are a summary of previous examples on the organization of viral resistance Cassette inserted into the plant genome.
**Concepts** (**1**): Many viral genomes require residence in the nucleus for transcription and replication of their genome (e.g. DNA viruses like *Geminiviruses*). Transcription of viral genes requires a virally encoded transcription factor that has a high affinity for a specific DNA sequence on the viral genome. This DNA sequence is termed the viral promoter (VP). Binding of the viral transcription factor allows for transcription of viral genes.
**Concepts** (**2**): If the host genome contains a sequence that is the same as the viral promoter, the viral transcription factor will bind to that site and induce transcription of the downstream gene(s). If several viral promoters (e.g. >10) are present in a tandem array, the viral transcription factor will be recruited with high efficiency to **the ectopic** site and induce transcription of the downstream gene(s).
**Concepts (3):** If the tandem array of viral promoters is present upstream of a gene that induces host cell death, upon viral infection, the death inducing gene will be expressed and kill the cell and prevent viral replication and spread.
**Concepts (4):** TRS syngenic plants, containing the virally controlled death inducing cassette, would not express the death inducing gene unless a virus invaded. Upon viral invasion, infected cells would rapidly die and prevent the spread of infectious virions to neighboring cells.
**Important Considerations:** Death must be induced via a method that does not produce any toxic byproducts that would be harmful to consumers of the syngenic plant.

### EXAMPLE 27

### Cell death induction by Phytophthora Elicitins.

Elicitins are small secreted proteins synthesized by species of the plant-pathogenic oomycet Phytophthora and Pythium (Ponchet el al., 1999). Elicitins Class I are secreted in culature and are conserved among Phytophthora species.

Elicitin treatment of responsive plants induces a hypersensitive response (HR) which results in a form of program cell death. The death cells releases numerous pathogenesis-resistance factors, include phytoalenxins proteins. The cells that survive the pathogenic effects of elicitins acquire resistance by the action of the factors release by HR and by the products of cell death induced by elicitins (Keizer et al., 1998).

After elicitin treatment, plant disease resistant mechanism is activated. They include the induction of calcium ion influx, production of oxygen radicals, and activation of mitogen-responsive protein kinases like MAPKs (Takemoto, D, et al., 2005; Tavernier et al., 1995).

One of the goals of this invention is to introduce death genes in tandem repeat sequences to be activated by viral, fungal, bacterial, prokaryotic or eukaryotic promoters when the plant cell is invaded by pathogenic organisms. Introduction of tandem repeat sequences encoding elicitins under the control of a viral inducible promoter responsive to viral transcription factors in a cassette construct should activate the synthesis of elicitins which will cause cell death with the elimination of both the pathogenic organism and the plant cell. The viral, fungal, bacterial, prokaryotic or eukaryotic inducible promoters can initiate cell death in response to a plethora of transcription factors which will activate the production of elicitins.

Phytophthora species are some of the most destructive pathogens of crops and natural plant communities. They produce highly conserved elicitor proteins denoted elicitins, which are specific for certain plant species. The DNA cassette constructs of instant invention by incorporating a third death gene in the form of an elicitin DNA sequence under the control of an inducible promoter in both the promoter and the elicitin sequence in tandem repeat should provide a large quantity of lethal elicitin in large quantities and rapid fashion which is compatible with the death of both the cell and the invading pathogenic agent.

**In summary:** A major goal of this invention is the production of crops with increased and durable resistance to a wide-spectrum of diseases. Experimental evidence exists that inducible promoters present in cassettes units which corresponds to a sequence able to bind to a specific viral transcription factor protein which is released when the pathogenic virus disassembles inside the infected cell. Thus, viral promoters with specific sequences bind to specific pathogenic viral transcription factors necessary for viral replication. The promoters can be under combinatorial and permutation control, depending on the physical or chemical environment, where they operate. It is highly likely that the promoters of the cassettes constructions of this invention will be the most active in meristematic cells in all tissues, since these cells contain all the necessary factors and cofactors for cell duplication. Suitable promoters should switch a gene on or off. Furthermore both abiotic and biotic stress cannot activate the background expression of the transgene. The modular nature of the cassette constructs with tandem repeated DNA promoters and genes will become apparent as the various examples are described.

In the case of plant resistance to pathogenic viruses, the combination of instructions in a string of DNA, with control elements connected in series, and adapted to respond to a pathogenic viral transcription factor in a specific fashion, resulting in the death of the pathogenic virus and the death of the infected cell. The method herein described of operation of inducible resistant to the virus consist of activating the promoter (s) responsive to the viral transcription factors in combination with the activation of a series of death genes in tandem repeated sequences.

## Claims

1. A combination of promoters for use with genes, said combination of promoters further comprising:
tissue-specific, inducible by pathogens, unmodified native promoters and synthetic minimal promoters controlling pathogen expression;
said promoters operably linking to death genes in tandem repeat sequences;
said promoters and linked death genes being suppressed in nucleotide constructs in the absence of pathogenic agents therein, inactive in the absence of pathogens; and,
said promoters operably linking to encoding polynucleotides of said promoter combination in tandem repeat sequence;
wherein said promoters provide strong resistance to pathogens by preventing pathogenic replication in infected cells.

2. The promoter combination of claim 1 wherein said promoters are synthetic and said death genes are one of synthetic or cloned.

3. The promoter combination of claim 1 further comprising:
said promoter combinations controlling the promoter elements upstream of the start point (0) of the death genes, and upstream located at about positions -100 to -150; and,
said DNA construct containing a sequence denoted CAAT box for promoter enhancement regulation.

4. The promoter combination of claim 3 further comprising:
said promoter control elements including positive and negative signals, said signals including at least one of
steroid response elements (+),
thyroid hormone (Th) response elements binding the Th receptor-repressor-dimmer to promoter DNA and releasing the Th repressor by the presence and binding of Th to one subunit of the Th receptor; or
a transition metal ion including one of copper or iron delivered to the nucleus by a derivative of picolinic acid including fusaric acid.

5. The promoter combination of claim 1 further comprising:
at least one cassette, said cassette having tandem repeat units, said tandem repeat units containing promoters, being induced by pathogenic transcription factors produced by virus, fungus, or bacteria wherein cell death proteins are only produced in the presence of transcription factors of the virus, fungus or bacteria, and are not transcribed in the absence of the infectious pathogens.

6. The promoter combination of claim 3 further comprising:
transgenes controlled by at least one of the following promoters Constitutive, Tissue-specific, hormonally inducible, Chemically inducible, Native, Synthetic, Minimal and Pathogen-inducible promoters being one of eukaryote, prokaryote or viral; and,
said promoters being inactive in the transgenic plants having said promoter combination under disease free conditions, and in the presence of endogenous or exogenous physical or chemical changes.

7. The promoter combination of claim 5 further comprising:
said at least one cassette including resistant genes being effective and durable, and
said promoters including rapid switching of a gene upon detection of viral, fungal, bacterial or other pathogenic eukaryote or prokaryote pathogenic organism;
said promoters including default off switching and rapid automatic degradation from the death of infected cells upon activation of said cassettes 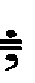 and,
said cassette containing said promoters having a synthetic construction selected for the lack of expression in the absence of viruses, strength in the presence of viral transcription factors, rapid inducibility, and rapid switch off.

8. The promoter combination of claim 5 further comprising:
said cassette being repressed when metallothionein without transition metal ions binds to the metallothionein promoter; and,
said cassette being dissociated when the pathogenic transcription factor has been detected on the promoter by addition of copper or iron chelated to picolinic acid.

9. A composition of matter introducing and releasing a series of early and late viral transcription factors into a cell nucleus comprising:
at least two cassette units containing at least one pathogen inducible promoter sequence in tandem repeat units, a nucleotide sequence encoding at least one death gene,

10. The composition of matter of claim 9 further comprising:
three of said pathogen inducible promoter sequence binding to the viral transcription factors, a first sequence producing a first death gene protein, a second sequence producing a second death gene protein, and a third sequence producing a third death gene protein wherein =the inducible death gene proteins lead to both pathogen and cell disintegration;
wherein said inducible promoter sequences prevent the propagation of any infection throughout adjacent cells.

11. The composition of matter of claim 10 further comprising:
said pathogen inducible promoter sequences including a first sequence producing a first death gene protein encoding PAC, a second sequence producing a second death gene protein encoding proteolytic enzyme, and a third sequence producing a third death gene protein encoding a second proteolytic enzyme.

12. The composition of matter of claim 10 further comprising:
each of said sequences encoding a death gene producing one of PAC, proteolytic enzyme, and metacaspase.

13. The composition of matter of claim 10 further comprising:
said cassette including about one to at least 200 of said inducible promoter sequences, wherein said sequences induce cell death after transcribing and expressing said first death gene and its resulting Picolinic Acid Carboxylase.

14. The composition of matter of claim 9 further comprising:
said first death gene producing picolinic acid intracellularly, inhibiting the function of zinc finger proteins in infected cells, inducing disintegration of pathogenic transcription metalloproteins, therein preventing propagation of infection.

15. The composition of matter of claim 10 further comprising:
said second death gene encoding a protease in the genome of an infected cell containing an inducible promoter responsive to infectious transcription factors, inductive of expression of subsequent death genes constructed in tandem repeat units of at least one to about 200 copies of protease sequences wherein the protease cleaves the infectious transcriptional factor, structural proteins, and cellular proteins, resulting in cell death.
